# EUROPEAN PATENT SPECIFICATION

(11) **EP 3 911 687 B1**
(45) Date of publication and mention of the grant of the patent: **01.11.2023**
(21) Application number: 20700138.9
(22) Date of filing: 10.01.2020
(51) Int. Cl.: C08F 222/10, B33Y 70/00, C08F 2/50, C07D 303/06, B29C 64/124

(54) **LIMONENE-BASED (METH)ACRYLATES FOR USE IN 3D PRINTING**
LIMONENBASIERTE (METH)ACRYLATE ZUR VERWENDUNG IM 3D-DRUCK
(MÉTH)ACRYLATES À BASE DE LIMONÈNE POUR L'IMPRESSION 3D

(30) Priority: 14.01.2019 EP 19151668
(43) Date of publication of application: 24.11.2021
(73) Proprietor: BASF SE, 67056 Ludwigshafen am Rhein (DE)
(72) Inventor: FUCHS, Andre, 4057 Basel (CH); MUELHAUPT, Rolf, 79104 Freiburg (DE); ASMACHER, Anne, 79104 Freiburg (DE); SCHIMPF, Vitalij, 79104 Freiburg (DE)
(74) Representative: BASF IP Association
(86) International application number: PCT/EP2020/050567
(87) International publication number: WO 2020/148189

(56) References cited:
- WO-A2-2014/012937
- CN-A- 105 785 714
- CN-A- 106 632 895
- CN-A- 108 864 378

## Description

The present invention relates to photocurable compositions, comprising a limonene-based (meth)acrylate (A) obtainable by reacting a) 1 equivalent of a compound of formula 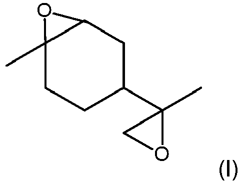 with 1 to 3 equivalents of a compound of formula 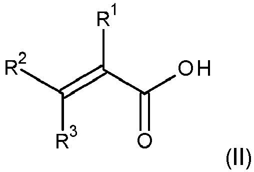 in the presence of a catalyst and an inhibitor at elevated temperature and its use in a photopolymerization 3D printing process. The limonene-based (meth)acrylate (A) significantly increases stiffness and glass transition temperatures of photo-cured acrylate compositions. While such effects are usually achieved with the use of aromatic or bisphenol A based compounds, the limonene-based (meth)acrylate (A) offers a sustainable alternative at much lower viscosity.

WO14012937 relates to a method for the manufacture of a terpene-based polymer comprising the steps of:
a) reacting a terpene oxide and/or a terpene dioxide with a carboxylic acid comprising at least one unsaturated carbon-carbon bond;
c) polymerizing the product obtained in step a) by free radical polymerization.

In step a) unsaturated hydroxy esters represented by formulae (IIIa), (lVa), (Va), (Vla), (Illb), (IVb), (Vb) and (Vlb) are obtained by reacting terpene dioxide with acrylic acid and methacrylic acid, respectively:

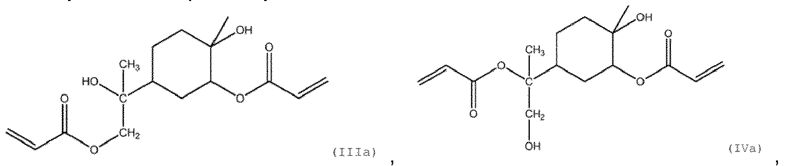

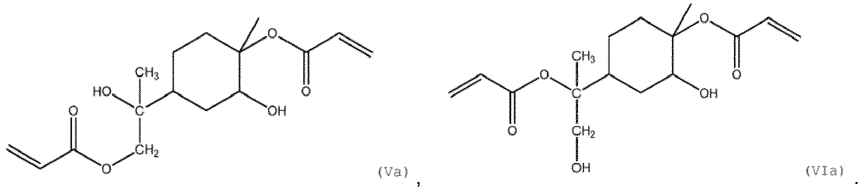

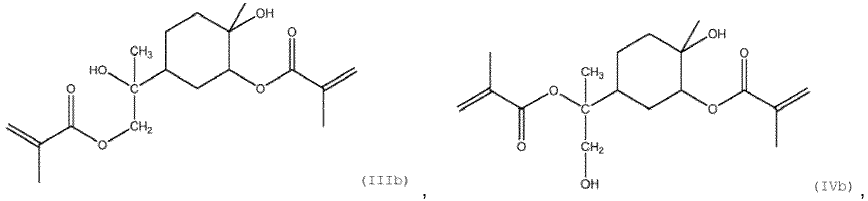

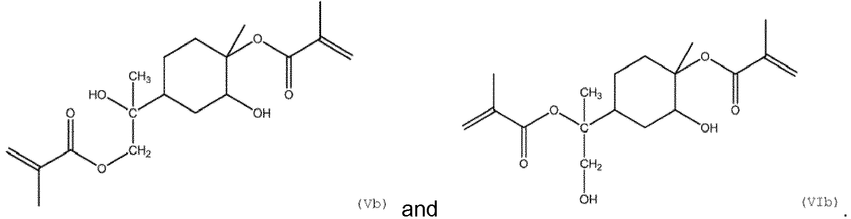

CN106632895A relates to low viscosities photocurable composition for application in 3D printing comprising a reactive diluent, a photoinitiator, a limonene cation photocuring accelerator and a urethane acrylate resin.

CN108864378A relates to photocurable composition for application in 3D printing comprising a reactive diluent, a photoinitiator, and a urethane acrylate resin or a polyester acrylate resin or an epoxy acrylate resin.

CN105785714A relates to photocurable composition for application in 3D printing comprising a reactive diluent, a photoinitiator, and a silicon modified polyether resin or a hyperbranched polyester acrylate resin or an epoxy acrylate resin.

It has now been found, surprisingly, that the use of limonene-based acrylates in photocurable compositions for the application in 3D printing results in materials with high stiffness and high glass transition temperatures. The natural terpene limonene can be extracted from orange peel wastes and thereby represents a sustainable building block from renewable resources which are not in competition with the food industry (P. T. Anastas et al., Innovations in Green Chemistry and Green Engineering, Springer, New York, 2013). The epoxidation of limonene to yield limonene dioxide (LDO) has already been described in literature and LDO, in fact, is already commercially available (Nitrochemie Aschau GmbH). LDO and (meth)acrylic acid (MA) readily react under ring-opening to form β -hydroxy ester linkages, thereby attaching the UV-reactive (meth)acrylate group to the limonene scaffold.

Accordingly, the present invention is directed to photocurable compositions, comprising
(A) a limonene-based (meth)acrylate obtainable by reacting
   a) 1 equivalent of a compound of formula 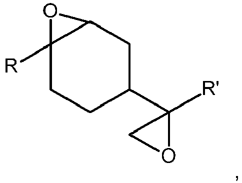 especially 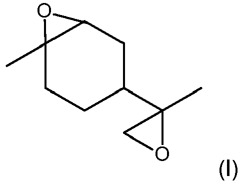 with 1 to 3 equivalents of a compound of formula 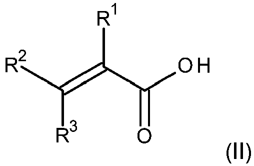 in the presence of a catalyst and an inhibitor at elevated temperature, wherein
      R and R' are independently of each other H, or a methyl group,
      R¹, R² and R³ are independently of each other H, or a C₁-C₄alkyl group, and
   b) optionally reacting the product obtained in step a) with an acid anhydride, or acyl halide;
(B) optionally an oligomer (B),
(C) a diluent (C), and
(D) a photoinitiator (D).

The viscosity of the photocurable compositions is in the range 10 to 3000 mPa s, preferably 10 to 1500 mPa s at 30 °C. In case of photopolymer jetting the viscosity of the photocurable composition is adjusted to be in the range of 10 to 150 mPas at 30 °C. In case of vat-based photopolymerization the viscosity of the photocurable composition is adjusted to be in the range of 50 to 1500 mPas at 30 °C. Most commercial print heads require heating to reduce ink viscosity which is typically in the range of 10 to 20 mPas.

The viscosities were measured on a MARS from Thermo Scientific using a plate/plate set-geometry with a plate diameter of 35 mm and a gap of 0.6 mm at various shear rates from 0.1 to 100 s⁻¹ at 25 °C (100 steps, logarithmic, 5 s per step, 3 s integration time, 25 °C) and the final viscosity received as an average over all 100 values.

R² and R³ are preferably H, or a methyl group, more preferably H. R¹ is preferably H, or a methyl group, more preferably a methyl group.

The limonene-based (meth)acrylate (A) significantly increases stiffness and glass transition temperatures of photo-cured acrylate compositions. While such effects are usually achieved with the use of aromatic or bisphenol A based compounds, the limonene-based (meth)acrylate (A) offers a sustainable alternative at much lower viscosity.

The present invention is directed to the use of the photocurable composition according to the present invention, or a limonene-based (meth)acrylate, obtainable by
a) reacting a) 1 equivalent of a compound of formula (I) with 1 to 3 equivalents of a compound of formula (II) in the presence of a catalyst and an inhibitor at elevated temperature, in a photopolymerization 3D printing process.

In addition, the present invention relates to the use of the photocurable composition of the present invention in a photopolymerization 3D printing process, in particular vat polymerization, or photopolymer jetting; and a method for producing a three-dimensional article, comprising
a) providing the photocurable composition according to the present invention,
b) exposing the photocurable composition to actinic radiation to form a cured crossection,
c) repeating steps (a) and (b) to build up a three-dimensional article.

The amount of component (A) is 5.0 to 90.0 % by weight, especially 20.0 to 70.0 % by weight, very especially 30.0 to 60.0 % by weight based on the amount of components (A), (B), (C) and (D).

The amount of component (B) is 0 to 70.0 % by weight, especially 5.0 to 60.0 % by weight, very especially 20.0 to 40.0 % by weight based on the amount of components (A), (B), (C) and (D).

The amount of component (C) is 20.0 to 80.0 % by weight, especially 30.0 to 60.0 % by weight based on the amount of components (A), (B), (C) and (D).

The amount of component (D) is 0.1 to 10 % by weight, especially 0.1 to 5.0 % by weight, very especially 0.5 to 2.0 % by weight based on the amount of components (A), (B), (C) and (D).

The present invention is illustrated in more detail on basis of the compound of formula (I).

### Limonene-based (meth)acrylate

The compound of formula 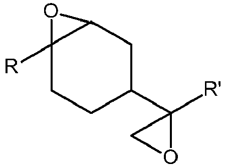 is preferably a compound of formula 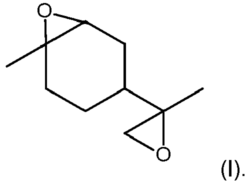 The present invention is illustrated in more detail on basis of the compound of formula (I).

The limonene-based (meth)acrylate (A) is obtained by reacting
a) 1 equivalent of a compound of formula 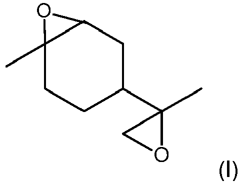 with 1 to 3 equivalents of a compound of formula 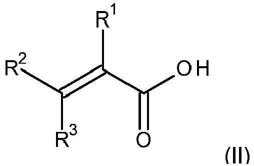 in the presence of a catalyst and an inhibitor at elevated temperature. R¹, R² and R³ are independently of each other H, or a C₁-C₄alkyl group. R² and R³ are preferably H, or a methyl group, more preferably H. R¹ is preferably H, or a methyl group, more preferably a methyl group.

"Elevated temperature" a temperature of 80 to 120 °C, preferably a temperature of 90 to 110 °C.

Optionally the product obtained in step a) can be reacted in a further step b) with an acid anhydride, or acyl halide. Examples of anhydrides are propionic anhydride, or acetic anhydride. Advantageously, the anhydrides may contain a double bond. Examples are acrylic and methacrylic anhydride. The acyl halide is a compound of formula R⁶COX, wherein R⁶ is for example a C₁-C₈alkyl group, or a C₂-C₈alkenyl group and X is Cl, Br, or I. Examples of acyl halides are acetyl chloride, acryloyl chloride and methacryloyl chloride.

The present disclosure is also directed to a process for the production of a limonene-based (meth)acrylate, comprising
a) reacting 1 equivalent of a compound of formula (I) with 1 to 3 equivalents of a compound of formula (II) in the presence of a catalyst and an inhibitor at elevated temperature, and
b) optionally reacting the product obtained in step a) with an acid anhydride, or acyl halide and the limonene-based (meth)acrylate obtainable by the process.

Preferably, no solvent or co-solvent is used in step a).

Catalysts are used in step a). Said catalysts are any catalysts suitable for the reaction of epoxy resins with carboxylic acids, such as quaternary ammonium salts (e.g. benzyltriethyl ammonium chloride), a compound comprising an electron-rich, non-nucleophilic nitrogen and/or oxygen atom, such as an amide, a urea (e.g. tetramethylurea), an amidine (e.g., 1,8-diazabicycloundec-7-ene ("DBU")), a pyridine, (e.g., pyridine), or an imide (1,2-dimethylimidazol), or triphenyl phosphine. In some embodiments, the catalyst comprising the nucleophilic nitrogen atom may also function as a solvent. For example, in some embodiments, the compound comprising an amide comprises dimethylformamide (DMF), dimethylacetamide (DMA), N-methyl-2-pyrrolidone (NMP), or combinations thereof.

The catalyst is added in an amount of 0.1 - 20 mol%, preferably 1 - 5 mol% based on mol of compound of formula (I).

Step a) is carried out at elevated temperature, such as a temperature of 80 to 120 °C, preferably 90 to 110 °C. Step a) can also be carried out at a higher temperature, provided no decomposition, or degradation, of the resulting product occurs, and/or provided that no premature polymerization occurs. The reaction time is 1 h to 6 d.

Inhibitors are used in step a). Said inhibitors are any inhibitors suitable for preventing the thermal polymerization, decomposition, or degradation of the resulting products. Examples of conventional inhibitors include butylated hydroxytoluene, hydroquinone, hydroquinone monomethyl ether, or derivatives thereof; 4-hydroxy-2,2,6,6-tetramethyl-1-piperidinyloxy (4-hydroxy-TEMPO), and phenothiazine. Other known types of polymerization inhibitors include diaryl amines, sulphur-coupled olefins, or hindered phenols.

The inhibitor is added in an amount of 0.01 -1 wt%, preferably 0.1 - 0.6 wt%.

Reaction of the compound of formula (I) with compound of formula (II) results in the formation of compounds of formula 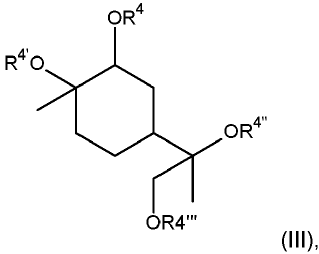 wherein one, or two of the substituents R⁴, R^{4'}, R^{4"} and R^{4‴} are a group of formula 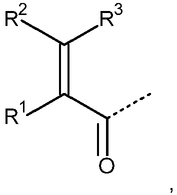 such as, for example, compounds of formula (IIIa), (IIIb), (IIIc) and (IIId) (limonene dimethacrylate (LDMA)):

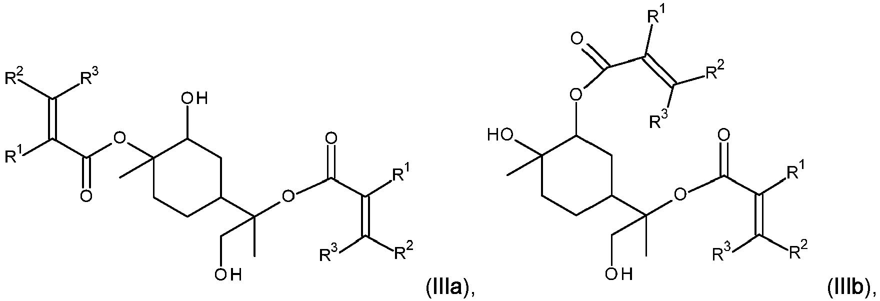

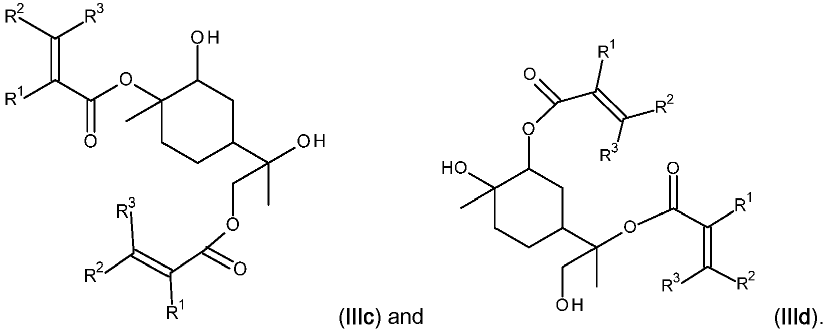

The compound of formula (Illa) represents the most favored product. The formation of the compounds of formula (IIIb) and (IIIc) is less favored and the compound of formula (IIId) is the least favored. The compound of formula (III) has one, or two, on average about 1.3 groups of formula 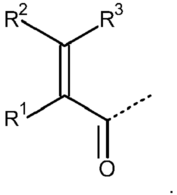 In addition, limonene oligomers (LMA-Oligomers) of formula 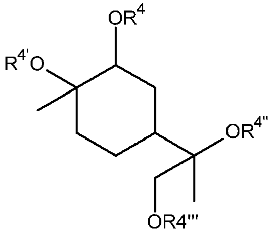 (IV) are formed, wherein one, or two of the substituents R⁴, R^{4'}, R^{4"} and R^{4‴} are a group of formula 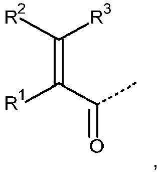 one of the substituents R⁴, R^{4'}, R^{4"} and R^{4‴} is a group of formula 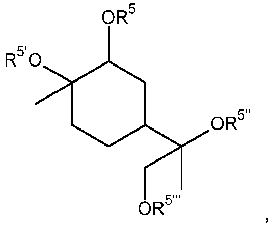 wherein one of the substituents R⁵, R^{5'}, R^{5"} and R^{5‴} represent the bond to an oxygen atom of the basic skeleton, one, or two of the substituents R⁵, R^{5'}, R^{5"} and R^{5‴} is a group of formula 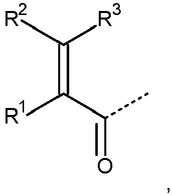 such as for example, compounds of 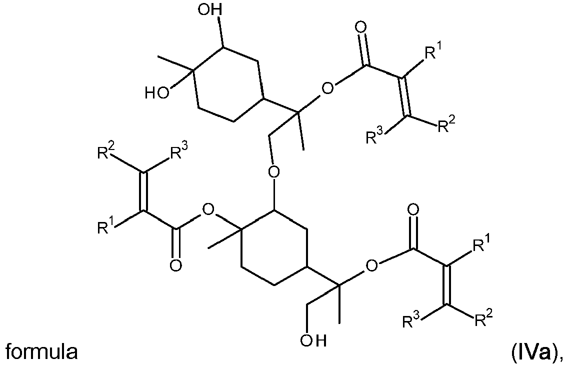 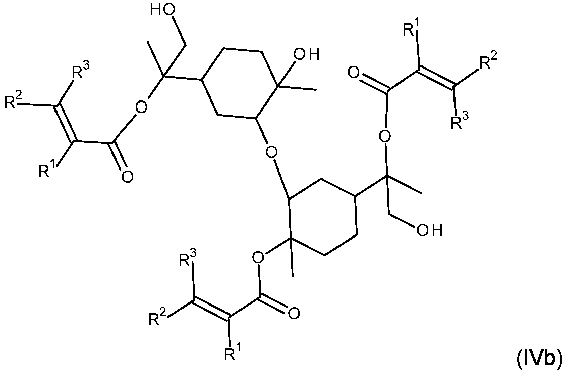 and 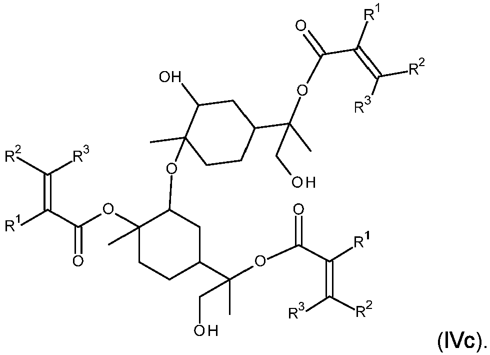 The compound of formula (IV) has two to four, on average about 2.6 groups of formula 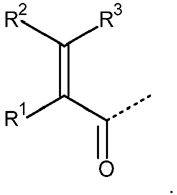 In addition, the limonene-based (meth)acrylate (A) may contain non-reacted compound of formula (II).

The reaction with the acid anhydride (step b) can be done without solvent. The reaction with the acyl halide (step b) can be done in a solvent, such as, for example, dichloromethane, in the presence of an acid scavenger, such as, for example, pyridine, or a tertiary amine.

The viscosity of the limonene-based (meth)acrylate (A) obtained by reacting compound (I) and (II) usually is in the range of 1 to 500, especially 1 to 200 Pa•s at room temperature (25 °C) and may be regulated by:
- varying the ratio of compound (I) and (II) in step a);
- modification of the limonene-based (meth)acrylate (A) obtained in step a) by reaction with an acid anhydride or an acyl halide (step b));
- the presence, or absence of reactive diluents, and/ or
- the presence, or absence of oligomers.

In a preferred embodiment of the present invention the limonene-based (meth)acrylate obtained after step a) or b) is used without further purification in the photocurable compositions of the present invention.

In another preferred embodiment step a) or b) are followed by one or more steps c):
- removing the non-reacted compound of formula (II);
- converting the compound of formula (II) into its non-volatile methacrylate anion;
- converting the compound of formula (II) into a non-volatile diluent;

The non-reacted compound of formula (II) can, for example, be removed by liquid-liquid extraction, or at elevated temperatures and reduced pressure using, for example, a thin-film evaporator.

The conversion of the compound of formula (II) into its non-volatile methacrylate anion can be done by reacting it with oxides of alkali and alkaline earth metals, such as, for example, MgO nanoparticles.

The conversion of the compound of formula (II) into a non-volatile diluent can be done by reacting it with glycidyl dimethacrylate (GMA) to obtain a non-volatile compound of formula

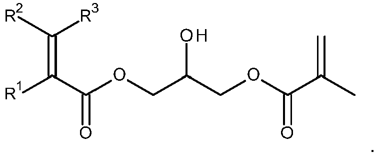

The present invention is illustrated in more detail on basis of the reaction of the compound of formula 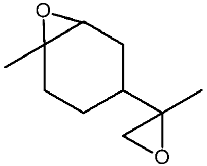 (I, limonene dioxide (LDO)) with the compound of formula 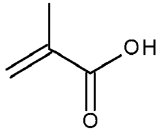 (IIa, methacrylic acid (MA)):

MA + LDO → LDMA + LMA-Oligomers + non-reacted MA

The reduction of the MA content to a negligible amount is important, as it is a toxic and volatile compound with an unpleasant odor. As evident from the table below reducing the MA equivalents from 2.1 to 1.7 and 1.3 yields reaction products with 22, 13 and 2 wt% of unreacted MA, respectively.

| **Limonene-based (metha)crylate (A)** | **MAeq.** | **Unreacted MA [wt%]^{a}** | **η (25°C)^{b} [Pa•s]** | **AcV^{d} [mmol g⁻¹]** |
|---|---|---|---|---|
| LDMA-1 | 2.1 | 22 | 5.2 ± 0.1 | - |
| LDMA-2 | 1.7 | 13 | 19.3 ± 0.4 | - |
| LDMA-3 | 1.3 | 2 | 117 ± 7 | 4.6 |
| LDMA-1_{purified}^{C} | 2.1 | 0 | 65 ± 2 | 4.5 |
| LDMA-4_{purified} ^{C} | 3.0 | 0 | 41 ± 2 | 4.3 |

| | | | | |
|---|---|---|---|---|
| [a] Determined via ¹H-NMR spectroscopy; [b] plate-plate rheometer (from 0.1 to 100 s⁻¹, 100 steps, logarithmic, 5 s per step, 3 s integration time); [c] Purified grades as received from liquid-liquid extraction using dichloromethane and 1 molar K₂CO₃ solution. [d] acrylate value = amount of acrylate groups per gram of product, determined via quantitative ¹H-NMR using 1,4-dimethoxybenzene as standard. | | | | |

LDMA-3 is particularly preferred. With less than 2 wt% of residual MA LDMA-3 does not exhibit any unpleasant odor. Furthermore, its viscosity with 117 ± 7 Pa*s lies significantly below the analogous, bisphenol A-based reference BisGMA with 560 ± 60 Pa*s.

One major side reaction occurs, namely the hydroxyl-induced ring-opening which causes oligomerization through etherification (formation of LMA-Oligomers). In order to decrease the formation of LMA-Oligomers, the equivalents of MA have to be tuned towards full conversion of both epoxy and carboxylic acid groups.

The low viscosity of LDMA-1 can be explained with significant amount of MA which acts as a reactive diluent. If LDMA-1 is purified via liquid-liquid extraction, the resulting MA-free product LDMA-1_{purified} exhibits a viscosity of 65 ± 2 Pa*s. Since the etherification is a competitive ring-opening pathway, applying an excess of MA will favor the carboxylic acid-induced ring-opening. As a consequence less oligomer formation occurs resulting in a reduced viscosity. Accordingly, when applying 3.0 equivalents of MA and purifying the product through liquid-liquid extraction the resulting MA-free LDMA-4_{purified} exhibits a viscosity of 41 ± 2 Pa*s.

Unreacted MA present in LDMA-2 or LDMA-1 may be reacted with glycidyl methacrylate (GMA) to yield the difunctional reactive diluent, glycerol dimethacrylate (GDMA):

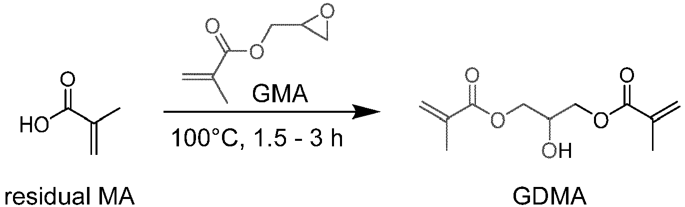

Applying 1.0 equivalents of GMA per equivalent MA causes 87 - 96 % of MA conversion and yield the products LDMA-1-GDMA and LDMA-2-GDMA with MA contents below 1 wt%. As evident from the table below the products contain 24.3 and 41.0 wt% of the reactive diluent GMDA and therefore exhibit low viscosity values of 1.48 ± 0.02 Pa*s and 4.92 ± 0.04 Pa*s, respectively.

| **Limonene-based (metha)crylate (A)** | **m_{f}(MA)^{a} [wt%]** | **Converted MA^{a} [mol%]** | **m_{f}(GDMA)^{a} [wt%]** | **η (25°C)^{b} [Pa•s]** |
|---|---|---|---|---|
| LDMA-1 | 22 | - | - | 5.2 ± 0.1 |
| LDMA-2 | 13 | - | - | 19.3 ± 0.4 |
| LDMA-1-GDMA | 0.6 | 96 | 41.0 | 1.48 ± 0.02 |
| LDMA-2-GDMA^{c} | 1.0 | 87 | 24.3 | 4.92 ± 0.04 |

| | | | | |
|---|---|---|---|---|
| [a] ¹H-NMR spectroscopy; [b] plate-plate rheometer (from 0.1 to 100 s⁻¹, 100 steps, logarithmic, 5 s per step, 3 s integration time); [c] AcV = 5.8 mmol/g. | | | | |

Typically the limonene-based (metha)crylates (A) have acrylate values (= amount of acrylate groups per gram of product, determined via quantitative ¹H-NMR using 1,4-dimethoxybenzene as standard) in the range of 4.0 to 7.0 mmol/g.

Alternatively, volatile MA can be bound by oxides of alkali and alkaline earth metals, such as, for example, MgO nanoparticles by deprotonating the carboxylic acid, thereby producing the non-volatile methacrylate anion.

In order to evaluate the impact of the above mentioned products on a given photoresin, a mixture of an acrylate prepolymer (Laromer UA9089) and a reactive dilutent (ACMO) in a weight ratio of 39:59 was used as a base formulation.

For all photoresins diphenyl(2,4,6-trimethylbenzoyl)phosphinoxid (TPO) was used with 1 wt%.

As shown in the following table introducing 10, 30 or 50 wt% of LDMA-3 to the base formulation (BF) accounts for higher viscosity of the resin and gradually increasing Youngs's moduli as well as glass transition temperature.

For comparison all the other building blocks were introduced with 30 wt%. The resin with 30 wt% LDMA-4_{purified} exhibits a 24 % lower viscosity over the use of LDMA-3, which can be attributed to the overall lower viscosity of LDMA-4_{purified} due to less oligomer formation, while the mechanical values are very alike within the margin of error.

Using 30 wt% of BisGMA, the bisphenol A-based reference, causes an increase of the resin viscosity by 78 % over the use of LDMA-3. At the same time, the influence on the mechanical properties is very similar within the margin of error as both components significantly increase stiffness, tensile strength and glass transition temperature. Hence, the use of LDMA-3 has a significant advantage over the commonly used BisGMA, since the viscosity of a photoresin is of utmost importance for the 3D printing process and typically needs to be below 1.5 Pa*s at a typical process temperature of 30 °C. Since performance enhancing building blocks typically exhibit high viscosity reactive diluents are added to adjust the resin viscosity to an appropriate level. Low viscosity performance components like LDMA-3 are therefore of high interest, because less reactive diluent is needed to maintain processability of the resin.

| **Tested component (TC)** | **m_{f}(TC) [%]** | **η (25°C)^{b} [mPa•s]** | **Y.'s mod.^{c} [GPa]** | **σₘₐₓ^{c} [MPa]** | **ε_{break} ^{c} [%]** | **T_{g}^{d} [°C]** |
|---|---|---|---|---|---|---|
| - | - | 160 ± 10 | 2.66 ± 0.05 | 71.9 ± 1.5 | 6.1 ± 0.9 | 97 |
| | | | | | | |
| LDMA-3 | 10 | 189 ± 13 | 2.8 ± 0.2 | 75.0 ± 1.2 | 6.6 ± 1.5 | 99 |
| LDMA-3 | 30 | 451 ± 16 | 3.4 ± 0.3 | 88 ± 8 | 3.9 ± 0.8 | 119 |
| LDMA-3 | 50 | 1320 ± 14 | 3.8 ± 0.2 | 85 ± 10 | 2.8 ± 0.5 | 132 |
| | | | | | | |
| LDMA-4_{purified} | 30 | 342 ± 21 | 3.20 ± 0.18 | 87 ± 6 | 4.3 ± 0.9 | 113 |
| | | | | | | |
| BisGMA | 30 | 800 ± 20 | 3.4 ± 0.4 | 83 ± 5 | 5.7 ± 0.9 | 119 |
| | | | | | | |
| LDMA-1-GDMA | 30 | 217 ± 9 | 3.7 ± 0.4 | 91 ± 3 | 5.1 ± 0.9 | - |
| LDMA-2-GDMA | 30 | 279 ± 7 | 3.7 ± 0.3 | 94 ± 2 | 5.1 ± 0.9 | 119 |
| | | | | | | |
| LDMA-1 | 30 | 246 ± 13 | 3.48 ± 0.08 | 87 ± 9 | 3.5 ± 1.2 | 117 |
| LDMA-1-MgO | 30^{a} | 504 ± 18 | 3.47 ± 0.09 | 97.9 ± 0.9 | 4.8 ± 0.6 | 135 |

| | | | | | | |
|---|---|---|---|---|---|---|
| [a] contains 1.5 wt% of MgO with respect to the overall resin, [b] determined with a plate-plate rheometer (from 0.1 to 100 s⁻¹, 100 steps, logarithmic, 5 s per step, 3 s integration time), [c] tensile testing (ISO 527 1/2, 5A, 5 mm min⁻¹) [d] DSC (10 K min, 2^{nd} heating cycle). | | | | | | |

Both components LDMA-1-GDMA and LDMA-2-GDMA contain 41.0 and 24.3 wt%, respectively, of the reactive diluent GDMA which accounts for quite low viscosity values in the test resins, while the mechanical properties even slightly outperform LDMA-3 and BisGMA in terms of stiffness and tensile strength. This is attributed to the increased network density provided by the small and difunctional GDMA. LDMA-1-GDMA (9 h) and LDMA-2-GDMA (9.5 h) can be produced in shorter overall reaction times when compared to LDMA-3 (13 h) and therefore also offer an economic advantage. Since LDMA-1 contains 22 wt% of MA, which acts a reactive diluent, the respective resin mixture exhibits a relatively low viscosity of 246 Pa*s. Introducing roughly 1.5 wt% of MgO nanoparticles [or 0.2 (g MgO) (g (MA)⁻¹] causes a significant rise of the resin viscosity by a factor of 2, which is an effect of the formation of magnesium dimethacrylate. In addition, it also accounts for a marked increase in tensile strength and glass transition temperature.

### Oligomer (B)

The oligomer (B) is selected from polyester (meth)acrylates, polyether (meth)acrylates, carbonate (meth)acrylates, epoxy (meth)acrylates and urethane (meth)acrylates, including amine-modified oligomers. The oligomer (B) may be single oligomer, or a mixture of two, or more oligomers.

Urethane (meth)acrylates are obtainable for example by reacting polyisocyanates with hydroxyalkyl (meth)acrylates and optionally chain extenders such as diols, polyols, diamines, polyamines, dithiols or polythiols.

Urethane (meth)acrylates of this kind comprise as synthesis components substantially:
(1) at least one organic aliphatic, aromatic or cycloaliphatic di- or polyisocyanate,
(2) at least one compound having at least one isocyanate-reactive group and at least one radically polymerizable unsaturated group, and
(3) optionally, at least one compound having at least two isocyanate-reactive groups.

Suitable components (1) are, for example, aliphatic, aromatic, and cycloaliphatic diisocyanates and polyisocyanates having an NCO functionality of at least 2, preferably 2 to 5, and more preferably more than 2 to 4.

Polyisocyanates contemplated include polyisocyanates containing isocyanurate groups, uretdione diisocyanates, polyisocyanates containing biuret groups, polyisocyanates containing urethane groups or allophanate groups, polyisocyanates comprising oxadiazinetrione groups, uretonimine-modified polyisocyantes of linear or branched C₄-C₂₀ alkylene diisocyanates, cycloaliphatic diisocyanates having a total of 6 to 20 C atoms, or aromatic diisocyanates having a total of 8 to 20 C atoms, or mixtures thereof.

Examples of customary diisocyanates are aliphatic diisocyanates such as tetramethylene diisocyanate, hexamethylene diisocyanate (1,6-diisocyanatohexane), trimethylhexamethylene diisocyanate, octamethylene diisocyanate, decamethylene diisocyanate, dodecamethylene diisocyanate, tetradecamethylene diisocyanate, derivatives of lysine diisocyanate, tetramethylxylylene diisocyanate, trimethylhexane diisocyanate or tetramethylhexane diisocyanate, cycloaliphatic diisocyanates such as 1,4-, 1,3- or 1,2-diisocyanatocyclohexane, 4,4'- or 2,4'-di(isocyanatocyclohexyl)methane, 1-isocyanato-3,3,5-trimethyl-5-(isocyanatomethyl)cyclohexane (isophorone diisocyanate), 1,3- or 1,4-bis(isocyanatomethyl)cyclohexane or 2,4- or 2,6-diisocyanato-1-methylcyclohexane, and also aromatic diisocyanates such as tolylene 2,4- or 2,6-diisocyanate and the isomer mixtures thereof, m- or p-xylylene diisocyanate, 2,4'- or 4,4'-diisocyanato-diphenylmethane and the isomer mixtures thereof, phenylene 1,3- or 1,4-diisocyanate, 1-chlorophenylene 2,4-diisocyanate, naphthylene 1,5-diisocyanate, diphenylene 4,4'-diisocyanate, 4,4'-diisocyanato-3,3'-dimethylbiphenyl, 3-methyldiphenylmethane 4,4'-diisocyanate, tetramethylxylylene diisocyanate, 1,4-diisocyanatobenzene or diphenyl ether 4,4'-diisocyanate.

Mixtures of the stated diisocyanates may also be present.

Contemplated as component (2) in accordance with the invention is at least one compound (2) which carries at least one isocyanate-reactive group and at least one radically polymerizable group.

The compounds (2) preferably have precisely one isocyanate-reactive group and 1 to 5, more preferably 1 to 4, and very preferably 1 to 3 radically polymerizable groups.

The components (2) preferably have a molar weight of below 10 000 g/mol, more preferably below 5000 g/mol, very preferably below 4000 g/mol, and more particularly below 3000 g/mol. Special components (b) have a molar weight of below 1000 or even below 600 g/mol.

Isocyanate-reactive groups may be, for example, -OH, -SH, -NH₂, and -NHR¹⁰⁰, where R¹⁰⁰ is hydrogen or an alkyl group containing 1 to 4 carbon atoms, such as methyl, ethyl, n-propyl, isopropyl, n-butyl, isobutyl, sec-butyl or *tert*-butyl, for example. Components (2) may be, for example, monoesters of α,β-unsaturated carboxylic acids, such as acrylic acid, methacrylic acid, crotonic acid, itaconic acid, fumaric acid, maleic acid, acrylamidoglycolic acid and methacrylamidoglycolic acid, and polyols, which have preferably 2 to 20 C atoms and at least two hydroxyl groups, such as ethylene glycol, diethylene glycol, triethylene glycol, propylene 1,2-glycol, propylene 1,3-glycol, 1,1-dimethyl-1,2-ethanediol, dipropylene glycol, triethylene glycol, tetraethylene glycol, pentaethylene glycol, tripropylene glycol, 1,2-, 1,3- or 1,4-butanediol, 1,5-pentanediol, neopentyl glycol, 1,6-hexanediol, 2-methyl-1,5-pentanediol, 2-ethyl-1,4-butanediol, 1,4-dimethylolcyclohexane, 2,2-bis(4-hydroxycyclohexyl)propane, glycerol, trimethylolethane, trimethylolpropane, trimethylolbutane, pentaerythritol, ditrimethylolpropane, erythritol, sorbitol, polyethylene glycol having a molar mass of between 106 and 2000, polypropylene glycol having a molar weight of between 134 and 2000, polyTHF having a molar weight of between 162 and 2000 or poly-1,3-propanediol having a molar weight of between 134 and 400. In addition it is also possible to use esters or amides of (meth)acrylic acid with amino alcohols such as 2-aminoethanol, 2-(methylamino)ethanol, 3-amino-1-propanol, 1-amino-2-propanol or2-(2-aminoethoxy)ethanol, for example, 2-mercaptoethanol or polyaminoalkanes, such as ethylenediamine or diethylenetriamine, or vinylacetic acid.

Also suitable, furthermore, albeit less preferably, are unsaturated polyetherols or polyesterols or polyacrylate polyols having an average OH functionality of 2 to 10.

Examples of amides of ethylenically unsaturated carboxylic acids with amino alcohols are hydroxyalkyl(meth)acrylamides such as N-hydroxymethylacrylamide, N-hydroxymethylmethacrylamide, N-hydroxyethylacrylamide, N-hydroxyethylmethacrylamide, 5-hydroxy-3-oxapentyl(meth)acrylamide, N-hydroxyalkylcrotonamides such as N-hydroxymethylcrotonamide, or N-hydroxyalkylmaleimides such as N-hydroxyethylmaleimide.

Preference is given to using 2-hydroxyethyl (meth)acrylate, 2- or 3-hydroxypropyl (meth)acrylate, 1,4-butanediol mono(meth)acrylate, neopentyl glycol mono(meth)acrylate, 1,5-pentanediol mono(meth)acrylate, 1,6-hexanediol mono(meth)acrylate, glycerol mono(meth)acrylate and di(meth)acrylate, trimethylolpropane mono(meth)acrylate and di(meth)acrylate, pentaerythritol mono(meth)acrylate, di(meth)acrylate, and tri(meth)acrylate, and also 2-aminoethyl (meth)acrylate, 2-aminopropyl (meth)acrylate, 3-aminopropyl (meth)acrylate, 4-aminobutyl (meth)acrylate, 6-aminohexyl (meth)acrylate, 2-thioethyl (meth)acrylate, 2-aminoethyl(meth)acrylamide, 2-aminopropyl(meth)acrylamide, 3-aminopropyl(meth)acrylamide, 2-hydroxyethyl(meth)acrylamide, 2-hydroxypropyl(meth)acrylamide, or 3-hydroxypropyl(meth)acrylamide. Particularly preferred are 2-hydroxyethyl acrylate, 2-hydroxyethyl methacrylate, 2- or 3-hydroxypropyl acrylate, 1,4-butanediol monoacrylate, 3-(acryloyloxy)-2-hydroxypropyl (meth)acrylate, and also the monoacrylates of polyethylene glycol with a molar mass of 106 to 238.

Contemplated as component (3) are compounds which have at least two isocyanate-reactive groups, examples being -OH, -SH, -NH₂ or -NH R¹⁰¹, in which R¹⁰¹ therein, independently of one another, may be hydrogen, methyl, ethyl, *iso*propyl, *n*-propyl, *n*-butyl, *iso*butyl, *sec*-butyl or *tert-*butyl.

Compounds (3) having precisely 2 isocyanate-reactive groups are preferably diols having 2 to 20 carbon atoms, examples being ethylene glycol, 1,2-propanediol, 1,3-propanediol, 1,1-dimethylethane-1,2-diol, 2-butyl-2-ethyl-1,3-propanediol, 2-ethyl-1,3-propanediol, 2-methyl-1,3-propanediol, neopentyl glycol, neopentyl glycol hydroxypivalate, 1,2-, 1,3- or 1,4-butanediol, 1,6-hexanediol, 1,10-decanediol, bis(4-hydroxycyclohexane)isopropylidene, tetramethylcyclobutanediol, 1,2-, 1,3- or 1,4-cyclohexanediol, cyclooctanediol, norbornanediol, pinanediol, decalindiol, 2-ethyl-1,3-hexanediol, 2,4-diethyloctane-1,3-diol, hydroquinone, bisphenol A, bisphenol F, bisphenol B, bisphenol S, 2,2-bis(4-hydroxycyclohexyl)propane, 1,1-, 1,2-, 1,3-, and 1,4-cyclohexanedimethanol, 1,2-, 1,3-, or 1,4-cyclohexanediol, polyTHF having a molar mass of between 162 and 2000, poly-1,2-propanediol or poly-1,3-propanediol having a molar mass of between 134 and 1178 or polyethylene glycol having a molar mass of between 106 and 2000, and also aliphatic diamines, such as methylene- and isopropylidene-bis(cyclohexylamine), piperazine, 1,2-, 1,3- or 1,4-diaminocyclohexane, 1,2-, 1,3-, or 1,4-cyclohexanebis(methylamine), etc., dithiols or polyfunctional alcohols, secondary or primary amino alcohols, such as ethanolamine, monopropanolamine, etc. or thio alcohols, such as thioethylene glycol.

Particularly suitable here are the cycloaliphatic diols, such as, for example, bis(4-hydroxycyclohexane)isopropylidene, tetramethylcyclobutanediol, 1,2-, 1,3-, or 1,4-cyclohexanediol, 1,1-, 1,2-, 1,3-, and 1,4-cyclohexanedimethanol, cyclooctanediol or norbornanediol.

Further compounds (3) may be compounds having at least three isocyanate-reactive groups.

For example, these components may have 3 to 6, preferably 3 to 5, more preferably 3 to 4, and very preferably 3 isocyanate-reactive groups.

The molecular weight of these components is generally not more than 2000 g/mol, preferably not more than 1500 g/mol, more preferably not more than 1000 g/mol, and very preferably not more than 500 g/mol.

The urethane (meth)acrylates preferably have a number-average molar weight Mₙ of 500 to 20 000, in particular of 500 to 10 000 and more preferably 600 to 3000 g/mol (determined by gel permeation chromatography using tetrahydrofuran and polystyrene as standard).

Epoxy (meth)acrylates are obtainable by reacting epoxides with (meth)acrylic acid. Examples of suitable epoxides include epoxidized olefins, aromatic glycidyl ethers or aliphatic glycidyl ethers, preferably those of aromatic or aliphatic glycidyl ethers.

Examples of possible epoxidized olefins include ethylene oxide, propylene oxide, isobutylene oxide, 1-butene oxide, 2-butene oxide, vinyloxirane, styrene oxide or epichlorohydrin, preference being given to ethylene oxide, propylene oxide, isobutylene oxide, vinyloxirane, styrene oxide or epichlorohydrin, particular preference to ethylene oxide, propylene oxide or epichlorohydrin, and very particular preference to ethylene oxide and epichlorohydrin.

Aromatic glycidyl ethers are, for example, bisphenol A diglycidyl ether, bisphenol F diglycidyl ether, bisphenol B diglycidyl ether, bisphenol S diglycidyl ether, hydroquinone diglycidyl ether, alkylation products of phenol/dicyclopentadiene, e.g., 2,5-bis[(2,3-epoxypropoxy)phenyl]octahydro-4,7-methano-5H-indene (CAS No. [13446-85-0]), tris[4-(2,3-epoxypropoxy)phenyl]methane isomers (CAS No. [66072-39-7]), phenol-based epoxy novolaks (CAS No. [9003-35-4]), and cresol-based epoxy novolaks (CAS No. [37382-79-9]).

Examples of aliphatic glycidyl ethers include 1,4-butanediol diglycidyl ether, 1,6-hexanediol diglycidyl ether, trimethylolpropane triglycidyl ether, pentaerythritol tetraglycidyl ether, 1,1,2,2-tetrakis[4-(2,3-epoxypropoxy)phenyl]ethane (CAS No. [27043-37-4]), diglycidyl ether of polypropylene glycol (α,ω)-bis(2,3-epoxypropoxy)poly(oxypropylene), CAS No. [16096-30-3]) and of hydrogenated bisphenol A (2,2-bis[4-(2,3-epoxypropoxy)cyclohexyl]propane, CAS No. [13410-58-7]).

The epoxy (meth)acrylates preferably have a number-average molar weight Mₙ of 200 to 20 000, more preferably of 200 to 10 000 g/mol, and very preferably of 250 to 3000 g/mol; the amount of (meth)acrylic groups is preferably 1 to 5, more preferably 2 to 4, per 1000 g of epoxy (meth)acrylate (determined by gel permeation chromatography using polystyrene as standard and tetrahydrofuran as eluent).

Carbonate (meth)acrylates comprise on average preferably 1 to 5, especially 2 to 4, more preferably 2 to 3 (meth)acrylic groups, and very preferably 2 (meth)acrylic groups.

The number-average molecular weight Mₙ of the carbonate (meth)acrylates is preferably less than 3000 g/mol, more preferably less than 1500 g/mol, very preferably less than 800 g/mol (determined by gel permeation chromatography using polystyrene as standard, tetrahydrofuran as solvent).

The carbonate (meth)acrylates are obtainable in a simple manner by transesterifying carbonic esters with polyhydric, preferably dihydric, alcohols (diols, hexanediol for example) and subsequently esterifying the free OH groups with (meth)acrylic acid, or else by transesterification with (meth)acrylic esters, as described for example in EP-A 92 269. They are also obtainable by reacting phosgene, urea derivatives with polyhydric, e.g., dihydric, alcohols.

Also conceivable are (meth)acrylates of polycarbonate polyols, such as the reaction product of one of the aforementioned diols or polyols and a carbonic ester and also a hydroxyl-containing (meth)acrylate.

Examples of suitable carbonic esters include ethylene carbonate, 1,2- or 1,3-propylene carbonate, dimethyl carbonate, diethyl carbonate or dibutyl carbonate.

Examples of suitable hydroxyl-containing (meth)acrylates are 2-hydroxyethyl (meth)acrylate, 2- or 3-hydroxypropyl (meth)acrylate, 1,4-butanediol mono(meth)acrylate, neopentyl glycol mono(meth)acrylate, glyceryl mono- and di(meth)acrylate, trimethylolpropane mono- and di(meth)acrylate, and pentaerythritol mono-, di-, and tri(meth)acrylate.

Particularly preferred carbonate (meth)acrylates are those of the formula: 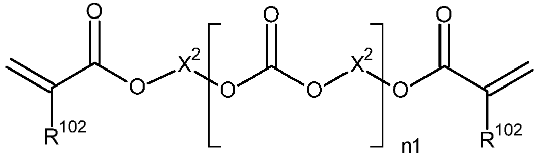 in which R¹⁰² is H or CH₃, X² is a C₂-C₁₈ alkylene group, and n1 is an integer from 1 to 5, preferably 1 to 3.

R¹⁰² is preferably H and X² is preferably C₂ to C₁₀ alkylene, examples being 1,2-ethylene, 1,2-propylene, 1,3-propylene, 1,4-butylene, and 1,6-hexylene, more preferably C₄ to C₈ alkylene. With very particular preference X² is C₆ alkylene.

The carbonate (meth)acrylates are preferably aliphatic carbonate (meth)acrylates.

Among the oligomers (B) urethane (meth)acrylates are particularly preferred.

A urethane (meth)acrylate may refer to a single urethane (meth)acrylate or to a mixture of different urethane (meth)acrylates. Suitable urethane (meth)acrylates can be monofunctional, but preferably are difunctional, or of higher functionality. The functionality refers to the number of (meth)acrylate functional groups exhibited by the compound.

Preferred are urethane (meth)acrylates made from polyetherdiols, or polyester diols, aliphatic, aromatic, or cyclic diisocyanates and hydroxyalkyl (meth)acrylates. More preferred are urethane (meth)acrylates made from polyester diols, aromatic, or cyclic diisocyanates and hydroxyalkyl (meth)acrylates.

The diisocyanates are preferably selected from 4,4'-, 2,4'- and/or 2,2'-methylenedicyclohexyl diisocyanate (H12MDI), isophorone diisocyanates (IPDI) and tolylene 2,4- and/or 2,6-diisocyanate (TDI).

The hydroxyalkyl (meth)acrylates are preferably selected from 2-hydroxyethyl acrylate, 2-hydroxyethyl methacrylate, 2- or 3-hydroxypropyl acrylate, 2- or 3-hydroxypropyl methacrylate, 4-hydroxybutyl methacrylate, and 4-hydroxybutyl acrylate.

Also preferred are urethane (meth)acrylates made from lactones of formula 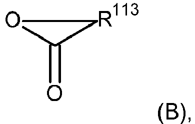 aliphatic, aromatic, or cyclic diisocyanates and hydroxyalkyl (meth)acrylates. More preferred are urethane (meth)acrylates made from caprolactone, aliphatic, or cyclic diisocyanates and hydroxyalkyl (meth)acrylates.

The diisocyanates are preferably selected from di(isocyanatocyclohexyl)methane, 2,2,4-and 2,4,4-trimethylhexane diisocyanate, and especially 4,4'-, 2,4'- and/or 2,2'-methylenedicyclohexyl diisocyanate (H12MDI).

The hydroxyalkyl (meth)acrylates are preferably selected from 2-hydroxyethyl acrylate, 2-hydroxyethyl methacrylate, 2- or 3-hydroxypropyl acrylate, 2- or 3-hydroxypropyl methacrylate, 4-hydroxybutyl methacrylate, and 4-hydroxybutyl acrylate.

Also preferred are those having polyfunctionality of (meth)acrylates or mixed acrylic and methacrylic functionality.

In a preferred embodiment the polyester urethane (meth)acrylate (B) is obtained by reacting
(B1) a hydroxyalkylacrylate, or hydroxyalkylmethacrylate,
(B2) an aliphatic diisocyanate, an aliphatic polyisocyanate, a cycloaliphatic diisocyanate, a cycloaliphatic polyisocyanate, an aromatic diisocyanate, or an aromatic polyisocyanate, or mixtures thereof, especially an aliphatic diisocyanate, cycloaliphatic diisocyanate, or an aromatic diisocyanate, or mixtures thereof,
(B3) a polyester polyol, which is derived from aliphatic dicarboxylic acids and aliphatic diols, and (B4) optionally a secondary polyol, especially glycerol.

The hydroxyalkylacrylate, or hydroxyalkylmethacrylate (B1) is preferably a compound of formula 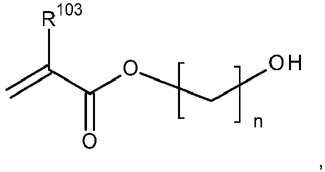 wherein R¹⁰³ is a hydrogen atom, or a methyl group, and n is 2 to 6, especially 2 to 4. Examples of (B1) include 2-hydroxyethyl acrylate, 2-hydroxyethyl methacrylate, 2- or 3-hydroxypropyl acrylate, 2- or 3-hydroxypropyl methacrylate, 4-hydroxybutyl methacrylate and 4-hydroxybutyl acrylate. 2-Hydroxyethyl acrylate is most preferred.

Hydroxyalkylacrylates, or hydroxyalkylmethacrylates (B1) having shorter alkyl chains (n is 2 to 4, especially 2) lead to a higher E modulus of the UV cured composition. Hydroxyalkylmethacrylates (B1) lead to a higher E modulus as compared to hydroxyalkylacrylates.

The organic diisocyanate (B2) used for making the polyester urethane acrylate is either an aliphatic, a cycloaliphatic, or an aromatic diisocyanate.

Examples of customary aliphatic and cycloaliphatic diisocyanates are tri-, tetra-, penta-, hexa-, hepta- and/or octamethylene diisocyanate, 2-methylpentamethylene 1,5-diisocyanate, 2-ethyltetramethylene 1,4-diisocyanate, hexamethylene 1,6-diisocyanate (HDI), pentamethylene 1,5-diisocyanate, butylene 1,4-diisocyanate, trimethylhexamethylene 1,6-diisocyanate, 1-isocyanato-3,3,5-trimethyl-5-isocyanatomethylcyclohexane (isophorone diisocyanate, IPDI), 1,4- and/or 1,3-bis(isocyanatomethyl)cyclohexane (HXDI), 1,4-cyclohexane diisocyanate, 1-methyl-2,4-and/or 1-methyl-2,6-cyclohexane diisocyanate, 4,4'-, 2,4'- and/or 2,2'-methylenedicyclohexyl diisocyanate (H12MDI).

Preferred aliphatic and cycloaliphatic polyisocyanates are hexamethylene 1,6-diisocyanate (HDI), 1-isocyanato-3,3,5-trimethyl-5-isocyanatomethylcyclohexane (isophorone diisocyanate, IPDI) and 4,4'-, 2,4'- and/or 2,2'-methylenedicyclohexyl diisocyanate (H12MDI); particular preference is given to H12MDI and IPDI or mixtures thereof.

Suitable aromatic diisocyanates include naphthylene 1.5- diisocyanate (NDI), tolylene 2,4-and/or 2,6-diisocyanate (TDI), diphenylmethane 2,2'-, 2,4'- and/or 4,4'-diisocyanate (MDI), 3,3'-dimethyl-4,4'-diisocyanato-diphenyl (TODI), p-phenylene diisocyanate (PDI), diphenylethan-4,4'-diisoyanate (EDI), diphenylmethandiisocyanate, 3,3'-dimethyl-diphenyldiisocyanate, 1,2-diphenylethandiisocyanate and/or phenylene diisocyanat.

The at present most preferred diisocyanates are 4,4'-, 2,4'- and/or 2,2'-methylenedicyclohexyl diisocyanate (H12MDI), isophorone diisocyanates (IPDI), or tolylene 2,4- and/or 2,6-diisocyanate (TDI).

Polyester polyols (B3) derived from dicarboxylic acid and diols are preferred and, for example, described in US20160122465. The dicarboxylic acids used for making the polyester polyol include aliphatic, or cycloaliphatic dicarboxylic acids, or combinations thereof. Among them, aliphatic dicarboxylic acids are preferred. Suitable aliphatic dicarboxylic acids which can be used alone or in mixture typically contain from 4 to 12 carbon atoms and include: succinic acid, glutaric acid, adipic acid, pimelic acid, suberic acid, and the like. Adipic acid is preferred.

The diols used for making the polyester polyol include aliphatic, or cycloaliphatic diols, or combinations thereof, preferably aliphatic diols containing 2 to 8 carbon atoms and more preferably 2 to 6 carbon atoms. Some representative examples of aliphatic diols that can be used include ethylene glycol, 1,3-propanediol, 1,4-butanediol, 1,5-pentanediol, 1,6-hexanediol and the like.

In a preferred embodiment, only one kind of aliphatic dicarboxylic acid is used in making the polyester polyol. In another preferred embodiment, one or two kinds of aliphatic diols are used in making the polyester polyol. Most preferably, the polyester polyol is derived from adipic acid and ethylene glycol and 1,4-butanediol (polyethylene 1,4-butylene adipate) diol, PEBA). In the PEBA, the molar ratio of ethylene glycol to 1,4-butanediol is from 0.05:1 to 10:1, preferably from 0.2:1 to 5:1, more preferably 0.5:1 to 1.5:1, most preferred from 0.75: 1 to 1.25: 1.

The linear polyester polyol will typically have a number average molecular weight within the range of 4 ×10² to 7.0 × 10³, preferably 8 ×10² to 6.0 × 10³, more preferably 1×10³ to 5.0 × 10³. In a preferred embodiment, the linear polyol is polyester polyol derived from one kind of aliphatic dicarboxylic acid and two kinds of aliphatic diols and has a number average molecular weight of from 2.0 × 10³ to 4.0 × 10³. In another preferred embodiment, the linear polyol is polyester polyol derived from one kind of aliphatic dicarboxylic acid and one kind of aliphatic diol and has a number average molecular weight of from 1.5 × 10³ to 4.0 × 10³, and more preferably from 1.8 × 10³ to 3.5 × 10³. All molecular weights specified in this text have the unit of [g/mol] and refer, unless indicated otherwise, to the number average molecular weight (Mn).

The polyester urethane acrylates, or methacrylates (A) have viscosities in the range of 2000 to 20000 mPas at 60 °C.

A secondary polyol, such as, for example, glycerol, may be used, to finetune the mechanical properties of the inventive urethane (meth)acrylates by introducing linear or branched structural elements.

In another preferred embodiment the polyester urethane (meth)acrylate (B) is obtained by reacting a hydroxyalkyl(meth)acrylate of formula 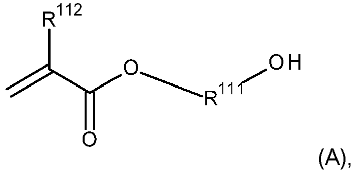 with a lactone of formula 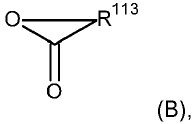 and at least one cycloaliphatic or asymmetric aliphatic diisocyanate, wherein R¹¹¹ is a divalent alkylene radical having 2 to 12 carbon atoms and which may optionally be substituted by C₁-C₄alkyl groups and/or interrupted by one or more oxygen atoms, R¹¹² in each case independently of any other is methyl or hydrogen, R¹¹³ is a divalent alkylene radical having 1 to 12 carbon atoms and which may optionally be substituted by C₁ to C₄ alkyl groups and/or interrupted by one or more oxygen atoms. Reference is made to WO14191228A1

R¹¹¹ is preferably selected from the group consisting of 1,2-ethylene, 1,2- or 1,3-propylene, 1,2-, 1,3-, or 1,4-butylene, 1,1-dimethyl-1,2-ethylene, 1,2-dimethyl-1,2-ethylene, 1,5-pentylene, 1,6-hexylene, 1,8-octylene, 1,10-decylene, and 1,12-dodecylene.

R¹¹³ is preferably selected from the group consisting of methylene, 1,2-ethylene, 1,2-propylene, 1,3-propylene, 1,2-butylene, 1,3-butylene, 1,4-butylene, 1,5-pentylene, 1,5-hexylene, 1,6-hexylene, 1,8-octylene, 1,10-decylene, 1,12-dodecylene, 2-oxa-1,4-butylene, 3-oxa-1,5-pentylene, and 3-oxa-1,5-hexylene.

The hydroxyalkyl(meth)acrylate of formula (A) is preferably selected from the group consisting of 2-hydroxyethyl(meth)acrylate, 2- or 3-hydroxypropyl(meth)acrylate, 1,4-butanediol mono(meth)acrylate, neopentyl glycol mono(meth)acrylate, 1,5-pentanediol mono(meth)acrylate, and 1,6-hexanediol mono(meth)acrylate.

The lactone of formula 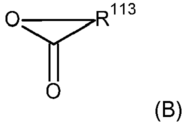 is preferably selected from the group consisting of β-propiolactone, γ-butyrolactone, γ-ethyl-gamma-butyrolactone, γ-valerolactone, delta-valerolactone, ε-caprolactone, 7-methyloxepan-2-one, 1,4-dioxepan-5-one, oxacyclotridecan-2-one, and 13-butyl-oxacyclotridecan-2-one.

Cycloaliphatic diisocyanates are 1,4-, 1,3-, or 1,2-diisocyanatocyclohexane, 4,4'-, 2,4'-and/or 2,2'-methylenedicyclohexyl diisocyanate (H12MDI), bis(isocyanatomethyl)bicyclo[2.2.1]heptane (NBDI), 1-isocyanato-3,3,5-trimethyl-5-(isocyanatomethyl)cyclohexane(isophorone diisocyanate), 1,3- or 1,4-bis(isocyanatomethyl)cyclohexane or 2,4- or 2,6-diisocyanato-1-methylcyclohexane, and also 3(or 4),8(or 9)-bis(isocyanatomethyl)tricyclo[5.2.1.02.6]decane isomer mixtures.

Asymmetric aliphatic diisocyanates are derivatives of lysine diisocyanate, or tetramethylxylylene diisocyanate, trimethylhexane diisocyanate, or tetramethylhexane diisocyanate.

Very particular preference is given to di(isocyanatocyclohexyl)methane, 2,2,4- and 2,4,4-trimethylhexane diisocyanate, and especially 4,4'-, 2,4'- and/or 2,2'-methylenedicyclohexyl diisocyanate (H12MDI).

The urethane (meth)acrylates can be in particular produced by reacting ε-caprolactone, 4,4'-, 2,4'- and/or 2,2'-methylenedicyclohexyl diisocyanate (H12MDI) and hydroxyethylacrylate.

In another preferred embodiment the polyester urethane (meth)acrylate (B) is obtained by reacting a polyalkylene glycol with a lactone of formula 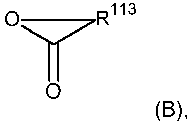 at least one cycloaliphatic or asymmetric aliphatic diisocyanate, and an hydroxyalkyl(meth)acrylate of formula (A).

The hydroxyalkyl(meth)acrylate of formula (A) is preferably selected from the group consisting of 2-hydroxyethyl(meth)acrylate, 2- or 3-hydroxypropyl(meth)acrylate, 1,4-butanediol mono(meth)acrylate, neopentyl glycol mono(meth)acrylate, 1,5-pentanediol mono(meth)acrylate, and 1,6-hexanediol mono(meth)acrylate.

The urethane (meth)acrylates can be in particular produced by reacting a polyalkylene glycol, preferably a polyethylene glycol, with ε-caprolactone, 4,4'-, 2,4'- and/or 2,2'-methylenedicyclohexyl diisocyanate (H12MDI) and hydroxyethylacrylate.

### Diluent (C)

The second diluent (C) represents a "reactive diluent", which is a component that contains at least one free radically reactive group (e.g., an ethylenically-unsaturated group) that can co-react with components (A) and (B) (e.g., is capable of undergoing addition polymerization).

The diluent (C) may be a single diluent, or a mixture of two, or more diluents.

Suitable monofunctional, difunctional, or tetrafunctional acrylate, methacrylate, or vinylamide components are listed below. Monofunctional refers to the fact that the molecule of the compound exhibits only one acrylate, methacrylate, or vinylamide functional group.

Examples of monofunctional vinylamide components include such as N-vinyl-pyrrolidone, vinyl-imidazole, N-vinylcaprolactame, N-(hydroxymethyl)vinylamide, N-hydroxyethyl vinylamide, N-isopropylvinylamide, N-isopropylmethvinylamide, N-tert-butylvinylamide, N,N'-methylenebisvinylamide, N-(isobutoxymethyl)vinylamide, N-(butoxymethyl)vinylamide, N-[3-(dimethylamino)propyl]methvinylamide, N,N-dimethylvinylamide, N,N-diethylvinylamide and N-methyl-N-vinylacetamide.

Examples of monofunctional methacrylate include isobornyl methacrylate, tetrahydrofurfuryl methacrylate, ethoxylated phenyl methacrylate, cyclohexylmethacrylate, lauryl methacrylate, stearyl methacrylate, octyl methacrylate, isodecyl methacrylate, tridecyl methacrylate, caprolactone methacrylate, nonyl phenol methacrylate, cyclic trimethylolpropane formal methacrylate, methoxy polyethyleneglycol methacrylates, methoxy polypropyleneglycol methacrylates, hydroxyethyl methacrylate, hydroxypropyl methacrylate and glycidyl methacrylate.

The photocurable composition of the present invention may contain a difunctional, or tetrafunctional diluent having two unsaturated carbon-carbon bonds, such as, for example, difunctional, or tetrafunctional (meth)acrylates.

Examples of the bifunctional monomer include 1,3-butylene glycol di(meth)acrylate, 1,4-butanediol di(meth)acrylate, ethylene glycol di(meth)acrylate, diethylene glycol di(meth)acrylate, triethylene glycol di(meth)acrylate, 1,6-hexanediol di(meth)acrylate, polyethylene glycol (200) di(meth)acrylate, tetraethylene glycol di(meth)acrylate, triethylene glycol di(meth)acrylate, tripropylene glycol di(meth)acrylate, polyethylene glycol (400) di(meth)acrylate, ethoxylated (3) bisphenol A di(meth)acrylate, dipropylene glycol di(meth)acrylate, alkoxylated hexanediol di(meth)acrylate, ethoxylated (4) bisphenol A di(meth)acrylate, ethoxylated (10) bisphenol A di(meth)acrylate, polyethylene glycol (600) di(meth)acrylate, tricyclodecane dimethanol di(meth)acrylate, 1,9-nonanediol di(meth)acrylate, 1,10-decanediol di(meth)acrylate, neopentyl glycol di(meth)acrylate, polytetramethylene glycol di(meth)acrylate, 3-methyl-1,5-pentanediol di(meth)acrylate, and dimethylol-tricyclodecane di(meth)acrylate. One of these may be used alone, or two or more of these may be used in combination.

Polyethylene glycol (200) diacrylate, polyethylene glycol (400) diacrylate, and polyethylene glycol (600) diacrylate mentioned above are represented by the chemical formulae below.

### Polyethylene glycol (200) diacrylate

CH₂=CH-CO-(OC₂H₄)ₙ-OCOCH=CH₂

where n≈4

### Polyethylene glycol (400) diacrylate

CH₂=CH-CO-(OC₂H₄)ₙ-OCOCH=CH₂

where n≈14

### Polyethylene glycol (600) diacrylate

CH₂=CH-CO-(OC₂H₄)ₙ-OCOCH=CH₂

where n≈14

Examples of tetrafunctional (meth)acrylates are bistrimethylolpropane tetraacrylate, pentaerythritol tetracrylate, tetramethylolmethane tetramethacrylate, pentaerythritol tetramethacrylate, bistrimethylolpropane tetramethacrylate, ethoxylated pentaerythritol tetraacrylate, propoxylated pentaerythritol tetraacrylate, dipentaerythritol tetraacrylate, ethoxylated dipentaerythritol tetraacrylate, propoxylated dipentaerythritol tetraacrylate, aryl urethane tetraacrylates, aliphatic urethane tetraacrylates, melamine tetraacrylates, epoxy novolac tetraacrylates and polyester tetraacrylates.

The photocurable composition of the present invention may contain monofunctional acrylamides or methacrylamides. Examples include acryloylmorpholine, methacryloylmorpholine, N-(hydroxymethyl)acrylamide, N-hydroxyethyl acrylamide, N-isopropylacrylamide, N-isopropylmethacrylamide, N-tert-butylacrylamide, N,N'-methylenebisacrylamide, N-(isobutoxymethyl)acrylamide, N-(butoxymethyl)acrylamide, N-[3-(dimethylamino)propyl]methacrylamide, N,N-dimethylacrylamide, N,N-diethylacrylamide, N-(hydroxymethyl)methacrylamide, N-hydroxyethyl methacrylamide, N-isopropylmethacrylamide, N-isopropylmethmethacrylamide, N-tert-butylmethacrylamide, N,N'-methylenebismethacrylamide, N-(isobutoxymethyl)methacrylamide, N-(butoxymethyl)methacrylamide, N-[3-(dimethylamino)propyl]methmethacrylamide, N,N-dimethylmethacrylamide and N,N-diethylmethacrylamide.

### Photoinitiator (D)

The photoinitiator (D) may be a single compound, or a mixture of compounds.

Examples of photoinitiators (D) are known to the person skilled in the art and for example published by Kurt Dietliker in "A compilation of photoinitiators commercially available for UV today", Sita Technology Textbook, Edinburgh, London, 2002.

Examples of suitable acylphosphine oxide compounds are of the formula XII 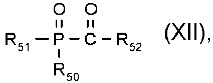 wherein
R₅₀ is unsubstituted cyclohexyl, cyclopentyl, phenyl, naphthyl or biphenylyl; or is cyclohexyl, cyclopentyl, phenyl, naphthyl or biphenylyl substituted by one or more halogen, C₁-C₁₂alkyl, C₁-C₁₂alkoxy, C₁-C₁₂alkylthio or by NR₅₃R₅₄;
or R₅₀ is unsubstituted C₁-C₂₀alkyl or is C₁-C₂₀alkyl which is substituted by one or more halogen, C₁-C₁₂alkoxy, C₁-C₁₂alkylthio, NR₅₃R₅₄ or by -(CO)-O-C₁-C₂₄alkyl;
R₅₁ is unsubstituted cyclohexyl, cyclopentyl, phenyl, naphthyl or biphenylyl; or is cyclohexyl, cyclopentyl, phenyl, naphthyl or biphenylyl substituted by one or more halogen, C₁-C₁₂alkyl, C₁-C₁₂alkoxy, C₁-C₁₂alkylthio or by NR₅₃R₅₄; or R₅₁ is -(CO)R'₅₂; or R₅₁ is C₁-C₁₂alkyl which is unsubstituted or substituted by one or more halogen, C₁-C₁₂alkoxy, C₁-C₁₂alkylthio, or by NR₅₃R₅₄;
R₅₂ and R'₅₂ independently of each other are unsubstituted cyclohexyl, cyclopentyl, phenyl, naphthyl or biphenylyl, or are cyclohexyl, cyclopentyl, phenyl, naphthyl or biphenylyl substituted by one or more halogen, C₁-C₄alkyl or C₁-C₄alkoxy; or R₅₂ is a 5- or 6-membered heterocyclic ring comprising an S atom or N atom;
R₅₃ and R₅₄ independently of one another are hydrogen, unsubstituted C₁-C₁₂alkyl or C₁-C₁₂alkyl substituted by one or more OH or SH wherein the alkyl chain optionally is interrupted by one to four oxygen atoms; or R₅₃ and R₅₄ independently of one another are C₂-C₁₂-alkenyl, cyclopentyl, cyclohexyl, benzyl or phenyl;

In a particularly preferred embodiment the photoinitiator (C) is a compound of the formula (XII), such as, for example, bis(2,4,6-trimethylbenzoyl)-phenylphosphine oxide; 2,4,6-trimethylbenzoyl-diphenyl-phosphine oxide; ethyl (2,4,6 trimethylbenzoyl phenyl) phosphinic acid ester; (2,4,6-trimethylbenzoyl)-2,4-dipentoxyphenylphosphine oxide and bis(2,6-dimethoxybenzoyl)-2,4,4-trimethylpentylphosphine oxide.

Interesting further are mixtures of the compounds of the formula (XII) with compounds of the formula (XI) as well as mixtures of different compounds of the formula (XII).

Examples are mixtures of bis(2,6-dimethoxybenzoyl)-2,4,4-trimethylpentylphosphine oxide with 1-hydroxy-cyclohexyl-phenyl-ketone, of bis(2,4,6-trimethylbenzoyl)-phenylphosphine oxide with 2-hydroxy-2-methyl-1-phenyl-propan-1-one, of bis(2,4,6-trimethylbenzoyl)-phenylphosphine oxide with ethyl (2,4,6 trimethylbenzoyl phenyl) phosphinic acid ester, etc.

Examples of suitable benzophenone compounds are compounds of the formula 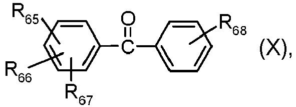 wherein
R₆₅, R₆₆ and R₆₇ independently of one another are hydrogen, C₁-C₄alkyl, C₁-C₄-halogenalkyl, C₁-C₄alkoxy, Cl or N(C₁-C₄alkyl)₂;
R₆₈ is hydrogen, C₁-C₄alkyl, C₁-C₄halogenalkyl, phenyl, N(C₁-C₄alkyl)₂, COOCH₃, 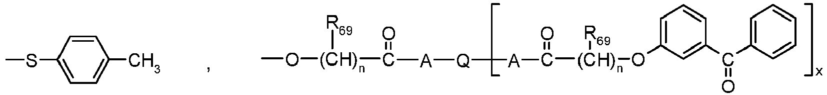 or 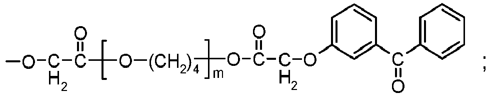
Q is a residue of a polyhydroxy compound having 2 to 6 hydroxy groups;
x is a number greater than 1 but no greater than the number of available hydroxyl groups in Q;
A is -[O(CH₂)_{b}CO]_{y}- or -[O(CH₂)_{b}CO]_{(y-1)}-[O(CHR₆₉CHR_{69'})ₐ]_{y}- ;
R₆₉ and R_{69'} independently of one another are hydrogen, methyl or ethyl; and if n (or a) is greater than 1 the radicals R₆₉ may be the same as or different from each other;
a is a number from 1 to 2;
b is a number from 4 to 5;
y is a number from 1 to 10;
n is ; and
m is an integer 2-10.

Specific examples are benzophenone, Esacure TZT^{®} available from IGM, (a mixture of 2,4,6-trimethylbenzophenone and 4-methylbenzophenone), 4-phenylbenzophenone, 4-methoxybenzophenone, 4,4'-dimethoxybenzophenone, 4,4'-dimethylbenzophenone, 4,4'-dichlorobenzophenone, 4,4'-dimethylaminobenzophenone, 4,4'-diethylaminobenzophenone, 4-methylbenzophenone, 2,4,6-trimethylbenzophenone, 4-(4-methylthiophenyl)benzophenone, 3,3'-dimethyl-4-methoxybenzophenone, methyl-2-benzoylbenzoate, 4-(2-hydroxyethylthio)benzophenone, 4-(4-tolylthio)benzophenone, 4-benzoyl-N,N,N-trimethylbenzenemethanaminium chloride, 2-hydroxy-3-(4-benzoylphenoxy)-N,N,N-trimethyl-1-propanaminium chloride monohydrate, 4-(13-acryloyl-1,4,7,10,13-pentaoxatridecyl)benzophenone, 4-benzoyl-N,N-dimethyl-N-[2-(1-oxo-2-propenyl)oxy]ethylbenzenemethanaminium chloride; [4-(2-hydroxy-ethylsulfanyl)-phenyl]-(4-isopropylphenyl)-methanone; biphenyl-[4-(2-hydroxy-ethylsulfanyl)-phenyl]-methanone; biphenyl-4-yl-phenyl-methanone; biphenyl-4-yl-p-tolyl-methanone; biphenyl-4-yl-m-tolyl-methanone; [4-(2-hydroxy-ethylsulfanyl)-phenyl]-p-tolyl-methanone; [4-(2-hydroxy-ethylsulfanyl)-phenyl]-(4-isopropyl-phenyl)-methanone; [4-(2-hydroxy-ethylsulfanyl)-phenyl]-(4-methoxy-phenyl)-methanone; 1-(4-benzoyl-phenoxy)-propan-2-one; [4-(2-hydroxy-ethylsulfanyl)-phenyl]-(4-phenoxy-phenyl)-methanone; 3-(4-benzoyl-phenyl)-2-dimethylamino-2-methyl-1-phenyl-propan-1-one; (4-chloro-phenyl)-(4-octylsulfanyl-phenyl)-methanone; (4-chloro-phenyl)-(4-dodecylsulfanyl-phenyl)-methanone; (4-bromo-phenyl)-(4-octylsulfanyl-phenyl)-methanone; (4-dodecylsulfanyl-phenyl)-(4-methoxy-phenyl)-methanone; (4-benzoyl-phenoxy)-acetic acid methyl ester; biphenyl-[4-(2-hydroxy-ethylsulfanyl)-phenyl]-methanone; 1-[4-(4-benzoylphenylsulfanyl)phenyl]-2-methyl-2-(4-methylphenylsulfonyl)propan-1-one (Esacure^{®}1001 available from IGM).

Examples of suitable alpha-hydroxy ketone, alpha-alkoxyketone or alpha-aminoketone compounds are of the formula 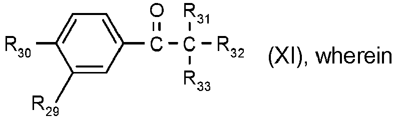
R₂₉ is hydrogen or C₁-C₁₈alkoxy;
R₃₀ is hydrogen, C₁-C₁₈alkyl, C₁-C₁₂hydroxyalkyl ,C₁-C₁₈alkoxy, OCH₂CH₂-OR₃₄, morpholino, S-C₁-C₁₈alkyl, a group -HC=CH₂, -C(CH₃)=CH₂, 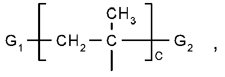 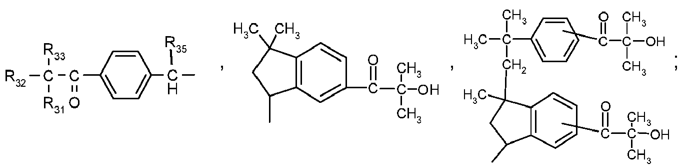 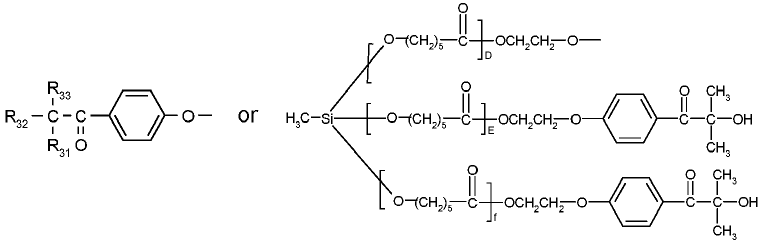
D, E and fare 1-3;
c is 2-10;
G₁ and G₂ independently of one another are end groups of the polymeric structure, preferably hydrogen or methyl;
R34 is hydrogen, 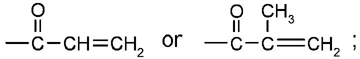
R₃₁ is hydroxy, C₁-C₁₆alkoxy, morpholino, dimethylamino or -O(CH₂CH₂O)_{g}-C₁-C₁₆alkyl;
g is 1-20;
R₃₂ and R₃₃ independently of one another are hydrogen, C₁-C₆alkyl, C₁-C₁₆alkoxy or -O(CH₂CH₂O)_{g}-C₁-C₁₆alkyl; or are unsubstituted phenyl or benzyl; or phenyl or benzyl substituted by C₁-C₁₂-alkyl; or R₃₂ and R₃₃ together with the carbon atom to which they are attached form a cyclohexyl ring;
R₃₅ is hydrogen, OR₃₆ or NR₃₇R₃₈;
R₃₆ is hydrogen, C₁-C₁₂alkyl which optionally is interrupted by one or more non-consecutive O-atoms and which uninterrupted or interrupted C₁-C₁₂alkyl optionally is substituted by one or more OH,
or R₃₆ is 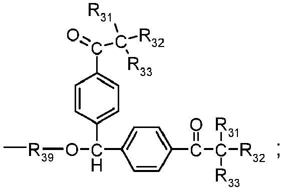
R₃₇ and R₃₈ independently of each other are hydrogen or C₁-C₁₂alkyl which is unsubstituted or is substituted by one or more OH;
R₃₉ is C₁-C₁₂alkylene which optionally is interrupted by one or more non-consecutive O, - (CO)-NH-C₁-C₁₂alkylene-NH-(CO)- or 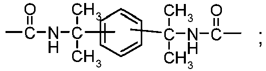 with the proviso that R₃₁, R₃₂ and R₃₃ not all together are C₁-C₁₆alkoxy or -O(CH₂CH₂O)_{g}-C₁-C₁₆alkyl.

Specific examples are 1-hydroxy-cyclohexyl-phenyl-ketone or a mixture of 1-hydroxy-cyclohexyl-phenyl-ketone with benzophenone), 2-methyl-1[4-(methylthio)phenyl]-2-morpholinopropan-1-one, 2-benzyl-2-dimethylamino-1-(4-morpholinophenyl)-butan-1-one, 2-dimethylamino-2-(4-methyl-benzyl)-1-(4-morpholin-4-yl-phenyl)-butan-1-one, (3,4-dimethoxy-benzoyl)-1-benzyl-1-dimethylamino propane, 1-[4-(2-hydroxyethoxy)-phenyl]-2-hydroxy-2-methyl-1-propan-1-one, 2,2-dimethoxy-1,2-diphenylethan-1-one, 2-hydroxy-2-methyl-1-phenyl-propan-1-one, 2-hydroxy-1-{4-[4-(2-hydroxy-2-methyl-propionyl)-benzyl]-phenyl}-2-methyl-propan-1-one, 2-hydroxy-1-{4-[4-(2-hydroxy-2-methyl-propionyl)-phenoxy]-phenyl}-2-methyl-propan-1-one, Esacure KIP provided by IGM, 2-hydroxy-1-{1-[4-(2-hydroxy-2-methyl-propionyl)-phenyl]-1,3,3-trimethyl-indan-5-yl}-2-methyl-propan-1-one.

Examples of suitable phenylglyoxylate compounds are of the formula 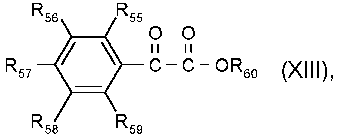 wherein
R₆₀ is hydrogen, C₁-C₁₂alkyl or 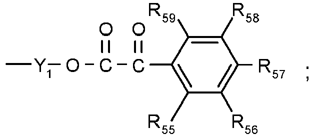
R₅₅, R₅₆, R₅₇, R₅₈ and R₅₉ independently of one another are hydrogen, unsubstituted C₁-C₁₂alkyl or C₁-C₁₂alkyl substituted by one or more OH, C₁-C₄alkoxy, phenyl, naphthyl, halogen or by CN; wherein the alkyl chain optionally is interrupted by one or more oxygen atoms; or R₅₅, R₅₆, R₅₇, R₅₈ and R₅₉ independently of one another are C₁-C₄alkoxy, C₁-C₄alkythio or NR₅₂R_{53;}
R₅₂ and R₅₃ independently of one another are hydrogen, unsubstituted C₁-C₁₂alkyl or C₁-C₁₂alkyl substituted by one or more OH or SH wherein the alkyl chain optionally is interrupted by one to four oxygen atoms; or R₅₂ and R₅₃ independently of one another are C₂-C₁₂-alkenyl, cyclopentyl, cyclohexyl, benzyl or phenyl; and
Y₁ is C₁-C₁₂alkylene optionally interrupted by one or more oxygen atoms.

Specific examples of the compounds of the formula XIII are oxo-phenyl-acetic acid 2-[2-(2-oxo-2-phenyl-acetoxy)-ethoxy]-ethyl ester (Irgacure^{®}754), methyl α-oxo benzeneacetate.

Examples of suitable oxime ester compounds are of the formula 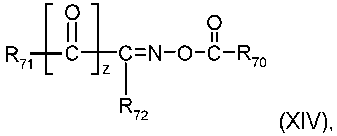 wherein
z is 0 or 1;
R₇₀ is hydrogen, C₃-C₈cycloalkyl; C₁-C₁₂alkyl which is unsubstituted or substituted by one or more halogen, phenyl or by CN; or R₇₀ is C₂-C₅alkenyl; phenyl which is unsubstituted or substituted by one or more C₁-C₆alkyl, halogen, CN, OR₇₃, SR₇₄ or by NR₇₅R₇₆; or R₇₀ is C₁-C₈alkoxy, benzyloxy; or phenoxy which is unsubstituted or substituted by one or more C₁-C₆alkyl or by halogen;
R₇₁ is phenyl, naphthyl, benzoyl or naphthoyl, each of which is substituted by one or more halogen, C₁-C₁₂alkyl, C₃-C₈cycloalkyl, benzyl, phenoxycarbonyl, C₂-C₁₂alkoxycarbonyl, OR₇₃, SR₇₄, SOR₇₄, SO₂R₇₄ or by NR₇₅R₇₆, wherein the substituents OR₇₃, SR₇₄ and NR₇₅R₇₆ optionally form 5- or 6-membered rings *via* the radicals R₇₃, R₇₄, R₇₅ and/or R₇₆ with further substituents on the phenyl or naphthyl ring; or each of which is substituted by phenyl or by phenyl which is substituted by one or more OR₇₃, SR₇₄ or by NR₇₅R₆₆;
or R₇₁ is thioxanthyl, or 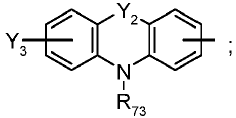
R₇₂ is hydrogen; unsubstituted C₁-C₂₀alkyl or C₁-C₂₀alkyl which is substituted by one or more halogen, OR₇₃, SR₇₄, C₃-C₈cycloalkyl or by phenyl; or is C₃-C₈cycloalkyl; or is phenyl which is unsubstituted or substituted by one or more C₁-C₆alkyl, phenyl, halogen, OR₇₃, SR₇₄ or by NR₇₅R₇₆; or is C₂-C₂₀alkanoyl or benzoyl which is unsubstituted or substituted by one or more C₁-C₆alkyl, phenyl, OR₇₃, SR₇₄ or by NR₇₅R₇₆; or is C₂-C₁₂alkoxycarbonyl, phenoxycarbonyl, CN, CONR₇₅R₇₆, NO₂, C₁-C₄haloalkyl, S(O)_{y}-C₁-C₆alkyl, or S(O)_{y}-phenyl, y is 1 or 2;
Y₂ is a direct bondor no bond;
Y₃ is NO₂ or 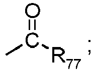
R₇₃ and R₇₄ independently of one another are hydrogen, C₁-C₂₀alkyl, C₂-C₁₂alkenyl, C₃-C₈cycloalkyl, C₃-C₈cycloalkyl which is interrupted by one or more, preferably 2, O, phenyl-C₁-C₃alkyl; or are C₁-C₈alkyl which is substituted by OH, SH, CN, C₁-C₈alkoxy, C₁-C₈alkanoyl, C₃-C₈cycloalkyl, by C₃-C₈cycloalkyl which is interrupted by one or more O, or which C₁-C₈alkyl is substituted by benzoyl which is unsubstituted or substituted by one or more C₁-C₆alkyl, halogen, OH, C₁-C₄alkoxy or by C₁-C₄alkylsulfanyl; or are phenyl or naphthyl, each of which is unsubstituted or substituted by halogen, C₁-C₁₂alkyl, C₁-C₁₂alkoxy, phenyl-C₁-C₃alkyloxy, phenoxy, C₁-C₁₂alkylsulfanyl, phenylsulfanyl, N(C₁-C₁₂alkyl)₂, diphenylamino or by 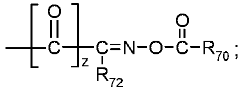
R₇₅ and R₇₆ independently of each other are hydrogen, C₁-C₂₀alkyl, C₂-C₄hydroxyalkyl, C₂-C₁₀alkoxyalkyl, C₂-C₅alkenyl, C₃-C₈cycloalkyl, phenyl-C₁-C₃alkyl, C₁-C₈alkanoyl, C₃-C₁₂alkenoyl, benzoyl; or are phenyl or naphthyl, each of which is unsubstituted or substituted by C₁-C₁₂alkyl, benzoyl or by C₁-C₁₂alkoxy; or R₇₅ and R₇₆ together are C₂-C₆alkylene optionally interrupted by O or NR₇₃ and optionally are substituted by hydroxyl, C₁-C₄alkoxy, C₂-C₄alkanoyloxy or by benzoyloxy;
R₇₇ is C₁-C₁₂alkyl, thienyl or phenyl which is unsubstituted or substituted by C₁-C₁₂alkyl, OR₇₃, morpholino or by N-carbazolyl.

Specific examples are 1,2-octanedione 1-[4-(phenylthio)phenyl]-2-(O-benzoyloxime), ethanone 1-[9-ethyl-6-(2-methylbenzoyl)-9H-carbazol-3-yl]-1-(O-acetyloxime), 9H-thioxanthene-2-carboxaldehyde 9-oxo-2-(O-acetyloxime), ethanone 1-[9-ethyl-6-(4morpholinobenzoyl)-9H-carbazol-3-yl]-1-(O-acetyloxime), ethanone 1-[9-ethyl-6-(2-methyl-4-(2-(1,3-dioxo-2-dimethyl-cyclopent-5-yl)ethoxy)-benzoyl)-9H-carbazol-3-yl]-1-(O-acetyloxime) (Adeka N-1919), ethanone 1-[9-ethyl-6-nitro-9H-carbazol-3-yl]-1-[2-methyl-4-(1-methyl-2-methoxy)ethoxy)phenyl]-1-(O-acetyloxime) (Adeka NCI831), etc.

It is also possible to add cationic photoinitiators, such as benzoyl peroxide (other suitable peroxides are described in US 4 950 581, column 19, lines 17-25), or aromatic sulfonium, phosphonium or iodonium salts, such as are described, for example, in US 4 950 581, column 18, line 60 to column 19, line 10.

Suitable sulfonium salt compounds are of formula 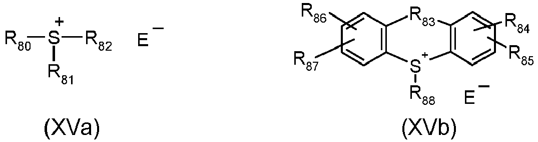 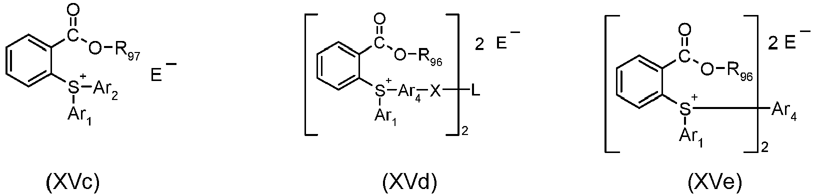 wherein
R₈₀, R₈₁ and R₈₂ are each independently of the others unsubstituted phenyl, or phenyl substituted by -S-phenyl, 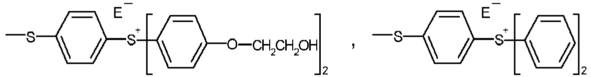 or by 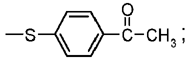
R₈₃ is a direct bond, S, O, CH₂, (CH₂)₂, CO or NR₈₉;
R₈₄, R₈₅, R₈₆ and R₈₇ independently of one another are hydrogen, C₁-C₂₀alkyl, C₃-C₈cycloalkyl, C₁-C₂₀alkoxy, C₂-C₂₀alkenyl, CN, OH, halogen, C₁-C₆alkylthio, phenyl, naphthyl, phenyl-C₁-C₇alkyl, naphtyl-C₁-C₃alkyl, phenoxy, naphthyloxy, phenyl-C₁-C₇alkyloxy, naphtyl-C₁-C₃alkyloxy, phenyl-C₂-C₆alkenyl, naphthyl-C₂-C₄alkenyl, S-phenyl, (CO)R₈₉, O(CO)R₈₉, (CO)OR₈₉, SO₂R₈₉ or OSO₂R₈₉;
R₈₈ is C₁-C₂₀alkyl, C₁-C₂₀hydroxyalkyl, 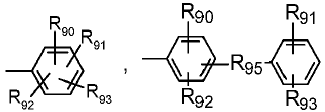 or 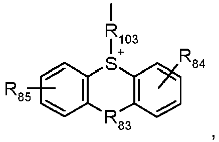
R₈₉ is hydrogen, C₁-C₁₂alkyl, C₁-C₁₂hydroxyalkyl, phenyl, naphthyl or biphenylyl;
R₉₀, R₉₁, R₉₂ and R₉₃ independently of one another have one of the meanings as given for R₈₄; or R₉₀ and R₉₁ are joined to form a fused ring system with the benzene rings to which they are attached;
R₉₅ is a direct bond, S, O or CH₂;
R₉₆ is hydrogen, C₁-C₂₀alkyl; C₂-C₂₀alkyl interrupted by one or more O; or is -L-M-R₉₈ or -L-R₉₈;
R₉₇ has one of the meanings as given for R₉₆ or is 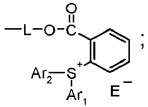
R₉₈ is a monovalent sensitizer or photoinitiator moiety;
Ar₁ and Ar₂ independently of one another are phenyl unsubstituted or substituted by C₁-C₂₀lkyl, halogen or OR₉₉;
or are unsubstituted naphthyl, anthryl, phenanthryl or biphenylyl;
or are naphthyl, anthryl, phenanthryl or biphenylyl substituted by C₁-C₂₀alkyl, OH or OR₉₉;
or are -Ar₄-A₁-Ar₃ or 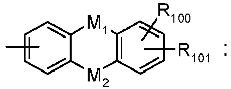
Ar₃ is unsubstituted phenyl, naphthyl, anthryl, phenanthryl or biphenylyl;
or is phenyl, naphthyl, anthryl, phenanthryl or biphenylyl substituted by C₁-C₂₀alkyl, OR₉₉ or benzoyl;
Ar₄ is phenylene, naphthylene, anthrylene or phenanthrylene;
A₁ is a direct bond, S, O or C₁-C₂₀alkylene;
X is CO, C(O)O, OC(O), O, S or NR₉₉ ;
L is a direct bond, S, O, C₁-C₂₀alkylene or C₂-C₂₀alkylene interrupted by one or more non-consecutive O;
R₉₉ is C₁-C₂₀alkyl or C₁-C₂₀hydroxyalkyl; or is C₁-C₂₀alkyl substituted by O(CO)R₁₀₂;
M₁ is S, CO or NR₁₀₀;
M₂ is a direct bond, CH₂, O or S;
R₁₀₀ and R₁₀₁ independently of one another are hydrogen, halogen, C₁-C₈alkyl, C₁-C₈alkoxy or phenyl;
R₁₀₂ is C₁-C₂₀alkyl;
R₁₀₃ is 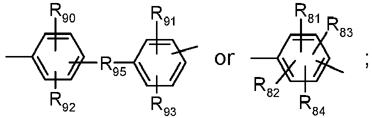 and
E is an anion, especially PF₆, SbF₆, AsF₆, BF₄, (C₆F₅)₄B, Cl, Br, HSO₄, CF₃-SO₃, F-SO₃, 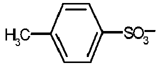 , CH₃-SO₃, CIO₄, PO₄, NO₃, SO₄, CH₃-SO₄, or

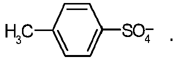

Specific examples of sulfonium salt compounds are for example Irgacure^{®}270 (BASF SE); Cyracure^{®} UVI-6990, Cyracure^{®}UVI-6974 (Union Carbide), Degacure^{®}KI 85 (Degussa), SP-55, SP-150, SP-170 (Asahi Denka), GE UVE 1014 (General Electric), SarCat^{®} KI-85 (= triarylsulfonium hexafluorophosphate; Sartomer), SarCat^{®} CD 1010 (= mixed triarylsulfonium hexafluoroantimonate; Sartomer); SarCat^{®} CD 1011(= mixed triarylsulfonium hexafluorophosphate; Sartomer),

Suitable iodonium salt compounds are of formula 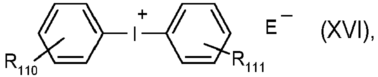 wherein
R₁₁₀ and R₁₁₁ are each independently of the other hydrogen, C₁-C₂₀alkyl, C₁-C₂₀alkoxy, OH-substituted C₁-C₂₀alkoxy, halogen, C₂-C₁₂alkenyl, C₃-C₈cycloalkyl, especially methyl, isopropyl or isobutyl; and
E is an anion, especially PF₆, SbF₆, AsF₆, BF₄, (C₆F₅)₄B, Cl, Br, HSO₄, CF₃-SO₃, F-SO₃, 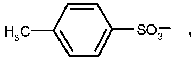 CH₃-SO₃, CIO₄, PO₄, NO₃, SO₄, CH₃-SO₄ or

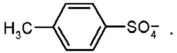

Specific examples of iodonium salt compounds are e.g. tolylcumyliodonium tetrakis(pentafluorophenyl)borate, 4-[(2-hydroxy-tetradecyloxy)phenyl]phenyliodonium hexafluoroantimonate or hexafluorophosphate, tolylcumyliodonium hexafluorophosphate, 4-isopropylphenyl-4'-methylphenyliodonium hexafluorophosphate, 4-isobutylphenyl-4'-methylphenyliodonium hexafluorophosphate (Irgacure^{®}250, BASF SE), 4-octyloxyphenyl-phenyliodonium hexafluorophosphate or hexafluoroantimonate, bis(dodecylphenyl)iodonium hexafluoroantimonate or hexafluorophosphate, bis(4-methylphenyl)iodonium hexafluorophosphate, bis(4-methoxyphenyl)iodonium hexafluorophosphate, 4-methylphenyl-4'-ethoxyphenyliodonium hexafluorophosphate, 4-methylphenyl-4'-dodecylphenyliodonium hexafluorophosphate, 4-methylphenyl-4'-phenoxyphenyliodonium hexafluorophosphate.

Of all the iodonium salts mentioned, compounds with other anions are, of course, also suitable. The preparation of iodonium salts is known to the person skilled in the art and described in the literature, for example US 4151175, US 3862333, US 4694029, EP 562897, US 4399071, US 6306555, WO 98/46647 J. V. Crivello, "Photoinitiated Cationic Polymerization" in: UV Curing: Science and Technology, Editor S. P. Pappas, pages 24-77, Technology Marketing Corporation, Norwalk, Conn. 1980, ISBN No. 0-686-23773-0; J. V. Crivello, J. H. W. Lam, Macromolecules, 10, 1307 (1977) and J. V. Crivello, Ann. Rev. Mater. Sci. 1983, 13, pages 173-190 and J. V. Crivello, Journal of Polymer Science, Part A: Polymer Chemistry, Vol. 37, 4241-4254 (1999).

Acylphosphinoxides, such as, for example, bis(2,4,6-trimethylbenzoyl)-phenylphosphine oxide, 2,4,6-trimethylbenzoyl-diphenyl-phosphine oxide and ethyl phenyl(2,4,6-trimethylbenzoyl)phosphinate, are preferred for curing with light sources having emission peak(s) in the UV-A range and (near) VIS range (Laser, LEDs, LCD). alpha-Hydroxy ketone type compounds, such as, for example, 1-hydroxy-cyclohexyl-phenyl-ketone, 2-hydroxy-2-methyl-1-phenyl-propan-1-one, 2-hydroxy-1-{4-[4-(2-hydroxy-2-methyl-propionyl)-benzyl]-phenyl}-2-methyl-propan-1-one, 1-[4-(2-hydroxyethoxy)-phenyl]-2-hydroxy-2-methyl-1-propan-1-one, Esacure KIP provided by Lamberti, 2-hydroxy-1-{1-[4-(2-hydroxy-2-methylpropionyl)-phenyl]-1,3,3-trimethyl-indan-5-yl}-2-methyl-propan-1-one and mixtures thereof, are preferred for curing with UV laser having emission peak at 355 nm (SLA).

If the light source emitts radiation over a broad range, UV and visible range (e.g. mercury bilbs), or light sources of different wavelengths are combined (e.g. LEDs, laser), the absorption range of one photoinitiator might not cover the entire range. This can be achieved by combining two different photoinitiator types, e.g. alpha-hydroxy ketones (1-hydroxy-cyclohexyl-phenyl-ketone, 2-hydroxy-2-methyl-1-phenyl-propan-1-one, or 2-hydroxy-1-{4-[4-(2-hydroxy-2-methyl-propionyl)-benzyl]-phenyl}-2-methyl-propan-1-one) with acyl phosphinoxides (bis(2,4,6-trimethylbenzoyl)-phenylphosphine oxide, 2,4,6-trimethylbenzoyl-diphenyl-phosphine oxide and ethyl phenyl(2,4,6-trimethylbenzoyl)phosphinate. If visible radiation is used for curing specific photoinitiators like titanocenes, such as, for example, bis (cyclopentadienyl) bis [2,6-difluoro- 3-(1-pyrryl)phenyl titanium (Omnirad 784) are required.

In a preferred embodiment trialkyl benzoyl and dialkyl dibenzoyl germanium compounds, such as, for example, dibenzoyldiethyl germanium, benzoyltriethyl germanium and bis-4-(methoxybenzoyl)diethyl germanium can be used as photoinitiators.

In another preferred embodiment, camphorquinone in combination with a tertiary amine as coninitiator is used as photoinitiator. The tertiary amine is preferably selected from ethyl 4-(dimethylamino)benzoate, triethanolamine, 2-(dimethylamino)ethylmethacrylate, 2-[4-(dimethylamino)phenyl]ethanol and N,N-dimethyl-p-toluidine.

The photoinitiators are used typically in a proportion of from about 0.1 to 10 % by weight, especially 0.1 to 5.0% by weight, very especially especially 0.5 to 2.0% by weight based on the total weight of composition.

Halogen is fluorine, chlorine, bromine and iodine.

C₁-C₂₄alkyl (C₁-C₂₀alkyl, especially C₁-C₁₂alkyl) is typically linear or branched, where possible. Examples are methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl and 2-ethylhexyl, n-nonyl, decyl, undecyl, dodecyl, tridecyl, tetradecyl, pentadecyl, hexadecyl, heptadecyl, or octadecyl. C₁-C₈alkyl is typically methyl, ethyl, n-propyl, isopropyl, n-butyl, sec.-butyl, isobutyl, tert.-butyl, n-pentyl, 2-pentyl, 3-pentyl, 2,2-dimethyl-propyl, n-hexyl, n-heptyl, n-octyl, 1,1,3,3-tetramethylbutyl and 2-ethylhexyl. C₁-C₄alkyl is typically methyl, ethyl, n-propyl, isopropyl, n-butyl, sec.-butyl, isobutyl, tert.-butyl.

C₂-C₁₂alkenyl (C₂-C₅alkenyl) groups are straight-chain or branched alkenyl groups, such as e.g. vinyl, allyl, methallyl, isopropenyl, 2-butenyl, 3-butenyl, isobutenyl, n-penta-2,4-dienyl, 3-methyl-but-2-enyl, n-oct-2-enyl, or n-dodec-2-enyl.

C₁-C₁₂alkoxy groups (C₁-C₈alkoxy groups) are straight-chain or branched alkoxy groups, e.g. methoxy, ethoxy, n-propoxy, isopropoxy, n-butoxy, sec-butoxy, tert-butoxy, amyloxy, isoamyloxy or tert-amyloxy, heptyloxy, octyloxy, isooctyloxy, nonyloxy, decyloxy, undecyloxy and dodecyloxy.

C₁-C₁₂alkylthio groups (C₁-C₈ alkylthio groups) are straight-chain or branched alkylthio groups and have the same preferences as the akoxy groups, except that oxygen is exchanged against sulfur.

C₁-C₁₂alkylene is bivalent C₁-C₁₂alkyl, i.e. alkyl having two (instead of one) free valencies, e.g. trimethylene or tetramethylene.

A cycloalkyl group is typically C₃-C₈cycloalkyl, such as, for example, cyclopentyl, cyclohexyl, cycloheptyl, or cyclooctyl, which may be unsubstituted or substituted.

In several cases it is advantageous to in addition to the photoinitiator employ a sensitizer compound. Examples of suitable sensitizer compounds are disclosed in WO 06/008251, page 36, line 30 to page 38, line 8, the disclosure of which is hereby incorporated by reference. As sensitizer inter alia benzophenone compounds as described above can be employed.

If desired, the photocurable compositions may comprise further mixture constituents which are preferably selected from
- at least one component D which is in turn selected from
   (H.4) defoamers and deaerating agents;
   (H.5) lubricants and leveling agents;
   (H.6) thermally curing and/or radiation-curing auxiliaries;
   (H.7) substrate wetting auxiliaries;
   (H.8) wetting and dispersing auxiliaries;
   (H.9) hydrophobizing agents;
   (H.10) in-can stabilizers; and
   (H.11) auxiliaries for improving scratch resistance;
- at least one component E which is in turn selected from
   (E.1) dyes; and
   (E.2) pigments;
- at least one component F which is in turn selected from light, heat and oxidation stabilizers; and
- at least one component G which is in turn selected from IR-absorbing compounds.

The effect of the defoamers and deaerating agents (H.4), lubricants and leveling agents (H.5), thermally curing or radiation-curing auxiliaries (H.6), substrate wetting auxiliaries (H.7), wetting and dispersing auxiliaries (H.8), hydrophobizing agents (H.9), in-can stabilizers (H.10) and auxiliaries for improving scratch resistance (H.11) listed under component D usually cannot be strictly distinguished from one another. For instance, lubricants and leveling agents often additionally act as defoamers and/or deaerating agents and/or as auxiliaries for improving scratch resistance. Radiation-curing auxiliaries can in turn act as lubricants and leveling agents and/or deaerating agents and/or also as substrate wetting auxiliaries. In accordance with the above statements, a certain additive may therefore be attributed to more than one of the groups (H.4) to (H.11) described below.

The defoamers of group (H.4) include silicon-free and silicon-containing polymers. The silicon-containing polymers are, for example, unmodified or modified polydialkylsiloxanes or branched copolymers, comb copolymers or block copolymers composed of polydialkylsiloxane and polyether units, the latter being obtainable from ethylene oxide or propylene oxide.

The deaerating agents of group (H.4) include, for example, organic polymers, for instance polyethers and polyacrylates, dialkylpolysiloxanes, especially dimethylpolysiloxanes, organically modified polysiloxanes, for instance arylalkyl-modified polysiloxanes, or else fluorosilicones. The action of defoamers is based essentially on preventing foam formation or destroying foam which has already formed. Deaerating agents act essentially in such a way that they promote the coalescence of finely distributed gas or air bubbles to larger bubbles in the medium to be deaerated, for example the inventive mixtures, and hence accelerate the escape of the gas (or of the air). Since defoamers can often also be used as deaerating agents and vice versa, these additives have been combined together under group (H.4). Such auxiliaries are, for example, obtainable commercially from Tego as TEGO^{®} Foamex 800, TEGO^{®} Foamex 805, TEGO^{®} Foamex 810, TEGO^{®} Foamex 815, TEGO^{®} Foamex 825, TEGO^{®} Foamex 835, TEGO^{®} Foamex 840, TEGO^{®} Foamex 842, TEGO^{®} Foamex 1435, TEGO^{®} Foamex 1488, TEGO^{®} Foamex 1495, TEGO^{®} Foamex 3062, TEGO^{®} Foamex 7447, TEGO^{®} Foamex 8020, Tego^{®} Foamex N, TEGO^{®} Foamex K 3, TEGO^{®} Antifoam 2-18, TEGO^{®} Antifoam 2-57, TEGO^{®} Antifoam 2-80, TEGO^{®} Antifoam 2-82, TEGO^{®} Antifoam 2-89, TEGO^{®} Antifoam 2-92, TEGO^{®} Antifoam 14, TEGO^{®} Antifoam 28, TEGO^{®} Antifoam 81, TEGO^{®} Antifoam D 90, TEGO^{®} Antifoam 93, TEGO^{®} Antifoam 200, TEGO^{®} Antifoam 201, TEGO@ Antifoam 202, TEGO^{®} Antifoam 793, TEGO^{®} Antifoam 1488, TEGO^{®} Antifoam 3062, TEGOPREN^{®} 5803, TEGOPREN^{®} 5852, TEGOPREN^{®} 5863, TEGOPREN^{®} 7008, TEGO^{®} Antifoam 1-60, TEGO^{®} Antifoam 1-62, TEGO^{®} Antifoam 1-85, TEGO^{®} Antifoam 2-67, TEGO^{®} Antifoam WM 20, TEGO^{®} Antifoam 50, TEGO^{®} Antifoam 105, TEGO^{®} Antifoam 730, TEGO^{®} Antifoam MR 1015, TEGO^{®} Antifoam MR 1016, TEGO^{®} Antifoam 1435, TEGO^{®} Antifoam N, TEGO^{®} Antifoam KS 6, TEGO^{®} Antifoam KS 10, TEGO^{®} Antifoam KS 53, TEGO^{®} Antifoam KS 95, TEGO^{®} Antifoam KS 100, TEGO^{®} Antifoam KE 600, TEGO^{®} Antifoam KS 911, TEGO^{®} Antifoam MR 1000, TEGO^{®} Antifoam KS 1100, Tego^{®} Airex 900, Tego^{®} Airex 910, Tego^{®} Airex 931, Tego^{®} Airex 935, Tego^{®} Airex 960, Tego^{®} Airex 970, Tego^{®} Airex 980 and Tego^{®} Airex 985, and from BYK as BYK^{®}-011, BYK^{®}-019, BYK^{®}-020, BYK^{®}-021, BYK^{®}-022, BYK^{®}-023, BYK^{®}-024, BYK^{®}-025, BYK^{®}-027, BYK^{®}-031, BYK^{®}-032, BYK^{®}-033, BYK^{®}-034, BYK^{®}-035, BYK^{®}-036, BYK^{®}-037, BYK^{®}-045, BYK^{®}-051, BYK^{®}-052, BYK^{®}-053, BYK^{®}-055, BYK^{®}-057, BYK^{®}-065, BYK^{®}-067, BYK^{®}-070, BYK^{®}-080, BYK^{®}-088, BYK^{®}-141 and BYK^{®}-A 530.

The auxiliaries of group (H.4) are typically used in a proportion of from about 0.05 to 3.0% by weight, preferably from about 0.5 to 2.0% by weight, based on the total weight of the composition.

The group (H.5) of the lubricants and leveling agents includes, for example, silicon-free but also silicon-containing polymers, for example polyacrylates or modified low molecular weight polydialkylsiloxanes. The modification consists in replacing some of the alkyl groups with a wide variety of organic radicals. These organic radicals are, for example, polyethers, polyesters or else long-chain alkyl radicals, the former finding most frequent use.

The polyether radicals of the correspondingly modified polysiloxanes are typically formed by means of ethylene oxide and/or propylene oxide units. The higher the proportion of these alkylene oxide units is in the modified polysiloxane, the more hydrophilic is generally the resulting product.

Such auxiliaries are obtainable commercially, for example, from Tego as TEGO^{®} Glide 100, TEGO^{®} Glide ZG 400, TEGO^{®} Glide 406, TEGO^{®} Glide 410, TEGO^{®} Glide 411, TEGO^{®} Glide 415, TEGO^{®} Glide 420, TEGO^{®} Glide 435, TEGO^{®} Glide 440, TEGO^{®} Glide 450, TEGO^{®} Glide A 115, TEGO^{®} Glide B 1484 (also usable as a defoamer and deaerating agent), TEGO^{®} Flow ATF, TEGO^{®} Flow ATF2, TEGO^{®} Flow 300, TEGO^{®} Flow 460, TEGO^{®} Flow 425 and TEGO^{®} Flow ZFS 460. The radiation-curable lubricants and leveling agents used, which additionally also serve to improve scratch resistance, can be the products TEGO^{®} Rad 2100, TEGO^{®} Rad 2200, TEGO^{®} Rad 2300, TEGO^{®} Rad 2500, TEGO^{®} Rad 2600, TEGO^{®} Rad 2700 and TEGO^{®} Twin 4000, likewise obtainable from Tego. Such auxiliaries are obtainable from BYK, for example as BYK^{®}-300, BYK^{®}-306, BYK^{®}-307, BYK^{®}-310, BYK^{®}-320, BYK^{®}-322, BYK^{®}-331, BYK^{®}-333, BYK^{®}-337, BYK^{®}-341, Byk^{®} 354, Byk^{®} 361 N, BYK^{®}-378 and BYK^{®}-388.

The auxiliaries of group (H.5) are typically used in a proportion of from about 0.005 to 1.0% by weight, preferably from about 0.01 to 0.2% by weight, based on the total weight of the composition.

Group (H.6) includes, as radiation-curing auxiliaries, in particular polysiloxanes with terminal double bonds which are, for example, part of an acrylate group. Such auxiliaries can be made to crosslink by actinic or, for example, electron beam radiation. These auxiliaries generally combine several properties in one. In the uncrosslinked state, they can act as defoamers, deaerating agents, lubricants and leveling agents and/or substrate wetting aids; in the crosslinked state, they increase in particular the scratch resistance, for example of coatings or films which can be produced with the inventive mixtures. The improvement in the shine performance, for example, coatings or films can essentially be regarded as the effect of the action of these auxiliaries as defoamers, devolatilizers and/or lubricants and leveling agents (in the uncrosslinked state). The radiation-curing auxiliaries which can be used are, for example, the products TEGO^{®} Rad 2100, TEGO^{®} Rad 2200, TEGO^{®} Rad 2500, TEGO^{®} Rad 2600 and TEGO^{®} Rad 2700 obtainable from Tego, and the product BYK^{®}-371 obtainable from BYK. Thermally curing auxiliaries of group (H.6) comprise, for example, primary OH groups which can react with isocyanate groups.

The thermally curing auxiliaries used can, for example, be the products BYK^{®}-370, BYK^{®}-373 and BYK^{®}-375 obtainable from BYK. The auxiliaries of group (H.6) are typically used in a proportion of from about 0.1 to 5.0% by weight, preferably from about 0.1 to 3.0% by weight, based on the total weight of the composition.

The auxiliaries of group (H.7) of the substrate wetting aids serve in particular to increase the wettability of the substrate, which is to be imprinted or coated, for instance, by printing inks or coating compositions, for example compositions (a.1) to (a.5). The generally associated improvement in the lubricating and leveling performance of such printing inks or coating compositions has an effect on the appearance of the finished (for example crosslinked) print or of the finished (for example crosslinked) layer. A wide variety of such auxiliaries are commercially available, for example, from Tego as TEGO^{®} Wet KL 245, TEGO^{®} Wet 250, TEGO^{®} Wet 260 and TEGO^{®} Wet ZFS 453, and from BYK as BYK^{®}-306, BYK^{®}-307, BYK^{®}-310, BYK^{®}-333, BYK^{®}-344, BYK^{®}-345, BYK^{®}-346 and Byk^{®}-348.

Also very suitable are the products of the Zonyl^{®} brand from Dupont, such as Zonyl^{®} FSA and Zonyl^{®} FSG. These are fluorinated surfactants/wetting agents.

The auxiliaries of group (H.7) are typically used in a proportion of from about 0.01 to 3.0% by weight, preferably from about 0.01 to 1.5% by weight and especially from 0.03 to 1.5% by weight, based on the total weight of the composition.

The auxiliaries of group (H.8) of the wetting and dispersing aids serve in particular to prevent the leaching and floating and also the settling of pigments, and are therefore useful, if necessary, in pigmented compositions in particular.

These auxiliaries stabilize pigment dispersions essentially by electrostatic repulsion and/or steric hindrance of the additized pigment particles, the interaction of the auxiliary with the surrounding medium (for example binder) playing a major role in the latter case. Since the use of such wetting and dispersing aids is common practice, for example, in the technical field of printing inks and paints, the selection of such a suitable auxiliary in the given case generally presents no difficulties to the person skilled in the art.

Such wetting and dispersing aids are supplied commercially, for example, by Tego as TEGO^{®} Dispers 610, TEGO^{®} Dispers 610 S, TEGO^{®} Dispers 630, TEGO^{®} Dispers 700, TEGO^{®} Dispers 705, TEGO^{®} Dispers 710, TEGO^{®} Dispers 720 W, TEGO^{®} Dispers 725 W, TEGO^{®} Dispers 730 W, TEGO^{®} Dispers 735 W and TEGO^{®} Dispers 740 W, and by BYK as Disperbyk^{®}, Disperbyk^{®}-107, Disperbyk^{®}-108, Disperbyk^{®}-110, Disperbyk^{®}-111, Disperbyk^{®}-115, Disperbyk^{®}-130, Disperbyk^{®}-160, Disperbyk^{®}-161, Disperbyk^{®}-162, Disperbyk^{®}-163, Disperbyk^{®}-164, Disperbyk^{®}-165, Disperbyk^{®}-166, Disperbyk^{®}-167, Disperbyk^{®}-170, Disperbyk^{®}-174, Disperbyk^{®}-180, Disperbyk^{®}-181, Disperbyk^{®}-182, Disperbyk^{®}-183, Disperbyk^{®}-184, Disperbyk^{®}-185, Disperbyk^{®}-190, Anti-Terra^{®}-U, Anti-Terra^{®}-U 80, Anti-Terra^{®}-P, Anti-Terra^{®}-203, Anti-Terra^{®}-204, Anti-Terra^{®} 5 206, BYK^{®}-151, BYK^{®}-154, BYK^{®}-155, BYK^{®}-P 104 S, BYK^{®}-P 105, Lactimon^{®}, Lactimon^{®}-WS and Bykumen^{®}. The abovementioned Zonyl^{®} brands, such as Zonyl^{®} FSA and Zonyl^{®} FSG, from DuPont are also useful here.

The dosage of the auxiliaries of group (H.8) depends mainly upon the surface area of the pigments to be covered and upon the mean molar mass of the auxiliary.

For inorganic pigments and low molecular weight auxiliaries, a content of the latter of from about 0.5 to 2.0% by weight based on the total weight of pigment and auxiliary is typically assumed. In the case of high molecular weight auxiliaries, the content is increased to from about 1.0 to 30% by weight.

In the case of organic pigments and low molecular weight auxiliaries, the content of the latter is from about 1.0 to 5.0% by weight based on the total weight of pigment and auxiliary. In the case of high molecular weight auxiliaries, this content may be in the range from about 10.0 to 90% by weight. In every case, therefore, preliminary experiments are recommended, which can, though, be accomplished by the person skilled in the art in a simple manner.

The hydrophobizing agents of group (H.9) can be used with a view, for example, to providing 3D prints obtained with inventive compositions with water-repellent properties. This means that swelling resulting from water absorption and hence a change, for example, in the optical properties of such 3D prints coatings is no longer possible or at least greatly suppressed. In addition, when the mixtures are used, for example, as a printing ink in offset printing, their absorption of water can be prevented or at least greatly inhibited. Such hydrophobizing agents are commercially available, for example, from Tego as Tego^{®} Phobe WF, Tego^{®} Phobe 1000, Tego^{®} Phobe 1000 S, Tego^{®} Phobe 1010, Tego^{®} Phobe 1030, Tego^{®} Phobe 1040, Tego^{®} Phobe 1050, Tego^{®} Phobe 1200, Tego^{®} Phobe 1300, Tego^{®} Phobe 1310 and Tego^{®} Phobe 1400.

The auxiliaries of group (H.9) are used typically in a proportion of from about 0.05 to 5.0% by weight, preferably from about 0.1 to 3.0% by weight, based on the total weight of the composition.

In-can stabilizers of group (H.10) provide increased storage stability from manufacturing to curing. Examples of in-can stabilizers of group (H.10) are:
Phosphites and phosphonites (processing stabilizer), for example triphenyl phosphite, diphenylalkyl phosphites, phenyldialkyl phosphites, tris(nonylphenyl) phosphite, trilauryl phosphite, trioctadecyl phosphite, distearylpentaerythritol diphosphite, tris(2,4-di-tert-butylphenyl) phosphite, diisodecyl pentaerythritol diphosphite, bis(2,4-di-tert-butylphenyl)pentaerythritol diphosphite, bis(2,4-di-cumylphenyl)pentaerythritol diphosphite, bis(2,6-di-tert-butyl-4-methylphenyl)pentaerythritol diphosphite, diisodecyloxypentaerythritol diphosphite, bis(2,4-di-tert-butyl-6-methylphenyl)pentaerythritol diphosphite, bis(2,4,6-tris(tert-butylphenyl)pentaerythritol diphosphite, tristearyl sorbitol triphosphite, tetrakis(2,4-di-tert-butylphenyl) 4,4'-biphenylene diphosphonite, 6-isooctyloxy-2,4,8,10-tetra-tert-butyl-12H-dibenz[d,g]-1,3,2-dioxaphosphocin, bis(2,4-di-tert-butyl-6-methylphenyl)methyl phosphite, bis(2,4-di-tert-butyl-6-methylphenyl)ethyl phosphite, 6-fluoro-2,4,8,10-tetra-tert-butyl-12-methyl-dibenz[d,g]-1,3,2-dioxaphosphocin, 2,2',2"-nitrilo[triethyltris(3,3',5,5'-tetra-tert-butyl-1,1'-biphenyl-2,2'-diyl)phosphite], 2-ethylhexyl(3,3',5,5'-tetra-tert-butyl-1,1'-biphenyl-2,2'-diyl)phosphite, 5-butyl-5-ethyl-2-(2,4,6-tri-tert-butylphenoxy)-1,3,2-dioxaphosphirane, phosphorous acid, mixed 2,4-bis(1,1-dimethylpropyl)phenyl and 4-(1,1-dimethylpropyl)phenyl triesters (CAS No. 939402-02-5), Phosphorous acid, triphenyl ester, polymer with alpha-hydro-omega-hydroxypoly[oxy(methyl-1,2-ethanediyl)], C10-16 alkyl esters (CAS No. 1227937-46-3). The following phosphites are especially preferred:
Tris(2,4-di-tert-butylphenyl) phosphite, tris(nonylphenyl) phosphite, 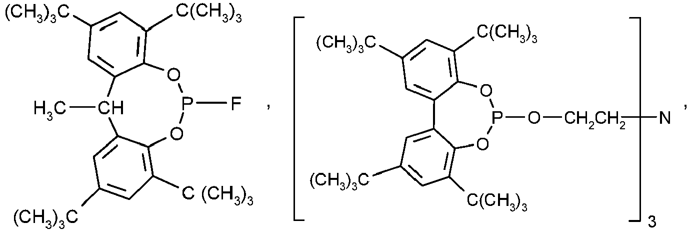 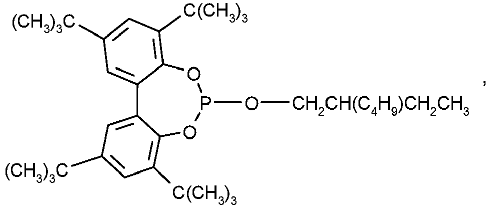 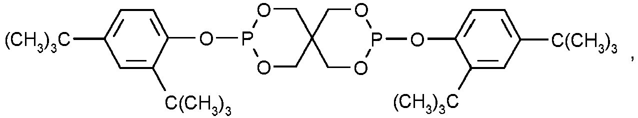 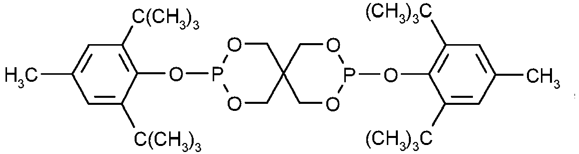 and

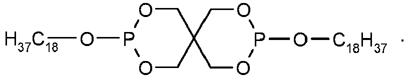

Quinone methides of the formula 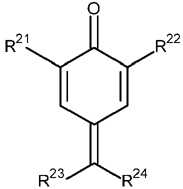 (providing long term shelf life stability), wherein
R²¹ and R²² independently of each other are C₁-C₁₈alkyl, C₅-C₁₂cycloalkyl, C₇-C₁₅-phenylalkyl, optionally substituted C₆-C₁₀aryl;
R²³ and R²⁴ independently of each other are H, optionally substituted C₆-C₁₀-aryl, 2-,3-,4-pyridyl, 2-,3-furyl or thienyl, COOH, COOR²⁵, CONH₂, CONHR²⁵, CONR²⁵R²⁶, -CN, - COR²⁵, -OCOR²⁵, -OPO(OR²⁵)₂, wherein R²⁵ and R²⁶ are independently of each other C₁-C₈alkyl, or phenyl. Quinone methides are preferred, wherein R²¹ and R²² are tert-butyl; R²³ is H, and R²⁴ is optionally substituted phenyl, COOH, COOR²⁵, CONH₂, CONHR²⁵, CONR²⁵R²⁶, -CN, -COR²⁵, -OCOR²⁵, -OPO(OR²⁵)₂, wherein R²⁵ and R²⁶ are C₁-C₈alkyl, or phenyl. Examples of quinone methides are

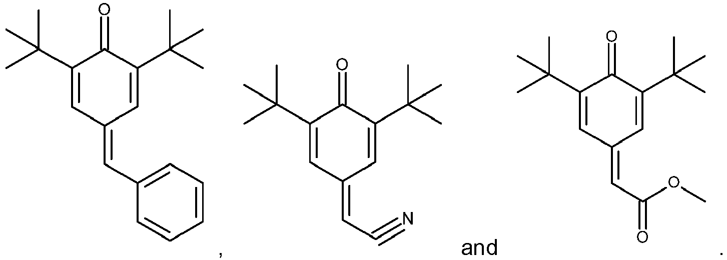

The quinone methides may be used in combination with highly sterically hindered nitroxyl radicals as described, for example, in US20110319535.

In-can stabilizers of group (H.10) are used typically in a proportion of from about 0.01 to 0.3% by weight, preferably from about 0.04 to 0.15% by weight, based on the total weight of the composition.

The group (H.11) of the auxiliaries for improving scratch resistance includes, for example, the products TEGO^{®} Rad 2100, TEGO^{®} Rad 2200, TEGO^{®} Rad 2500, TEGO^{®} Rad 2600 and TEGO^{®} Rad 2700 which are obtainable from Tego and have already been mentioned above.

For these auxiliaries, useful amounts are likewise those mentioned in group (H.6), i.e. these additives are typically used in a proportion of from about 0.1 to 5.0% by weight, preferably from about 0.1 to 3.0% by weight, based on the total weight of the composition.

The group (E.1) of the dyes includes, for example, dyes from the class of the azo dyes, metal complex dyes, basic dyes such as di- and triarylmethane dyes and salts thereof, azomethine derivatives, polymethines, antraquinone dyes and the like. An overview of suitable dyes which can be used in the inventive mixture is given by the book by H. Zollinger, "Color Chemistry", Wiley-VCH, Weinheim, 3rd edition 2003.

It is in particular also possible to add to the inventive compositions photochromic, thermochromic or luminescent dyes, and dyes which have a combination of these properties. In addition to the typical fluorescent dyes, fluorescent dyes should also be understood to mean optical brighteners. Optical brighteners may be used for the optimization of the absorption characteristics (critical energy and depth of penetration) of the photocurable composition.

Examples of the latter include the class of the bisstyrylbenzenes, especially of the cyanostyryl compounds, and correspond to the formula

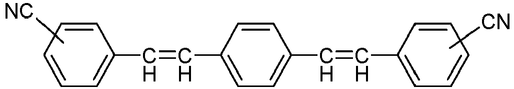

Further suitable optical brighteners from the class of the stilbenes are, for example, those of the formulae 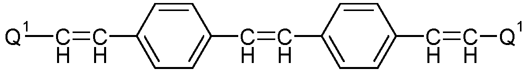 and

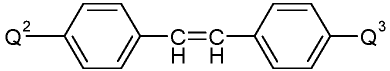

in which Q¹ is in each case C₁-C₄-alkoxycarbonyl or cyano, Q² is benzoxazol-2-yl, which may be mono- or disubstituted by C₁-C₄-alkyl, especially methyl, Q³ is C₁-C₄-alkoxycarbonyl or 3-(C₁-C₄-alkyl)-1,2,4-oxadiazol-3-yl.

Further suitable optical brighteners from the class of the benzoxazoles obey, for example, the formulae 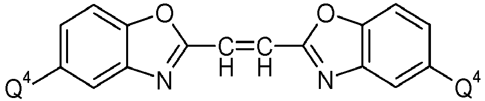 or 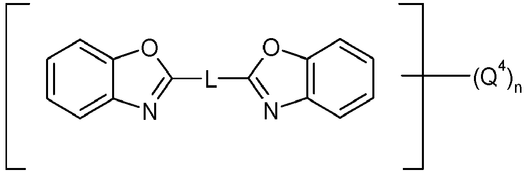
in which Q⁴ is in each case C₁-C₄-alkyl, especially methyl, L is a radical of the formula 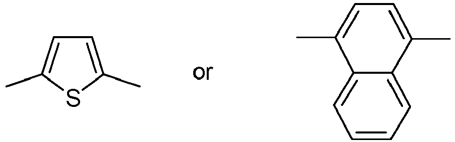
and n is an integer from 0 to 2.

Suitable optical brighteners from the class of the coumarins have, for example, the formula 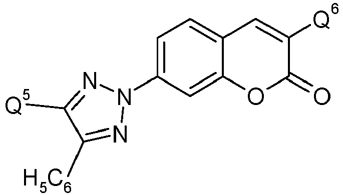 in which
Q⁵ is C₁-C₄-alkyl and
Q⁶ is phenyl or 3-halopyrazol-1-yl, especially 3-chloropyrazol-1-yl.

Further suitable optical brighteners from the class of the pyrenes correspond, for example, to the formula 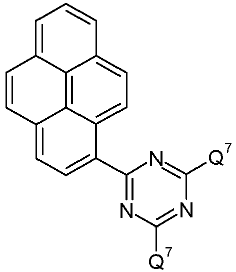 in which
Q⁷ is in each case C₁-C₄-alkoxy, especially methoxy.

The abovementioned brighteners can be used either alone or in a mixture with one another.

The abovementioned optical brighteners are generally commercially available products known per se. They are described, for example, in Ullmann's Encyclopedia of Industrial Chemistry, 5th edition, volume A18, pages 156 to 161, or can be obtained by the methods described there.

In particular, if desired, one or more optical brighteners from the class of the bisstyrylbenzenes is used, especially of the cyanostyrylbenzenes. The latter may be used as individual compounds, but also as a mixture of the isomeric compounds.

In this case, the isomers correspond to the formulae

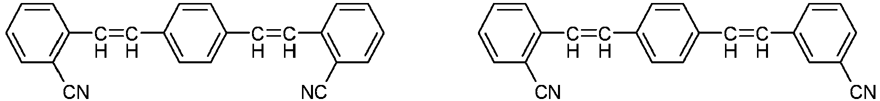

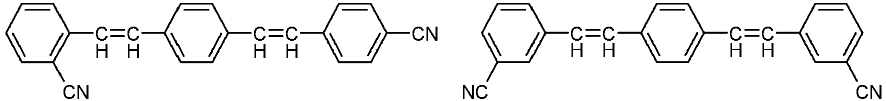

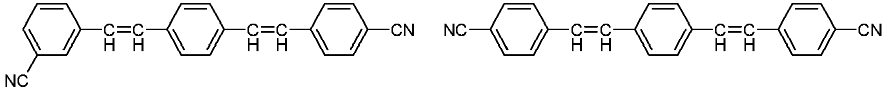

Optical brighteners are sold, for example, commercially as Ultraphor^{®} SF 004, Ultraphor^{®} SF MO, Ultraphor^{®} SF MP and Ultraphor^{®} SF PO from BASF SE.

The group (E.2) of the pigments includes both inorganic and organic pigments. An overview of inorganic colored pigments which can be used in the inventive mixtures is given by the book by H. Endriß "Aktuelle anorganische Bunt-Pigmente" ["Current inorganic colored pigments"] (publisher U. Zorll, Curt-R.-Vincentz-Verlag Hanover 1997), and the book by G. Buxbaum, "Industrial Inorganic Pigments", Wiley-VCH, Weinheim, 3rd edition 2005. In addition, useful further pigments which are not listed in the aforementioned book are also Pigment Black 6 and Pigment Black 7 (carbon black), Pigment Black 11 (iron oxide black, Fe₃O₄), Pigment White 4 (zinc oxide, ZnO), Pigment White 5 (lithopone, ZnS/BaSO₄), Pigment White 6 (titanium oxide, TiO₂) and Pigment White 7 (zinc sulfide, ZnS).

An overview of organic pigments which can be added to the inventive mixtures is provided by the book by W. Herbst and K. Hunger "Industrielle organische Pigmente" ["Industrial Organic Pigments"], Wiley-VCH, Weinheim, 3rd edition 2004.

It is also possible to add to the inventive mixtures magnetic, electrically conductive, photochromic, thermochromic or luminescent pigments, and also pigments which have a combination of these properties.

In addition to some organic pigments, for example Lumogen^{®} Yellow 0795 (BASF SE), useful pigments having luminescent properties are also inorganic, doped or undoped compounds essentially based on alkaline earth metal oxides, alkaline earth metal/transition metal oxides, alkaline earth metal/aluminum oxides, alkaline earth metal/silicon oxides or alkaline earth metal/phosphorus oxides, alkaline earth metal halides, Zn/silicon oxides, Zn/alkaline earth metal halides, rare earth metal oxides, rare earth metal/transition metal oxides, rare earth metal/aluminum oxides, rare earth metal/silicon oxides or rare earth metal/phosphorus oxides, rare earth metal oxide sulfides or oxide halides, zinc oxide, sulfide or selenide, cadmium oxide, sulfide or selenide or zinc/cadmium oxide, sulfide or selenide, the cadmium compounds being of lower importance owing to their toxicological and ecological relevance.

The dopants used in these compounds are usually aluminum, tin, antimony, rare earth metals, such as cerium, europium or terbium, transition metals, such as manganese, copper, silver or zinc, or combinations of these elements.

Luminescent pigments are specified below by way of example, the notation "compound:element(s)" being taken to mean to the relevant person skilled in the art that said compound has been doped with the corresponding element(s). In addition, for example, the notation "(P,V)", denotes that the corresponding lattice positions in the solid structure of the pigment are randomly occupied by phosphorus and vanadium.

Examples of such compounds which are capable of luminescence are MgWO₄, CaWO₄, Sr₄Al₁₄O₂₅:Eu, BaMg₂Al₁₀O₂₇:Eu, MgAl₁₁O₁₉:Ce,Tb, MgSiO₃:Mn, Ca₁₀(PO₄)₆(F,Cl):Sb,Mn, (SrMg)₂P₂O₇:Eu, SrMg₂P₂O₇:Sn, BaFCI:Eu, Zn₂SiO₄:Mn, (Zn,Mg)F₂:Mn, Y₂O₃:Eu, YVO₄:Eu, Y(P,V)O₄:Eu, Y₂SiO₅:Ce,Tb, Y₂O₂S:Eu, Y₂O₂S:Tb, La₂O₂S:Tb, Gd₂O₂S:Tb, LaOBr:Tb, ZnO:Zn, ZnS:Mn, ZnS:Ag, ZnS/CdS:Ag, ZnS:Cu,AI, ZnSe:Mn, ZnSe:Ag and ZnSe:Cu.

Examples of light, heat and/or oxidation stabilizers as component F include:
alkylated monophenols, such as 2,6-di-tert-butyl-4-methylphenol, 2-tert-butyl-4,6-dimethylphenol, 2,6-di-tert-butyl-4-ethylphenol, 2,6-di-tert-butyl-4-n-butylphenol, 2,6-di-tert-butyl-4-isobutylphenol, 2,6-dicyclopentyl-4-methylphenol, 2-(α-methylcyclohexyl)-4,6-dimethylphenol, 2,6-dioctadecyl-4-methylphenol, 2,4,6-tricyclohexylphenol, 2,6-di-tert-butyl-4-methoxymethylphenol, nonylphenols which have a linear or branched side chain, for example 2,6-dinonyl-4-methylphenol, 2,4-dimethyl-6-(1'-methylundec-1'-yl)phenol, 2,4-dimethyl-6-(1'-methylheptadec-1'-yl)phenol, 2,4-dimethyl-6-(1'-methyltridec-1'-yl)phenol and mixtures of these compounds,
alkylthiomethylphenols, such as 2,4-dioctylthiomethyl-6-tert-butylphenol, 2,4-dioctylthiomethyl-6-methylphenol, 2,4-dioctylthiomethyl-6-ethylphenol and 2,6-didodecylthiomethyl-4-nonylphenol,
hydroquinones and alkylated hydroquinones, such as 2,6-di-tert-butyl-4-methoxyphenol, 2,5-di-tert-butylhydroquinone, 2,5-di-tert-amylhydroquinone, 2,6-diphenyl-4-octadecyloxyphenol, 2,6-di-tert-butylhydroquinone, 2,5-di-tert-butyl-4-hydroxyanisole, 3,5-di-tert-butyl-4-hydroxyanisole, 3,5-di-tert-butyl-4-hydroxyphenyl stearate and bis(3,5-di-tert-butyl-4-hydroxyphenyl)adipate,
tocopherols, such as α-tocopherol, β-tocopherol, γ-tocopherol, 8-tocopherol and mixtures of these compounds, and tocopherol derivatives, such as tocopheryl acetate, succinate, nicotinate and polyoxyethylenesuccinate ("tocofersolate"),
hydroxylated diphenyl thioethers, such as 2,2'-thiobis(6-tert-butyl-4-methylphenol), 2,2'-thiobis(4-octylphenol), 4,4'-thiobis(6-tert-butyl-3-methylphenol), 4,4'-thiobis(6-tert-butyl-2-methylphenol), 4,4'-thiobis(3,6-di-sec-amylphenol) and 4,4'-bis(2,6-dimethyl-4-hydroxyphenyl) disulfide,
alkylidenebisphenols, such as 2,2'-methylenebis(6-tert-butyl-4-methylphenol), 2,2'-methylenebis(6-tert-butyl-4-ethylphenol), 2,2'-methylenebis[4-methyl-6-(a-methylcyclohexyl)phenol], 2,2'-methylenebis(4-methyl-6-cyclohexylphenol), 2,2'-methylenebis(6-nonyl-4-methylphenol), 2,2'-methylenebis(4,6-di-tert-butylphenol), 2,2-ethylidenebis(4,6-di-tert-butylphenol), 2,2'-ethylidenebis(6-tert-butyl-4-isobutylphenol), 2,2'-methylenebis[6-(a-methylbenzyl)-4-nonylphenol], 2,2'-methylenebis[6-(α,α-dimethylbenzyl)-4-nonylphenol], 4,4'-methylenebis(2,6-di-tert-butylphenol), 4,4'-methylenebis(6-tert-butyl-2-methylphenol), 1,1-bis(5-tert-butyl-4-hydroxy-2-methylphenyl)butane, 2,6-bis(3-tert-butyl-5-methyl-2-hydroxybenzyl)-4-methylphenol, 1,1,3-tris(5-tert-butyl-4-hydroxy-2-methylphenyl)butane, 1,1-bis(5-tert-butyl-4-hydroxy-2-methylphenyl)-3-n-dodecylmercaptobutane, ethylene glycol bis[3,3-bis(3'-tert-butyl-4'-hydroxyphenyl)butyrate], bis(3-tert-butyl-4-hydroxy-5-methylphenyl)dicyclopentadiene, bis[2-(3'-tert-butyl-2'-hydroxy-5'-methylbenzyl)-6-tert-butyl-4-methylphenyl] terephthalate, 1,1-bis(3,5-dimethyl-2-hydroxyphenyl)butane, 2,2-bis(3,5-di-tert-butyl-4-hydroxyphenyl)propane, 2,2-bis(5-tert-butyl-4-hydroxy-2-methylphenyl)-4-n-dodecyl-mercaptobutane and 1,1,5,5-tetrakis(5-tert-butyl-4-hydroxy-2-methylphenyl)pentane,
O-, N-and S-benzyl compounds, such as 3,5,3',5'-tetra-tert-butyl-4,4'-dihydroxydibenzyl ether, octadecyl 4-hydroxy-3,5-dimethylbenzylmercaptoacetate, tridecyl 4-hydroxy-3,5-di-tert-butylbenzylmercaptoacetate, tris(3,5-di-tert-butyl-4-hydroxybenzyl)amine, bis(4-tert-butyl-3-hydroxy-2,6-dimethylbenzyl) dithioterephthalate, bis(3,5-di-tert-butyl-4-hydroxybenzyl) sulfide and isooctyl-3,5-di-tert-butyl-4-hydroxybenzylmercaptoacetate, aromatic hydroxybenzyl compounds, such as 1,3,5-tris(3,5-di-tert-butyl-4-hydroxybenzyl)-2,4,6-trimethylbenzene, 1,4-bis(3,5-di-tert-butyl-4-hydroxybenzyl)-2,3,5,6-tetramethylbenzene and 2,4,6-tris(3,5-di-tert-butyl-4-hydroxybenzyl)phenol,
triazine compounds, such as 2,4-bis(octylmercapto)-6-(3,5-di-tert-butyl-4-hydroxyanilino)-1,3,5-triazine, 2-octylmercapto-4,6-bis(3,5-di-tert-butyl-4-hydroxyanilino)-1,3,5-triazine, 2-octylmercapto-4,6-bis(3,5-di-tert-butyl-4-hydroxyphenoxy)-1,3,5-triazine, 2,4,6-tris(3,5-di-tert-butyl-4-hydroxyphenoxy)-1,2,3-triazine, 1,3,5-tris(3,5-di-tert-butyl-4-hydroxybenzyl) isocyanurate, 1,3,5-tris(4-tert-butyl-3-hydroxy-2,6-dimethylbenzyl) isocyanurate, 2,4,6-tris(3,5-di-tert-butyl-4-hydroxyphenylethyl)-1,3,5-triazine, 1,3,5-tris-(3,5-di-tert-butyl-4-hydroxyphenylpropionyl)hexahydro-1,3,5-triazine, 1,3,5-tris(3,5-dicyclohexyl-4-hydroxybenzyl) isocyanurate and 1,3,5-tris(2-hydroxyethyl) isocyanurate, benzylphosphonates, such as dimethyl 2,5-di-tert-butyl-4-hydroxybenzylphosphonate, diethyl 3,5-di-tert-butyl-4-hydroxybenzylphosphonate, dioctadecyl 3,5-di-tert-butyl-4-hydroxybenzylphosphonate and dioctadecyl 5-tert-butyl-4-hydroxy-3-methylbenzylphosphonate,
acylaminophenols, such as 4-hydroxylauroylanilide, 4-hydroxystearoylanilide and octyl N-(3,5-di-tert-butyl-4-hydroxyphenyl)carbamate,
propionic and acetic esters, for example of monohydric or polyhydric alcohols, such as methanol, ethanol, n-octanol, isooctanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl) isocyanurate, N,N'-bis(hydroxyethyl)oxamide, 3-thiaundecanol, 3-thiapentadecanol, trimethylhexanediol, trimethylolpropane and 4-hydroxymethyl-1-phospha-2,6,7-trioxabicyclo[2.2.2]octane, propionamides based on amine derivatives, such as N,N'-bis(3,5-di-tert-butyl-4-hydroxyphenylpropionyl)hexamethylenediamine, N,N'-bis(3,5-di-tert-butyl-4-hydroxyphenylpropionyl)trimethylenediamine and N,N'-bis(3,5-di-tert-butyl-4-hydroxyphenylpropionyl)hydrazine,
ascorbic acid (Vitamin C) and ascorbic acid derivatives, such as ascorbyl palmitate, laurate and stearate, and ascorbyl sulfate and phosphate,
antioxidants based on amine compounds, such as N,N'-diisopropyl-p-phenylenediamine, N,N'-di-sec-butyl-p-phenylenediamine, N,N'-bis(1,4-dimethylpentyl)-p-phenylenediamine, N,N'-bis(1-ethyl-3-methylpentyl)-p-phenylenediamine, N,N'-bis(1-methylheptyl)-p-phenylenediamine, N,N'-dicyclohexyl-p-phenylenediamine, N,N'-diphenyl-p-phenylenediamine, N,N'-bis(2-naphthyl)-p-phenylenediamine, N-isopropyl-N'-phenyl-p-phenylenediamine, N-(1,3-dimethylbutyl)-N'-phenyl-p-phenylenediamine, N-(1-methylheptyl)-N'-phenyl-p-phenylenediamine, N-cyclohexyl-N'-phenyl-p-phenylenediamine, 4-(p-toluenesulfamoyl)diphenylamine, N,N'-dimethyl-N,N'-di-sec-butyl-p-phenylenediamine, diphenylamine, N-allyldiphenylamine, 4-isopropoxydiphenylamine, N-phenyl-1-naphthylamine, N-(4-tert-octylphenyl)-1-naphthylamine, N-phenyl-2-naphthylamine, octyl-substituted diphenylamine, such as p,p'-di-tert-octyldiphenylamine, 4-n-butylaminophenol, 4-butyrylaminophenol, 4-nonanoylaminophenol, 4-dodecanoylaminophenol, 4-octadecanoylaminophenol, bis[4-methoxyphenyl)amine, 2,6-di-tert-butyl-4-dimethylaminomethylphenol, 2,4-diaminodiphenylmethane, 4,4'-diaminodiphenylmethane, N,N,N',N'-tetramethyl-4,4'-diaminodiphenylmethane, 1,2-bis[(2-methylphenyl)amino]ethane, 1,2-bis(phenylamino)propane, (o-tolyl)biguanide, bis[4-(1',3'-dimethylbutyl)phenyl]amine, tert-octyl-substituted N-phenyl-1-naphthylamine, a mixture of mono- and dialkylated tert-butyl/tert-octyldiphenylamine, a mixture of mono- and dialkylated nonyldiphenylamine, a mixture of mono- and dialkylated dodecyldiphenylamine, a mixture of mono- and dialkylated isopropyl/isohexyldiphenylamine, a mixture of mono- and dialkylated tert-butyldiphenylamine, 2,3-dihydro-3,3-dimethyl-4H-1,4-benzothiazine, phenothiazine, a mixture of mono- and dialkylated tert-butyl/tert-octylphenothiazine, a mixture of mono- and dialkylated tert-octylphenothiazine, N-allylphenothiazine, N,N,N',N'-tetraphenyl-1,4-diaminobut-2-ene, N,N-bis(2,2,6,6-tetramethylpiperidin-4-yl)hexamethylenediamine, bis(2,2,6,6-tetramethylpiperidin-4-yl) sebacate, 2,2,6,6-tetramethylpiperidin-4-one and 2,2,6,6-tetramethylpiperidin-4-ol,
phosphites and phosphonites, such as triphenylphosphite, diphenyl alkyl phosphite, phenyl dialkyl phosphite, tris(nonylphenyl) phosphite, trilauryl phosphite, trioctadecyl phosphite, distearyl pentaerythritol diphosphite, tris(2,4-di-tert-butylphenyl) phosphite, diisodecyl pentaerythritol diphosphite, bis(2,4-di-tert-butylphenyl) pentaerythritol diphosphite, bis(2,6-di-tert-butyl-4-methylphenyl) pentaerythritol diphosphite, diisodecyloxy pentaerythritol diphosphite, bis(2,4-di-tert-butyl-6-methylphenyl) pentaerythritol diphosphite, bis(2,4,6-tris(tert-butylphenyl)) pentaerythritol diphosphite, tristearyl sorbitol triphosphite, tetrakis(2,4-di-tert-butylphenyl) 4,4'-biphenylenediphosphonite, 6-isooctyloxy-2,4,8,10-tetra-tert-butyl-12H-dibenzo[d,g]-1,3,2-dioxaphosphocine, 6-fluoro-2,4,8,10-tetra-tert-butyl-12-methyl-dibenzo[d,g]-1,3,2-dioxaphosphocine, bis(2,4-di-tert-butyl-6-methylphenyl) methyl phosphite and bis(2,4-di-tert-butyl-6-methylphenyl) ethyl phosphite, 2-(2'-hydroxyphenyl)benzotriazoles, such as 2-(2'-hydroxy-5'-methylphenyl)benzotriazole, 2-(3',5'-di-tert-butyl-2'-hydroxyphenyl)benzotriazole, 2-(5'-tert-butyl-2'-hydroxyphenyl)benzotriazole, 2-(2'-hydroxy-5'-(1,1,3,3-tetramethylbutyl)phenyl)benzotriazole, 2-(3',5'-di-tert-butyl-2'-hydroxyphenyl)-5-chlorobenzotriazole, 2-(3'-tert-butyl-2'-hydroxy-5'-methylphenyl)-5-chlorobenzotriazole, 2-(3'-sec-butyl-5'-tert-butyl-2'-hydroxyphenyl)benzotriazole, 2-(2'-hydroxy-4'-octyloxyphenyl)benzotriazole, 2-(3',5'-di-tert-amyl-2'-hydroxyphenyl)benzotriazole, 2-(3',5'-bis-(a,a-dimethylbenzyl)-2'-hydroxyphenyl)benzotriazole, a mixture of 2-(3'-tert-butyl-2'-hydroxy-5'-(2-octyloxycarbonylethyl)phenyl)-5-chlorobenzotriazole, 2-(3'-tert-butyl-5'-[2-(2-ethylhexyloxy)carbonylethyl]-2'-hydroxyphenyl)-5-chlorobenzotriazole, 2-(3'-tert-butyl-2'-hydroxy-5'-(2-methoxycarbonylethyl)phenyl)-5-chlorobenzotriazole, 2-(3'-tert-butyl-2'-hydroxy-5'-(2-methoxycarbonylethyl)phenyl)benzotriazole, 2-(3'-tert-butyl-2'-hydroxy-5'-(2-octyloxycarbonylethyl)phenyl)benzotriazole, 2-(3'-tert-butyl-5'-[2-(2-ethylhexyloxy)carbonylethyl]-2'-hydroxyphenyl)benzotriazole, 2-(3'-dodecyl-2'-hydroxy-5'-methylphenyl)benzotriazole and 2-(3'-tert-butyl-2'-hydroxy-5'-(2-isooctyloxycarbonylethyl)phenylbenzotriazole, 2,2'-methylenebis[4-(1,1,3,3-tetramethylbutyl)-6-benzotriazol-2-ylphenol]; the product of complete esterification of 2-[3'-tert-butyl-5'-(2-methoxycarbonylethyl)-2'-hydroxyphenyl]-2H-benzotriazole with polyethylene glycol 300; [R-CH₂CH₂-COO(CH₂)₃]₂, where R = 3'-tert-butyl-4'-hydroxy-5'-2H-benzotriazol-2-ylphenyl],
sulfur-containing peroxide scavengers and sulfur-containing antioxidants, such as esters of 3,3'-thiodipropionic acid, for example the lauryl, stearyl, myristyl and tridecyl esters, mercaptobenzimidazole and the zinc salt of 2-mercaptobenzimidazole, dibutylzinc dithiocarbamate, dioctadecyl disulfide and pentaerythritol tetrakis(β-dodecylmercapto)propionate,
2-hydroxybenzophenones, such as the 4-hydroxy, 4-methoxy, 4-octyloxy, 4-decycloxy, 4-dodecyloxy, 4-benzyloxy, 4,2',4'-trihydroxy and 2'-hydroxy-4,4'-dimethoxy derivatives, esters of unsubstituted and substituted benzoic acids, such as 4-tert-butylphenyl salicylate, phenyl salicylate, octylphenyl salicylate, dibenzoylresorcinol, bis(4-tert-butylbenzoyl)resorcinol, benzoylresorcinol, 2,4-di-tert-butylphenyl 3,5-di-tert-butyl-4-hydroxybenzoate, hexadecyl-3,5-di-tert-butyl-4-hydroxybenzoate, octadecyl-3,5-di-tert-butyl-4-hydroxybenzoate and 2-methyl-4,6-di-tert-butylphenyl-3,5-di-tert-butyl-4-hydroxybenzoate,
acrylates, such as ethyl α-cyano-β,β-diphenylacrylate, isooctyl α-cyano-β,β-diphenylacrylate, methyl α-methoxycarbonylcinnamate, methyl α-cyano-β-methyl-p-methoxycinnamate, butyl-α-cyano-β-methyl-p-methoxycinnamate and methyl-α-methoxycarbonyl-p-methoxycinnamate,
sterically hindered amines, such as bis(2,2,6,6-tetramethylpiperidin-4-yl) sebacate, bis(2,2,6,6-tetramethylpiperidin-4-yl) succinate, bis(1,2,2,6,6-pentamethylpiperidin-4-yl) sebacate, bis(1-octyloxy-2,2,6,6-tetramethylpiperidin-4-yl) sebacate, bis(1,2,2,6,6-pentamethylpiperidin-4-yl)-n-butyl-3,5-di-tert-butyl-4-hydroxybenzylmalonate, the condensation product of 1-(2-hydroxyethyl)-2,2,6,6-tetramethyl-4-hydroxypiperidine and succinic acid, the condensation product of N,N'-bis(2,2,6,5-tetramethylpiperidin-4-yl)hexamethylenediamine and 4-tert-octylamino-2,6-dichloro-1,3,5-triazine, tris(2,2,6,6-tetramethylpiperidin-4-yl) nitrilotriacetate, tetrakis(2,2,6,6-tetramethylpiperidin-4-yl) 1,2,3,4-butanetetracarboxylate, 1,1'-(1,2-ethylene)bis(3,3,5,5-tetramethylpiperazinone), 4-benzoyl-2,2,6,6-tetramethylpiperidine, 4-stearyloxy-2,2,6,6-tetramethylpiperidine, bis(1,2,2,6,6-pentamethylpiperidin-4-yl) 2-n-butyl-2-(2-hydroxy-3,5-di-tert-butylbenzyl)malonate, 3-n-octyl-7,7,9,9-tetramethyl-1,3,8-triazaspiro[4.5]decane-2,4-dione, bis(1-octyloxy-2,2,6,6-tetramethylpiperidin-4-yl) sebacate, bis(1-octyloxy-2,2,6,5-tetramethylpiperidin-4-yl) succinate, the condensation product of N,N'-bis(2,2,6,6-tetramethylpiperidin-4-yl)hexamethylenediamine and 4-morpholino-2,6-dichloro-1,3,5-triazine, the condensation product of 2-chloro-4,6-bis(4-n-butylamino-2,2,6,6-tetramethylpiperidin-4-yl)-1,3,5-triazine and 1,2-bis(3-aminopropylamino)ethane, the condensation product of 2-chloro-4,6-di(4-n-butylamino-1,2,2,6,6-pentamethylpiperidin-4-yl)-1,3,5-triazine and 1,2-bis(3-aminopropylamino)ethane, 8-acetyl-3-dodecyl-7,7,9,9-tetramethyl-1,3,8-triazaspiro[4.5]decane-2,4-dione, 3-dodecyl-1-(2,2,6,6-tetramethylpiperidin-4-yl)pyrrolidine-2,5-dione, 3-dodecyl-1-(1,2,2,6,6-pentamethylpiperidin-4-yl)pyrrolidine-2,5-dione, a mixture of 4-hexadecyloxy- and 4-stearyloxy-2,2,6,6-tetramethylpiperidine, the condensation product of N,N'-bis(2,2,6,6-tetramethylpiperidin-4-yl)hexamethylenediamine and 4-cyclohexylamino-2,6-dichloro-1,3,5-triazine, the condensation product of 1,2-bis(3-aminopropylamino)ethane and 2,4,6-trichloro-1,3,5-triazine, 4-butylamino-2,2,6,6-tetramethylpiperidine, N-(2,2,6,6-tetramethylpiperidin-4-yl)-n-dodecylsuccinimide, N-(1,2,2,6,6-pentamethylpiperidin-4-yl)-n-dodecylsuccinimide, 2-undecyl-7,7,9,9-tetramethyl-1-oxa-3,8-diaza-4-oxo-spiro[4.5]decane, the condensation product of 7,7,9,9-tetramethyl-2-cycloundecyl-1-oxa-3,8-diaza-4-oxospiro-[4.5]decane and epichlorohydrin, the condensation products of 4-amino-2,2,6,6-tetramethylpiperidine with tetramethylolacetylenediureas and poly(methoxypropyl-3-oxy)-[4(2,2,6,6-tetramethyl)piperidinyl]siloxane,
oxamides, such as 4,4'-dioctyloxyoxanilide, 2,2'-diethoxyoxanilide, 2,2'-dioctyloxy-5,5'-di-tert-butoxanilide, 2,2'-didodecyloxy-5,5'-di-tert-butoxanilide, 2-ethoxy-2'-ethyloxanilide, N,N'-bis(3-dimethylaminopropyl)oxamide, 2-ethoxy-5-tert-butyl-2'-ethoxanilide and its mixture with 2-ethoxy-2'-ethyl-5,4'-di-tert-butoxanilide, and mixtures of ortho-, para-methoxy-disubstituted oxanilides and mixtures of ortho- and para-ethoxy-disubstituted oxanilides, and
2-(2-hydroxyphenyl)-1,3,5-triazines, such as 2,4,6-tris-(2-hydroxy-4-octyloxyphenyl)-1,3,5-triazine, 2-(2-hydroxy-4-octyloxyphenyl)-4,6-bis(2,4-dimethylphenyl)-1,3,5-triazine, 2-(2,4-dihydroxyphenyl)-4,6-bis(2,4-dimethylphenyl)-1,3,5-triazine, 2,4-bis(2-hydroxy-4-propyloxyphenyl)-6-(2,4-dimethylphenyl)-1,3,5-triazine, 2-(2-hydroxy-4-octyloxyphenyl)-4,6-bis(4-methyl-5 phenyl)-1,3,5-triazine, 2-(2-hydroxy-4-dodecyloxyphenyl)-4,6-bis(2,4-dimethylphenyl)-1,3,5-triazine, 2-(2-hydroxy-4-tridecyloxyphenyl)-4,6-bis(2,4-dimethylphenyl)-1,3,5-triazine, 2-[2-hydroxy-4-(2-hydroxy-3-butyloxypropoxy)phenyl]-4,6-bis(2,4-dimethyl)-1,3,5-triazine, 2-[2-hydroxy-4-(2-hydroxy-3-octyloxypropoxy)phenyl]-4,6-bis(2,4-dimethyl)-1,3,5-triazine, 2-[4-(dodecyloxy/tridecyloxy-2-hydroxypropoxy)-2-hydroxyphenyl]-4,6-bis(2,4-dimethylphenyl)-1,3,5-triazine, 2-[2-hydroxy-4-(2-hydroxy-3-dodecyloxypropoxy)phenyl]-4,6-bis-(2,4-dimethylphenyl)-1,3,5-triazine, 2-(2-hydroxy-4-hexyloxyphenyl)-4,6-diphenyl-1,3,5-triazine, 2-(2-hydroxy-4-methoxyphenyl)-4,6-diphenyl-1,3,5-triazine, 2,4,6-tris[2-hydroxy-4-(3-butoxy-2-hydroxypropoxy)phenyl]-1,3,5-triazine and 2-(2-hydroxyphenyl)-4-(4-methoxyphenyl)-6-phenyl-1,3,5-triazine.

The components G of the IR absorber used are compounds which exhibit one or more absorption bands in the infrared spectral region, i.e. from > 750 nm, e.g. from 751 nm, to 1 mm. Preference is given to compounds which exhibit one absorption band in the near infrared (NIR) spectral region, i.e. from > 750 (e.g. 751) to 2000 nm, and optionally additionally also in the visible spectral region, especially from 550 to 750 nm. When the compounds absorb both in the IR and in the visible spectral region, they preferably exhibit the greatest absorption maximum in the IR region and a smaller maximum (frequently in the form of a so-called absorption shoulder) in the visible region. In a particular embodiment, the compounds of component G additionally also exhibit fluorescence. Fluorescence is the transition of a system excited by absorption of electromagnetic radiation (usually visible light, UV radiation, X-rays or electron beams) to a state of lower energy by spontaneous emission of radiation of the same wavelength (resonance fluorescence) or longer wavelength. Preferred compounds of component G exhibit, when they fluoresce, a fluorescence in the IR spectral region, preferably in the NIR.

Such compounds are, for example, selected from naphthalenes, anthracenes, phenanthrenes, tetracenes, perylenes, terrylenes, quaterrylenes, pentarylenes, hexarylenes, anthraquinones, indanthrones, acridines, carbazoles, dibenzofuranes, dinaphthofuranes, benzimidazoles, benzthiazoles, phenazines, di-oxazines, quinacridones, metal phthalocyanines, metal naphthalocyanines, metal porphyrines, coumarines, dibenzofuranones, dinaphthofuranones, benzimidazolones, indigo compounds, thioindigo compounds, quinophthalones, naphthoquinophthalones and diketopyrrolopyrroles. Particularly preferred compounds of component G which absorb IR radiation and optionally fluoresce are selected from naphthalenes, anthracenes, phenanthrenes, tetracenes, perylenes, terrylenes, quaterrylenes, pentarylenes and hexarylenes, more preferably from perylenes, terrylenes and quaterrylenes and especially from terrylenes and quaterrylenes. The compound is especially a quaterrylene. Suitable compounds are described in WO 2008/012292.

The present disclosure(s) also provides methods suitable for making 3-dimensional structures comprising a plurality of polymer layers and 3-dimensional patterns.

Some embodiments provide methods of patterning a polymeric image on a substrate, each method comprising;
(a) depositing a layer of photocurable composition of any one of the compositions described herein on the substrate;
(b) irradiating a portion of the layer of photocurable composition with a light having appropriate wavelength(s), thereby providing a patterned layer of polymerized and unpolymerized regions. Certain other embodiments further comprise removing the unpolymerized region of the pattern.

The method may comprise depositing a plurality of layers of a photocurable composition on a substrate before irradiation, at least one of which is the photocurable composition of the present invention.

The irradiated portion is patterned through use of a photomask, by a direct writing application of light, by interference, nanoimprint, or diffraction gradient lithography, by inkjet 3D printing, stereolithography, holography, LCD or digital light projection (DLP).

The photocurable compositions may be irradiated by any variety of methods known in the art. Patterning may be achieved by photolithography, using a positive or negative image photomask, by interference lithography (i.e., using a diffraction grating), by proximity field nanopatterning by diffraction gradient lithography, or by a direct laser writing application of light, such as by multi-photon lithography, by nanoimprint lithography, by inkjet 3D printing, stereolithography and the digital micromirror array variation of stereolithography (commonly referred to as digital light projection (DLP). The photocurable compositions are especially amenable to preparing structures using stereolithographic methods, for example including digital light projection (DLP). The photocurable compositions may be processed as bulk structures, for example using vat polymerization, wherein the photopolymer is cured directly onto a translated or rotated substrate, and the irradiation is patterned via stereolithography, holography, or digital light projection (DLP).

Stereolithography (SLA) is a form of three-dimensional (3D) printing technology used for creating models, prototypes, patterns and production parts in a layer by layer fashion (so-called "additive manufacturing") using photo-polymerization, a process by which light causes chains of molecules to link, forming polymers. Those polymers then make up the body of a three-dimensional solid. Typically, an SLA additive manufacturing process uses a build platform having a build tray submerged in a liquid photosensitive material. A 3D model of the item to be manufactured is imported into an associated 3D printer software, which software slices the 3D model into 2D images that are then projected onto the build platform to expose the photopolymer.

FIG. 3 of US4575330 depicts a known prior art "top-down" approach to printing. A container 21 is filled with a UV curable liquid 22 or the like, to provide a designated working surface 23. A programmable source of ultraviolet (UV) light 26 produces a spot of ultraviolet light 27 in the plane of surface 23. The spot 27 is movable across the surface 23 by the motion of mirrors or other optical or mechanical elements that are a part of light source 26. The position of the spot 27 on surface 23 is controlled by a computer 28. A movable elevator platform 29 inside container 21 is moved up and down selectively, the position of the platform being controlled by the computer 28. The elevator platform may be driven mechanically, pneumatically, hydraulically or electrically, and it typically uses optical or electronic feedback to precisely control its position. As the device operates, it produces a three-dimensional object 30 by step-wise buildup of integrated laminate such as 30a, 30b, 30c. During this operation, the surface of the UV curable liquid 22 is maintained at a constant level in the container 21, and the spot of UV light 27 is moved across the working surface 23 in a programmed manner. As the liquid 22 cures and solid material forms, the elevator platform 29 that was initially just below surface 23 is moved down from the surface in a programmed manner by any suitable actuator. In this way, the solid material that was initially formed is taken below surface 23 and new liquid 22 flows across the surface 23. A portion of this new liquid is, in turn, converted to solid material by the programmed UV light spot 27, and the new material adhesively connects to the material below it. This process is continued until the entire three-dimensional object 30 is formed.

A computer controlled pump (not shown) may be used to maintain a constant level of the liquid 22 at the working surface 23. Appropriate level detection system and feedback networks can be used to drive a fluid pump or a liquid displacement device to offset changes in fluid volume and maintain constant fluid level at the surface 23. Alternatively, the source 26 can be moved relative to the sensed level 23 and automatically maintain sharp focus at the working surface 23. All of these alternatives can be readily achieved by conventional software operating in conjunction with the computer control system 28.

An alternative approach is to build the item from the "bottom-up" as depicted in FIG. 4 of US4575330. In this approach, the UV curable liquid 22 floats on a heavier UV transparent liquid 32 that is non-miscible and non-wetting with the curable liquid 22. By way of example, ethylene glycol or heavy water are suitable for the intermediate liquid layer 32. In the system of FIG. 4, the three-dimensional object 30 is pulled up from the liquid 22, rather than down and further into the liquid medium, as shown in the system of FIG. 3. In particular, the UV light source 26 in FIG. 4 focuses the spot 27 at the interface between the liquid 22 and the non-miscible intermediate liquid layer 32, the UV radiation passing through a suitable UV transparent window 33, of quartz or the like, supported at the bottom of the container 21.

According WO2018106977, and in lieu of printing just from resin in its liquid phase, one or more layers of the item are printed from resin that is foamed (at the build surface 23).

FIG. 3 of WO2018106977 depicts a representative implementation of an additive manufacturing method and apparatus wherein resin foam is the source material for the printer. A top-down printing method is depicted. In this example embodiment, the SLA apparatus comprises a radiation source 300 (e.g., DLP, laser, electron beam (EB), x-ray, etc. and scanner), a movement control mechanism 302 (e.g., a stepper motor) that moves a build platform 304 vertically up and down within a tank 305 that holds the photopolymer resin 306, and a sweeper 308 (also known as a "recoater" blade) that sweeps horizontally. These elements are used to print a part 310 in the manner previously described. The SLA apparatus is augmented with a foam producing and dispensing mechanism to facilitate production of resin foam at the printer interface, namely, the layer being printed. To this end, the mechanism comprises a foaming or pressure vessel 312, an electromechanical valve 314, and a hose or tube 316. A manifold 318 is attached to the sweeper 308 to evenly distribute the foamed resin across the top layer of the build surface. In particular, and as depicted, the foaming vessel receives liquid resin and a suitable gas (e.g., CO₂, N₂O, etc.). Gas is dissolved in the liquid resin within the foaming vessel (e.g., by shaking, missing, agitation, etc.) and selectively delivered to the build plate/platform via the hose 316 when the valve 314 is actuated, e.g., by a solenoid or other electromechanical, pneumatic, optical or electronic control device. Typically, the mechanism is under program control using a computer, which may be the same computer used to control the printer. In this embodiment, the mechanism includes a frother 320 (e.g., a mechanical agitator, an ultrasonic device, etc.) to shake or otherwise dissolve the gas within the liquid vessel if needed to produce foam.

Upon delivery of the resin and gas mixture (directly onto the build plate via the manifold 318), the gas spontaneously evolves out of the liquid mixture (due to the lower pressure) to produce a foam that is radiation-curable. The sweeper 308 spreads the foam evenly onto the plate, and the light engine is then activated to display the appropriate image to cure (solidify) the foam into a layer. Once the layer is formed, the movement control mechanism moves the platform down so that the next layer of the item can be built; the process is then repeated, once again preferably using the foam layer at the print interface.

While the preferred technique uses layer-wise additive manufacturing, other manufacturing processes may be used to process the foam to produce the build item, such as, for example, laser holography, wherein two lasers intersect in a tank of foamed resin and cure the resin at that spot.

The photocurable composition of the present invention is preferably used in vat photopolymerization (stereolithography) and photopolymerjetting/printing.

In addition, the present invention is directed to a method for producing a three-dimensional article, comprising
a) providing the photocurable composition of the present invention,
b) exposing the photocurable composition to actinic radiation to form a cured crossection,
c) repeating steps (a) and (b) to build up a three-dimensional article.

In a preferred embodiment the method comprises a vat photopolymerization, wherein the photocurable of the present invention in step b) is cured directly onto a translated or rotated substrate, and the irradiation is patterned via stereolithography, holography, or digital light projection (DLP).

In another preferred embodiment the method comprises
a) applying a layer of the photocurable composition of the present invention onto a surface;
b) exposing the layer imagewise to actinic radiation to form an imaged cured cross- section;
c) applying a second layer of the photocurable composition onto the previously exposed imaged cross-section;
d) exposing the layer from step (c) imagewise to actinic radiation to form an additional imaged cross-section, wherein the radiation causes curing of the second layer in the exposed areas and adhesion to the previously exposed cross-section; and
e) repeating steps (c) and (d) in order to build up a three-dimensional article.

Accordingly, the present invention is also directed to a three-dimensional article produced by the method of the present invention, or a three-dimensional article, which is a cured product of the photocurable composition of the present invention.

The photocurable compositions of the present invention may be used in dual cure stereolithography resins suitable for stereolithography techniques (particularly for CLIP). Reference is made to US9,453,142, US2016/0136889, US2016/0137838 and US2016/016077. These resins usually include a first polymerizable system typically polymerized by light (sometimes referred to as "Part A") from which an intermediate object is produced, and also include at least a second polymerizable system ("Part B") which is usually cured after the intermediate object is first formed, and which impart desirable structural and/or tensile properties to the final object. The photocurable compositions of the present invention may be comprised by Part A.

The following examples illustrate the invention without restricting it.

### Examples

### Materials

Limonene dioxide (LO) was purchased from Nitrochemie Aschau GmbH, 1,2-dimethylimidazol (DMI, 98 %), butylated hydroxytoluene (BHT, 99%), methacrylic acid (MA, 99 %, 250 ppm MEHQ), Diphenyl(2,4,6-trimethylbenzoyl)phosphine oxide (TPO, 97 %) and glycidyl methacrylate (GMA, 97 %, 100 ppm MEHQ) were obtained from Sigma Aldrich. Magnesium oxide (99 %, nanoparticles, 20 nm) and 4-methacryloylmorpholine (ACMO, 98 %) were purchased from abcr and TCI, respectively. Laromer^{©} UA 9089 (Laromer) was provided by BASF.

### Methods

NMR spectra were recorded in deuterated chloroform on an *ARX 300* spectrometer from Bruker at room temperature. The chemical shifts were referenced to the solvent signals. DSC measurements were performed using a *Perkin Elmer's Pyris 1* with a heating and cooling rate of 20 K min⁻¹ in the temperature range between 0 and 200 °C. Tensile testing was performed on a *ZwickZ005*(Ulm, Germany, ISO 527-1/2) with a drawing speed of 5 mm min^{- 1}. The mechanical properties such as elastic modulus, tensile strength and breaking elongation extracted from measurements at 21 °C by taking the statistical average of four to six test specimens (5A), which were conditioned before testing (24 h, 21 °C, const. humidity. The viscosities were measured on a *MARS* from *Thermo Scientific* using a plate-plate set-up with a plate diameter of 35 mm and a gap of 0.6 mm at various shear rates from 0.1 to 100 s⁻¹ (100 steps, logarithmic, 5 s perstep, 3 s integration time, 25 °C) and the final viscosity received as an average over all 100 values. Significant shear-thinning was usually not observed, as indicated by the standard deviations.

### Synthesis of limonene-based methacrylates (LMA)

Mixtures of limonene dioxide (LDO), methacrylic acid (MA), butylated hydroxytoluene (BHT) and the respective catalyst were added to a flask according to the weight portions described in Table 1. The flask was then lowered into a preheated oil bath, which marked the start of the reaction during which the reaction mixture was vigorously stirred under air atmosphere. The products were received as viscous liquids and used without further purification, or after liquid-liquid extraction in case of LDMA-4. For this extraction the respective LDMA grade was dissolved in 60 ml of dichloromethane and the solution washed four times with 50 ml 1 M K₂CO₃-Solution and one time with distilled water. The organic fraction was dried over MgSO₄ and the solvent subsequently evaporated under reduced pressure.

**Table 1: Weight portions and reaction conditions of limonene-based methacrylate (LMA) syntheses.**

| **Sample code** | **LO** | | **MA** | | **Catalyst** | **Catalyst m[mg]** | **m(BHT) [mg]** | **T [°C]** | **t [h]** |
|---|---|---|---|---|---|---|---|---|---|
| | **m [g]** | **n [mmol]** | **m [g)** | **n [mmol]** | | | | | |
| LDMA-1 | 9.165 | 54,48 | 9.837 | 114.3 | DMI | 379 | 190 | 100 | 6 |
| LDMA-2 | 106,52 | 633.1 | 92.51 | 1074.6 | DMI | 3985 | 796 | 100 | 8 |
| LDMA-3 | 75.11 | 446.5 | 49.93 | 580.0 | DMI | 2510 | 1250 | 100 | 13 |
| LDMA-4 | 23.75 | 141.1 | 36.45 | 423.4 | DMI | 1198 | 120.4 | 100 | 5 |

| | | | | | | | | | |
|---|---|---|---|---|---|---|---|---|---|
| LDMA-1 and LDMA-2 were further purified. | | | | | | | | | |

### Synthesis of LDMA-2-GDMA

Based on the weight portions of the starting materials and the methacrylic acid (MA) turnover of 72 % (calculated from ¹H-NMR spectrum) during the synthesis of LDMA-2, the amount of residual MA was calculated to be 1.48 mmol g⁻¹. LDMA-2 (77.26 g, 114.3 mmol MA) was mixed inside a flask with glycidyl methacrylate (16.25 g, 114.3 mmol) and the mixture was vigorously stirred under air atmosphere at 100°C for 90 minutes. The product was used without further purification.

### Synthesis of LDMA-1-GDMA

Based on the weight portions of the starting materials and the methacrylic acid (MA) turnover of 58 % (calculated from ¹H-NMR spectrum) during the synthesis of LDMA-1, the amount of residual MA was calculated to be 2.60 mmol g⁻¹. LDMA-1 (12.90 g, 33.54 mmol MA) was mixed inside a flask with glycidyl methacrylate (4.768 g, 33.54 mmol) and the mixture was vigorously stirred under air atmosphere at 100°C for 3 h. The product was used without further purification.

### Preparation and curing of acrylate resins

The base resin formulation (BF) consists of 4-methacryloylmorpholine (ACMO) and a polyester-urethane-acrylate prepolymer Laromer UA 9089 in a 59:39-ratio. Additionally 1 wt% diphenyl(2,4,6-trimethylbenzoyl)phosphine oxide (TPO) as a photoinitiator was applied in all cured samples. For the preparation of the acrylate resins, TPO was first dissolved in the respective amount of ACMO and subsequently Laromer homogenized with this solution according to the predefined ratio. Any tested component was afterwards added according to Table 2 and homogenized using a *SpeedMixerDAC 150. 1 FV* from *Hausschild* (2 min, 2500 RPM). Tested component LDMA-1-MgO consists of LDMA-1 and additional MgO (35.85 mg per gram of LDMA-1) but were not added simultaneously. In this case, TPO and MgO were added to ACMO and kept in an ultrasonic bath (10 min, 35 °C) to yield a homogenous dispersion. LDMA-1 was then added in three portions and after each addition the mixture was kept in an ultrasonic bath (10 min, 35 °C). During the last step the turbid dispersion turned translucent. Subsequently the respective amount of Laromer was added and the mixture homogenized using a *SpeedMixer DAC 150.1 FV* from *Hausschild* (2 min, 2500 RPM) to yield a completely transparent resin. The UV curing of the casted samples was performed under a Mercury-vapor lamp (400 W, 280-700 nm, 10 cm distance, 2 x 10 min from both sides) with a thermal post-cure (30 min, 150 °C) in an oven.

**Table 2: Acrylate resins and the respective weight ratios of their acrylate components.**

| **#** | **Resin** | **Tested component (TC)** | **m (BF) : m (TC)** | **m_{f} (TPO) [wt%]** | **m_{f} (MgO) [wt%]** |
|---|---|---|---|---|---|
| | BF^{a} | | 100 : 0 | 1 | 0 |
| | B-1 | LDMA-3 | 90 : 10 | 1 | 0 |
| | B-2 | LDMA-3 | 70 : 30 | 1 | 0 |
| | B-3 | LDMA-3 | 50 : 50 | 1 | 0 |
| | B-4 | LDMA-4_{purified} | 70 : 30 | 1 | 0 |
| | B-5 | BisGMA | 70 : 30 | 1 | 0 |
| | B-6 | LDMA-1 | 70 : 30 | 1 | 0 |
| | B-7 | LDMA-2-GDMA | 70 : 30 | 1 | 0 |
| | B-8 | LDMA-1-GDMA | 70 : 30 | 1 | 0 |
| | B-9 | LDMA-1-MgO | 70 : 30 | 1 | 1.1 |

| | | | | | |
|---|---|---|---|---|---|
| [a] BF = base formulation, which consist of a mixture of ACMO and Laromer in a 59:39-ratio. | | | | | |

## Claims

1. A photocurable composition, comprising
(A) a limonene-based (meth)acrylate obtainable by reacting
a) 1 equivalent of a compound of formula 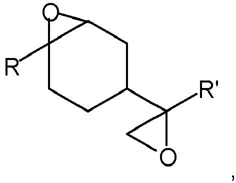 especially 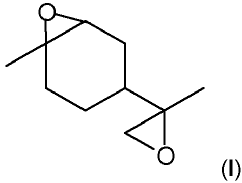 with 1 to 3 equivalents of a compound of formula 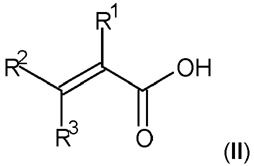 in the presence of a catalyst and a polymerization inhibitor at a temperature of 80 to 120 °C, wherein
R and R' are independently of each other H, or a methyl group,
R¹, R² and R³ are independently of each other H, or a C₁-C₄alkyl group, and
b) optionally reacting the product obtained in step a) with an acid anhydride, or acyl halide;
(B) optionally an oligomer (B),
(C) a diluent (C), and
(D) a photoinitiator (D).

2. The photocurable composition according to claim 1, wherein the viscosity of the photocurable composition is in the range 10 to 3000 mPa·s, preferably 10 to 1500 mPa·s at 30 °C, wherein the viscosity is measured on a *MARS* from *Thermo Scientific* using a plate-plate set-up with a plate diameter of 35 mm and a gap of 0.6 mm at various shear rates from 0.1 to 100 s⁻¹ (100 steps, logarithmic, 5 s per step, 3 s integration time, 25 ° C) and the final viscosity is received as an average over all 100 values.

3. The photocurable composition according to claim 1, or 2, wherein R² and R³ are H.

4. The photocurable composition according to any of claims 1 to 3, wherein R¹ is a methyl group.

5. The photocurable composition according to any of claims 1 to 4, wherein the viscosity of the limonene-based (meth)acrylate (A) obtained by reacting compound (**I**) and (**II**) is in the range of 1 to 500 Pa·s at 25 °C, wherein the viscosity is measured on a *MARS* from *Thermo Scientific* using a plate-plate set-up with a plate diameter of 35 mm and a gap of 0.6 mm at various shear rates from 0.1 to 100 s⁻¹ (100 steps, logarithmic, 5 s per step, 3 s integration time, 25 ° C) and the final viscosity is received as an average over all 100 values.

6. The photocurable composition according to any of claims 1 to 5, wherein the photoinitiator (D) is a compound of the formula XII 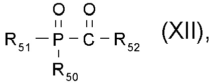 wherein
R₅₀ is unsubstituted cyclohexyl, cyclopentyl, phenyl, naphthyl or biphenylyl; or is cyclohexyl, cyclopentyl, phenyl, naphthyl or biphenylyl substituted by one or more halogen, C₁-C₁₂alkyl, C₁-C₁₂alkoxy, C₁-C₁₂alkylthio or by NR₅₃R₅₄;
or R₅₀ is unsubstituted C₁-C₂₀alkyl or is C₁-C₂₀alkyl which is substituted by one or more halogen, C₁-C₁₂alkoxy, C₁-C₁₂alkylthio, NR₅₃R₅₄ or by -(CO)-O-C₁-C₂₄alkyl;
R₅₁ is unsubstituted cyclohexyl, cyclopentyl, phenyl, naphthyl or biphenylyl; or is cyclohexyl, cyclopentyl, phenyl, naphthyl or biphenylyl substituted by one or more halogen, C₁-C₁₂alkyl, C₁-C₁₂alkoxy, C₁-C₁₂alkylthio or by NR₅₃R₅₄; or R₅₁ is -(CO)R'₅₂; or R₅₁ is C₁-C₁₂alkyl which is unsubstituted or substituted by one or more halogen, C₁-C₁₂alkoxy, C₁-C₁₂alkylthio, or by NR₅₃R₅₄;
R₅₂ and R'₅₂ independently of each other are unsubstituted cyclohexyl, cyclopentyl, phenyl, naphthyl or biphenylyl, or are cyclohexyl, cyclopentyl, phenyl, naphthyl or biphenylyl substituted by one or more halogen, C₁-C₄alkyl or C₁-C₄alkoxy; or R₅₂ is a 5-or 6-membered heterocyclic ring comprising an S atom or N atom;
R₅₃ and R₅₄ independently of one another are hydrogen, unsubstituted C₁-C₁₂alkyl or C₁-C₁₂alkyl substituted by one or more OH or SH wherein the alkyl chain optionally is interrupted by one to four oxygen atoms; or R₅₃ and R₅₄ independently of one another are C₂-C₁₂-alkenyl, cyclopentyl, cyclohexyl, benzyl or phenyl, or the photoinitiator (D) is a mixture of a compound of the formula (XII) and a compound of the formula 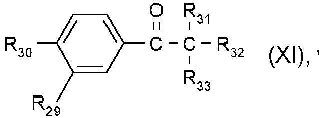 wherein
R₂₉ is hydrogen or C₁-C₁₈alkoxy;
R₃₀ is hydrogen, C₁-C₁₈alkyl, C₁-C₁₂hydroxyalkyl, C₁-C₁₈alkoxy, OCH₂CH₂-OR₃₄,
morpholino, S-C₁-C₁₈alkyl, a group -HC=CH₂, -C(CH₃)=CH₂, 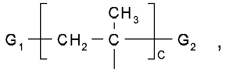 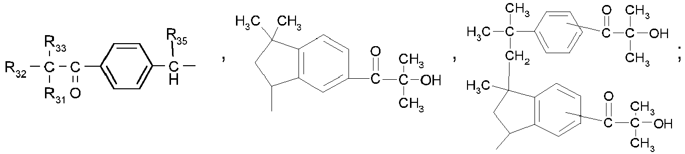 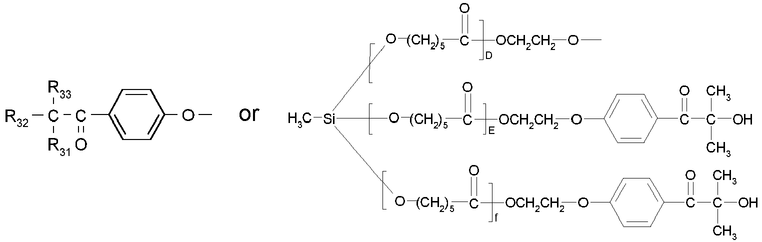
d, e and f are 1-3;
c is 2-10;
G₁ and G₂ independently of one another are end groups of the polymeric structure, preferably hydrogen or methyl;
R₃₄ is hydrogen, 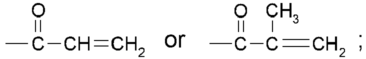
R₃₁ is hydroxy, C₁-C₁₆alkoxy, morpholino, dimethylamino or -O(CH₂CH₂O)_{g}-C₁-C₁₆alkyl;
g is 1-20;
R₃₂ and R₃₃ independently of one another are hydrogen, C₁-C₆alkyl, C₁-C₁₆alkoxy or -O(CH₂CH₂O)_{g}-C₁-C₁₆alkyl; or are unsubstituted phenyl or benzyl; or phenyl or benzyl substituted by C₁-C₁₂-alkyl; or R₃₂ and R₃₃ together with the carbon atom to which they are attached form a cyclohexyl ring;
R₃₅ is hydrogen, OR₃₆ or NR₃₇R₃₈;
R₃₆ is hydrogen, C₁-C₁₂alkyl which optionally is interrupted by one or more nonconsecutive O-atoms and which uninterrupted or interrupted C₁-C₁₂alkyl optionally is substituted by one or more OH,
or R₃₆ is 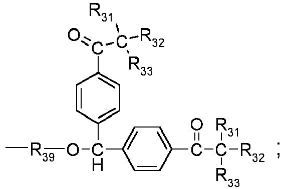
R₃₇ and R₃₈ independently of each other are hydrogen or C₁-C₁₂alkyl which is unsubstituted or is substituted by one or more OH;
R₃₉ is C₁-C₁₂alkylene which optionally is interrupted by one or more nonconsecutive O, -(CO)-NH-C₁-C₁₂alkylene-NH-(CO)- or 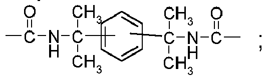
with the proviso that R₃₁, R₃₂ and R₃₃ not all together are C₁-C₁₆alkoxy or -O(CH₂CH₂O)_{g}-C₁-C₁₆alkyl, or the photoinitiator is a mixture of different compounds of the formula (XII), or the photoinitiator is a trialkyl benzoyl, or dialkyl dibenzoyl germanium compound, or the photoinitiator is camphorquinone in combination with a tertiary amine.

7. The photocurable composition according to any of claims 1 to 6, wherein the oligomer (B) is selected from polyester acrylates, polyether acrylates, epoxy acrylates and urethane acrylates.

8. The photocurable composition according to any of claims 1 to 7, wherein the oligomer (B) is obtained by reacting a polyalkylene glycol with caprolactone, isophorone diisocyanate and an hydroxyalkylacrylate, or
the oligomer (B) is obtained by reacting caprolactone, isophorone diisocyanate and an hydroxyalkylacrylate; or
the oligomer (B) is obtained by reacting caprolactone, isophorone diisocyanate and hydroxyethylacrylate; or
the oligomer (B) is obtained by reacting trimethylhexamethylene diisocyanate with hydroxyethylmethacrylate, or
the oligomer (B) is obtained by reacting
(B1) a hydroxyalkylacrylate, or hydroxyalkylmethacrylate,
(B2) an aliphatic diisocyanate, cycloaliphatic diisocyanate, or an aromatic diisocyanate,
(B3) a polyester polyol, which is derived from aliphatic dicarboxylic acids and aliphatic diols, and
(B4) optionally a second polyol, especially glycerol.

9. The photocurable composition according to any of claims 1 to 8, wherein the diluent (C) is selected from glycerol dimethacrylate, N-vinyl-pyrrolidone, vinyl-imidazole, N-vinylcaprolactame, N-(hydroxymethyl)vinylamide, N-hydroxyethyl vinylamide, N-isopropylvinylamide, N-isopropylmethvinylamide, N-tert-butylvinylamide, N,N'-methylenebisvinylamide, N-(isobutoxymethyl)vinylamide, N-(butoxymethyl)vinylamide, N-[3-(dimethylamino)propyl]methvinylamide, N,N-dimethylvinylamide, N,N-diethylvinylamide, N-methyl-N-vinylacetamide, acryloylmorpholine, methacryloylmorpholine, N-(hydroxymethyl)acrylamide, N-hydroxyethyl acrylamide, N-isopropylacrylamide, N-isopropylmethacrylamide, N-tert-butylacrylamide, N,N'-methylenebisacrylamide, N-(isobutoxymethyl)acrylamide, N-(butoxymethyl)acrylamide, N-[3-(dimethylamino)propyl]methacrylamide, N,N-dimethylacrylamide, N,N-diethylacrylamide, N-(hydroxymethyl)methacrylamide, N-hydroxyethyl methacrylamide, N-isopropylmethacrylamide, N-isopropylmethmethacrylamide, N-tert-butylmethacrylamide, N,N'-methylenebismethacrylamide, N-(isobutoxymethyl)methacrylamide, N-(butoxymethyl)methacrylamide, N-[3-(dimethylamino)propyl]methmethacrylamide, N,N-dimethylmethacrylamide and N,N-diethylmethacrylamide, isobornyl methacrylate, tetrahydrofurfuryl methacrylate, ethoxylated phenyl methacrylate, cyclohexylmethacrylate, lauryl methacrylate, stearyl methacrylate, octyl methacrylate, isodecyl methacrylate, tridecyl methacrylate, caprolactone methacrylate, nonyl phenol methacrylate, cyclic trimethylolpropane formal methacrylate, methoxy polyethyleneglycol methacrylates, methoxy polypropyleneglycol methacrylates, hydroxyethyl methacrylate, hydroxypropyl methacrylate, glycidyl methacrylate, 1,3-butylene glycol di(meth)acrylate, 1,4-butanediol di(meth)acrylate, ethylene glycol di(meth)acrylate, diethylene glycol di(meth)acrylate, triethylene glycol di(meth)acrylate, 1,6-hexanediol di(meth)acrylate, polyethylene glycol (200) di(meth)acrylate, tetraethylene glycol di(meth)acrylate, triethylene glycol di(meth)acrylate, tripropylene glycol di(meth)acrylate, polyethylene glycol (400) di(meth)acrylate, ethoxylated (3) bisphenol A di(meth)acrylate, dipropylene glycol di(meth)acrylate, alkoxylated hexanediol di(meth)acrylate, ethoxylated (4) bisphenol A di(meth)acrylate, ethoxylated (10) bisphenol A di(meth)acrylate, polyethylene glycol (600) di(meth)acrylate, tricyclodecane dimethanol di(meth)acrylate, 1,9-nonanediol di(meth)acrylate, 1,10-decanediol di(meth)acrylate, neopentyl glycol di(meth)acrylate, polytetramethylene glycol di(meth)acrylate, 3-methyl-1,5-pentanediol di(meth)acrylate, dimethylol-tricyclodecane di(meth)acrylate, bistrimethylolpropane tetraacrylate, pentaerythritol tetracrylate, tetramethylolmethane tetramethacrylate, pentaerythritol tetramethacrylate, bistrimethylolpropane tetramethacrylate, ethoxylated pentaerythritol tetraacrylate, propoxylated pentaerythritol tetraacrylate, dipentaerythritol tetraacrylate, ethoxylated dipentaerythritol tetraacrylate and propoxylated dipentaerythritol tetraacrylate and mixtures thereof.

10. A method for producing a three-dimensional article, comprising
a) providing the photocurable composition according to any one of claims 1 to 9,
b) exposing the photocurable composition to actinic radiation to form a cured crossection,
c) repeating steps (a) and (b) to build up a three-dimensional article.

11. The method according to claim 10, comprising a vat photopolymerization, wherein the photocurable composition according to claims 1 to 9 in step b) is cured directly onto a translated or rotated substrate, and the irradiation is patterned via stereolithography, holography, or digital light projection (DLP).

12. The method according to claim 10, comprising
a) applying a layer of the photocurable composition of any of claims 1 to 9 onto a surface;
b) exposing the layer imagewise to actinic radiation to form an imaged cured cross-section;
c) applying a second layer of the photocurable composition onto the previously exposed imaged cross-section;
d) exposing the layer from step (c) imagewise to actinic radiation to form an additional imaged cross-section, wherein the radiation causes curing of the second layer in the exposed areas and adhesion to the previously exposed cross-section; and
e) repeating steps (c) and (d) in order to build up a three-dimensional article.

13. A three-dimensional article, which is a cured product of the photocurable composition according to any of claims 1 to 9 and/or which is produced by the method of any of claims 10 to 12.

14. Use of the photocurable composition according to any one of claims 1 to 9, or a limonene-based (meth)acrylate, obtainable by
a) reacting a) 1 equivalent of a compound of formula 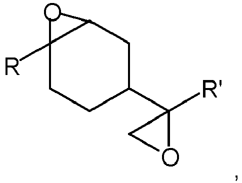 especially 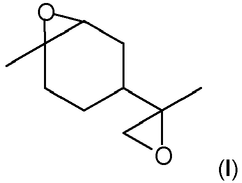 with 1 to 3 equivalents of a compound of formula 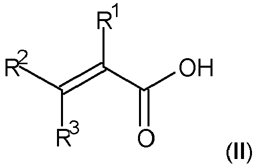 in the presence of a catalyst and an inhibitor at a temperature of 80 to 120 °C, wherein R and R' are independently of each other H, or a methyl group, R¹, R² and R³ are independently of each other H, or a C₁-C₄alkyl group, especially a methyl group, in a photopolymerization 3D printing process.

## Patentansprüche

1. Photohärtbare Zusammensetzung, umfassend
(A) ein (Meth)acrylat auf Limonen-Basis, das erhältlich ist durch Umsetzung von
a) 1 Äquivalent einer Verbindung der Formel 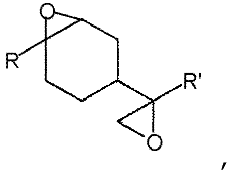 insbesondere 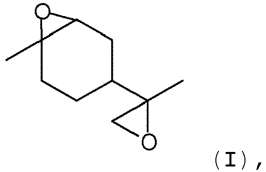 mit 1 bis 3 Äquivalenten einer Verbindung der Formel 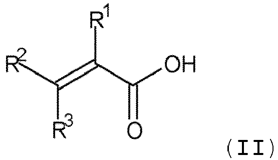 in Gegenwart eines Katalysators und eines Polymerisationsinhibitors bei einer Temperatur von 80 bis 120°C, wobei
R und R' unabhängig voneinander für H oder eine Methylgruppe stehen,
R¹, R² und R³ unabhängig voneinander für H oder eine C₁-C₄-Alkylgruppe stehen, und
b) gegebenenfalls Umsetzen des in Schritt a) erhaltenen Produkts mit einem Säureanhydrid oder Acylhalogenid;
(B) gegebenenfalls ein Oligomer (B),
(C) ein Verdünnungsmittel (C) und
(D) einen Photoinitiator (D).

2. Photohärtbare Zusammensetzung nach Anspruch 1, wobei die Viskosität der photohärtbaren Zusammensetzung im Bereich von 10 bis 3000 mPa·s, vorzugsweise 10 bis 1500 mPa·s, bei 30°C liegt, wobei die Viskosität auf einem *MARS* von *Thermo Scientific* unter Verwendung eines Platte-Platte-Aufbaus mit einem Plattendurchmesser von 35 mm und einem Spalt von 0,6 mm bei verschiedenen Scherraten von 0,1 bis 100 s⁻¹ (100 Schritte, logarithmisch, 5 s pro Schritt, 3 s Integrationszeit, 25 °C) gemessen wird und die letztendliche Viskosität als Durchschnitt über alle 100 Werte erhalten wird.

3. Photohärtbare Zusammensetzung nach Anspruch 1 oder 2, wobei R² und R³ für H stehen.

4. Photohärtbare Zusammensetzung nach einem der Ansprüche 1 bis 3, wobei R¹ für eine Methylgruppe steht.

5. Photohärtbare Zusammensetzung nach einem der Ansprüche 1 bis 4, wobei die Viskosität des durch Umsetzen von Verbindung (I) und (II) erhaltenen (Meth)acrylats auf Limonen-Basis (A) im Bereich von 1 bis 500 Pa·s bei 25 °C liegt, wobei die Viskosität auf einem *MARS* von *Thermo Scientific* unter Verwendung eines Platte-Platte-Aufbaus mit einem Plattendurchmesser von 35 mm und einem Spalt von 0,6 mm bei verschiedenen Scherraten von 0,1 bis 100 s⁻¹ (100 Schritte, logarithmisch, 5 s pro Schritt, 3 s Integrationszeit, 25 °C) gemessen wird und die letztendliche Viskosität als Durchschnitt über alle 100 Werte erhalten wird.

6. Photohärtbare Zusammensetzung nach einem der Ansprüche 1 bis 5, wobei es sich bei dem Photoinitiator (D) um eine Verbindung der Formel XII 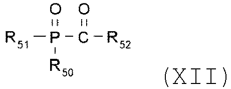 handelt, wobei
R₅₀ für unsubstituiertes Cyclohexyl, Cyclopentyl, Phenyl, Naphthyl oder Biphenylyl steht oder für Cyclohexyl, Cyclopentyl, Phenyl, Naphthyl oder Biphenylyl, das durch ein oder mehrere Halogen, C₁-C₁₂-Alkyl, C₁-C₁₂-Alkoxy, C₁-C₁₂-Alkylthio oder durch NR₅₃R₅₄ substituiert ist, steht;
oder R₅₀ für unsubstituiertes C₁-C₂₀-Alkyl oder C₁-C₂₀-Alkyl, das durch ein oder mehrere Halogen, C₁-C₁₂-Alkoxy, C₁-C₁₂-Alkylthio, NR₅₃R₅₄ oder durch - (CO)-O-C₁-C₂₄-Alkyl substituiert ist, steht;
R₅₁ für unsubstituiertes Cyclohexyl, Cyclopentyl, Phenyl, Naphthyl oder Biphenylyl steht oder für Cyclohexyl, Cyclopentyl, Phenyl, Naphthyl oder Biphenylyl, das durch ein oder mehrere Halogen, C₁-C₁₂-Alkyl, C₁-C₁₂-Alkoxy, C₁-C₁₂-Alkylthio oder durch NR₅₃R₅₄ substituiert ist, steht; oder R₅₁ für - (CO)R'₅₂ steht; oder R₅₁ für C₁-C₁₂-Alkyl, das unsubstituiert oder durch ein oder mehrere Halogen, C₁-C₁₂-Alkoxy, C₁-C₁₂-Alkylthio oder durch NR₅₃R₅₄ substituiert ist, steht;
R₅₂ und R'₅₂ jeweils unabhängig voneinander für unsubstituiertes Cyclohexyl, Cyclopentyl, Phenyl, Naphthyl oder Biphenylyl stehen oder für Cyclohexyl, Cyclopentyl, Phenyl, Naphthyl oder Biphenylyl, das durch ein oder mehrere Halogen, C₁-C₄-Alkyl oder C₁-C₄-Alkoxy substituiert ist, stehen; oder R₅₂ für einen 5- oder 6-gliedrigen heterocyclischen Ring mit einem S-Atom oder einem-Atom steht;
R₅₃ und R₅₄ unabhängig voneinander für Wasserstoff, unsubstituiertes C₁-C₁₂-Alkyl oder C₁-C₁₂-Alkyl, das durch ein oder mehrere OH oder SH substituiert ist, stehen, wobei die Alkylkette gegebenenfalls durch ein bis vier Sauerstoffatome unterbrochen ist; oder R₅₃ und R₅₄ unabhängig voneinander für C₂-C₂₂-Alkenyl, Cyclopentyl, Cyclohexyl, Benzyl oder Phenyl stehen; oder es sich bei dem Photoinitiator (D) um eine Mischung einer Verbindung der Formel (XII) und einer Verbindung der Formel 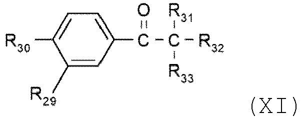 handelt, wobei
R₂₉ für Wasserstoff oder C₁-C₁₈-Alkoxy steht;
R₃₀ für Wasserstoff, C₁-C₁₈-Alkyl, C₁-C₁₂-Hydroxyalkyl, C₁-C₁₈-Alkoxy, OCH₂CH₂-OR₃₄, Morpholino, S-C₁-C₁₈-Alkyl, eine Gruppe -HC=CH₂, -C(CH₃)=CH₂, 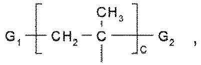 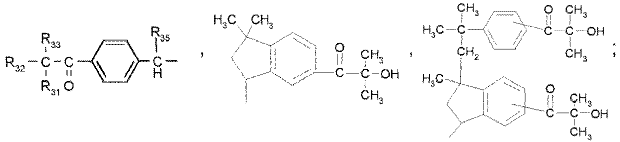 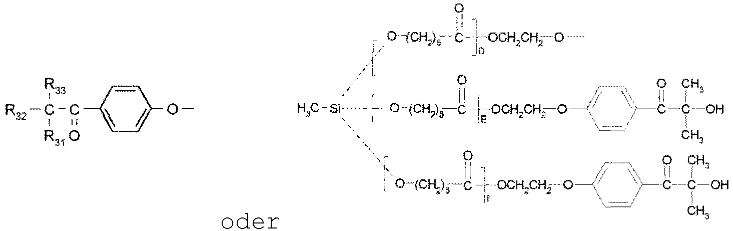 steht;
d, e und f für 1-3 stehen;
c für 2-10 steht;
G₁ und G₂ unabhängig voneinander für Endgruppen der polymeren Struktur, vorzugsweise Wasserstoff oder Methyl, stehen;
R₃₄ für Wasserstoff, 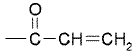 oder 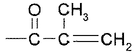 steht;
R₃₁ für Hydroxy, C₁-C₁₆-Alkoxy, Morpholino, Dimethylamino oder -O(CH₂CH₂O)_{g}-C₁-C₁₆-Alkyl steht;
g für 1-20 steht;
R₃₂ und R₃₃ unabhängig voneinander für Wasserstoff, C₁-C₆-Alkyl, C₁-C₁₆-Alkoxy oder -O(CH₂CH₂O)_{g}-C₁-C₁₆-Alkyl stehen; oder für unsubstituiertes Phenyl oder Benzyl; oder Phenyl oder Benzyl, das durch C₁-C₁₂-Alkyl substituiert ist, stehen; oder R₃₂ und R₃₃ zusammen mit dem Kohlenstoffatom, an das sie gebunden sind, einen Cyclohexylring bilden;
R₃₅ für Wasserstoff, OR₃₆ oder NR₃₇R₃₈ steht;
R₃₆ für Wasserstoff, C₁-C₁₂-Alkyl, das gegebenenfalls durch ein oder mehrere nicht aufeinanderfolgende O-Atome unterbrochen ist, wobei das nicht unterbrochene oder unterbrochene C₁-C₁₂-Alkyl gegebenenfalls durch ein oder mehrere OH substituiert ist, steht
oder R₃₆ für 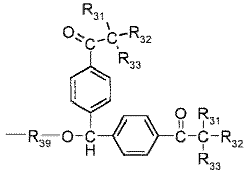 steht;
R₃₇ und R₃₈ unabhängig voneinander für Wasserstoff oder C₁-C₁₂-Alkyl, das unsubstituiert oder durch ein oder mehrere OH substituiert ist, stehen;
R₃₉ für C₁-C₁₂-Alkylen, das gegebenenfalls durch ein oder mehrere nicht aufeinanderfolgende O unterbrochen ist, -(CO)-NH-C₁-C₁₂-Alkylen-NH-(CO)-oder 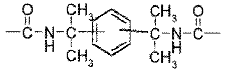 steht;
mit der Maßgabe, dass R₃₁, R₃₂ und R₃₃ nicht alle zusammen für C₁-C₁₆-Alkoxy oder -O(CH₂CH₂O)_{g}-C₁-C₁₆-Alkyl stehen, oder es sich bei dem Photoinitiator um eine Mischung von verschiedenen Verbindungen der Formel (XII) handelt oder es sich bei dem Photoinitiator um eine Trialkylbenzoyl- oder Dialkyldibenzoylgermaniumverbindung handelt oder es sich bei dem Photoinitiator um Campherchinon in Kombination mit einem tertiären Amin handelt.

7. Photohärtbare Zusammensetzung nach einem der Ansprüche 1 bis 6, wobei das Oligomer (B) aus Polyesteracrylaten, Polyetheracrylaten, Epoxyacrylaten und Urethanacrylaten ausgewählt ist.

8. Photohärtbare Zusammensetzung nach einem der Ansprüche 1 bis 7, wobei das Oligomer (B) durch Umsetzen eines Polyoxyalkylenglykols mit Caprolacton, Isophorondiisocyanat und einem Hydroxyalkylacrylat erhalten wird; oder
das Oligomer (B) durch Umsetzen von Caprolacton, Isophorondiisocyanat und einem Hydroxyalkylacrylat erhalten wird; oder
das Oligomer (B) durch Umsetzen von Caprolacton, Isophorondiisocyanat und Hydroxyethylacrylat erhalten wird; oder
das Oligomer (B) durch Umsetzen von Trimethylhexamethylendiisocyanat mit Hydroxyethylacrylat erhalten wird; oder
das Oligomer (B) durch Umsetzen von
(B1) einem Hydroxyalkylacrylat oder Hydroxyalkyl-(meth)acrylat,
(B2) einem aliphatischen Diisocyanat, cycloaliphatischen Diisocyanat oder einem aromatischen Diisocyanat,
(B3) einem Polyesterpolyol, das sich von aliphatischen Dicarbonsäuren und aliphatischen Diolen ableitet, und
(B4) gegebenenfalls einem zweiten Polyol, insbesondere Glycerin,
erhalten wird.

9. Photohärtbare Zusammensetzung nach einem der Ansprüche 1 bis 8, wobei das Verdünnungsmittel (C) aus Glycerindimethacrylat, N-Vinylpyrrolidon, Vinylimidazol, N-Vinylcaprolactam, N-(Hydroxymethyl)vinylamid, N-Hydroxyethylvinylamid, N-Isopropylvinylamid, N-Isopropylmethvinylamid, N-tert-Butylvinylamid, N,N'-Methylenbisvinylamid, N-(Isobutoxymethyl)vinylamid, N-(Butoxymethyl)vinylamid, N-[3-(Dimethylamino)propyl]methvinylamid, N,N-Dimethylvinylamid, N,N-Diethylvinylamid, N-Methyl-N-vinylacetamid, Acryloylmorpholin, Methacryloylmorpholin, N-(Hydroxymethyl)acrylamid, N- Hydroxyethylacrylamid, N-Isopropylacrylamid, N-Isopropylmethacrylamid, N-tert-Butylacrylamid, N,N'-Methylenbisacrylamid, N-(Isobutoxymethyl)acrylamid, N-(Butoxymethyl)acrylamid, N-[3-(Dimethylamino)propyl]methacrylamid, N,N-Dimethylacrylamid, N,N-Diethylacrylamid, N-(Hydroxymethyl)-methacrylamid, N-Hydroxyethylmethacrylamid, N-Isopropylmethacrylamid, N-Isopropylmethmethacrylamid, N-tert-Butylmethacrylamid, N,N'-Methylenbismethacrylamid, N-(Isobutoxymethyl)methacrylamid, N-(Butoxymethyl)methacrylamid, N-[3-(Dimethylamino)propyl]methmethacrylamid, N,N-Dimethylmethacrylamid und N,N-Diethylmethacrylamid, Isobornylmethacrylat, Tetrahydrofurfurylmethacrylat, ethoxyliertem Phenylmethacrylat, Cyclohexylmethacrylat, Laurylmethacrylat, Stearylmethacrylat, Octylmethacrylat, Isodecylmethacrylat, Tridecylmethacrylat, Caprolactonmethacrylat, Nonylphenolmethacrylat, cyclischem Trimethylolpropanformalmethacrylat, Methoxypolyethylenglykolmethacrylaten, Methoxypolypropylenglykolmethacrylaten, Hydroxyethylmethacrylat, Hydroxypropylmethacrylat, Glycidylmethacrylat, 1,3-Butylenglykoldi(meth)acrylat, 1,4-Butandioldi(meth)acrylat, Ethylenglykoldi(meth)acrylat, Diethylenglykoldi(meth)acrylat, Triethylenglykoldi(meth)acrylat, 1,6-Hexandioldi(meth)acrylat, Polyethylenglykol-(200)-di(meth)acrylat, Tetraethylenglykoldi(meth)acrylat, Triethylenglykoldi(meth)acrylat, Tripropylenglykoldi(meth)acrylat, Polyethylenglykol-(400)-di(meth)acrylat, ethoxyliertem (3) Bisphenol-A-di(meth)acrylat, Dipropylenglykoldi(meth)acrylat, alkoxyliertem Hexandioldi(meth)acrylat, ethoxyliertem (4) Bisphenol-A-di(meth)acrylat, ethoxyliertem (10) Bisphenol-A-di(meth)acrylat, Polyethylenglykol-(600)-di(meth)acrylat, Tricyclodecandimethanoldi(meth)acrylat, 1,9-Nonandioldi(meth)acrylat, 1,10-Decandioldi(meth)acrylat, Neopentylglykoldi(meth)acrylat, Polytetramethylenglykoldi(meth)acrylat, 3-Methyl-1,5-pentandioldi(meth)acrylat, Dimethyloltricyclodecandi(meth)acrylat, Bistrimethylolpropantetraacrylat, Pentaerythritoltetracrylat, Tetramethylolmethantetramethacrylat, Pentaerythritoltetramethacrylat, Bistrimethylolpropantetramethacrylat, ethoxyliertem Pentaerythritoltetraacrylat, propoxyliertem Pentaerythritoltetraacrylat, Dipentaerythritoltetraacrylat, ethoxyliertem Dipentaerythritoltetraacrylat und propoxyliertem Dipentaerythritoltetraacrylat und Mischungen davon ausgewählt ist.

10. Verfahren zur Herstellung eines dreidimensionalen Gegenstands, das Folgendes umfasst:
a) Bereitstellen der photohärtbare Zusammensetzung nach einem der Ansprüche 1 bis 9,
b) Belichten der photohärtbaren Zusammensetzung mit aktinischer Strahlung zur Bildung eines gehärteten Querschnitts,
c) Wiederholen der Schritte (a) und (b) zum Aufbauen eines dreidimensionalen Gegenstands.

11. Verfahren nach Anspruch 10, umfassend eine Vat-Photopolymerisation, wobei die photohärtbare Zusammensetzung nach den Ansprüchen 1 bis 9 in Schritt b) direkt auf einem translatierten oder gedrehten Substrat gehärtet wird und die Bestrahlung mittels Stereolithographie, Holographie oder digitaler Lichtprojektion (DLP) strukturiert wird.

12. Verfahren nach Anspruch 10, das Folgendes umfasst:
a) Aufbringen einer Schicht der photohärtbaren Zusammensetzung nach einem der Ansprüche 1 bis 9 auf eine Oberfläche;
b) bildmäßiges Belichten der Schicht mit aktinischer Strahlung zur Bildung eines bebilderten gehärteten Querschnitts;
c) Aufbringen einer zweiten Schicht der photohärtbaren Zusammensetzung auf den vorher belichteten bebilderten Querschnitt;
d) bildmäßiges Belichten der Schicht aus Schritt (c) mit aktinischer Strahlung zur Bildung eines zusätzlichen bebilderten Querschnitts, wobei die Strahlung das Härten der zweiten Schicht in den belichteten Bereichen und Haftung auf dem vorher belichteten Querschnitt bewirkt; und
e) Wiederholen der Schritte (c) und (d) zum Aufbauen eines dreidimensionalen Gegenstands.

13. Dreidimensionaler Gegenstand, bei dem es sich um ein gehärtetes Produkt der photohärtbaren Zusammensetzung nach einem der Ansprüche 1 bis 9 handelt und/oder der durch das Verfahren nach einem der Ansprüche 10 bis 12 hergestellt wird.

14. Verwendung der photohärtbaren Zusammensetzung nach einem der Ansprüche 1 bis 9 oder eines (Meth)acrylats auf Limonen-Basis, das erhältlich ist durch
a) Umsetzung von a) 1 Äquivalent einer Verbindung der Formel 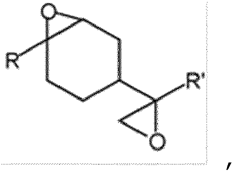 insbesondere 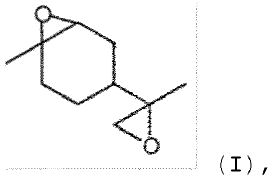 mit 1 bis 3 Äquivalenten einer Verbindung der Formel 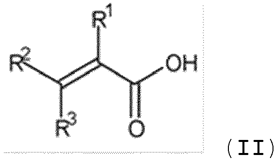 in Gegenwart eines Katalysators und eines Inhibitors bei einer Temperatur von 80 bis 120°C, wobei R und R' unabhängig voneinander für H oder eine Methylgruppe stehen, R¹, R² und R³ unabhängig voneinander für H oder eine C₁-C₄-Alkylgruppe, insbesondere eine Methylgruppe, stehen, bei einem Photopolymerisations-3D-Druckprozess.

## Revendications

1. Composition photodurcissable, comprenant :
(A) un (méth)acrylate à base de limonène pouvant être obtenu par réaction de
a) 1 équivalent d'un composé de formule 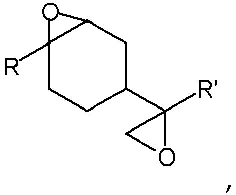 spécialement 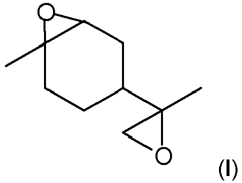 avec 1 à 3 équivalents d'un composé de formule 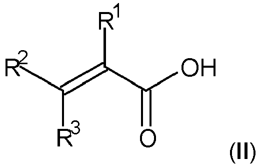 en présence d'un catalyseur et d'un inhibiteur de polymérisation à une température de 80 à 120 °C, dans laquelle
R et R' représentent indépendamment l'un de l'autre H ou un groupement méthyle,
R¹, R² et R³ représentent indépendamment les uns des autres H ou un groupement alkyle en C₁-C₄, et
b) réaction facultative du produit obtenu à l'étape a) avec un anhydride d'acide ou un halogénure d'acyle ;
(B) éventuellement un oligomère (B),
(C) un diluant (C), et
(D) un photoinitiateur (D).

2. Composition photodurcissable selon la revendication 1, dans laquelle la viscosité de la composition photodurcissable est dans la plage allant de 10 à 3000 mPa·s, préférentiellement de 10 à 1500 mPa·s à 30 °C, dans laquelle la viscosité est mesurée sur un dispositif *MARS* de *Thermo Scientific* en utilisant une configuration plaque-plaque avec un diamètre de plaque de 35 mm et un interstice de 0,6 mm à diverses vitesses de cisaillement entre 0,1 et 100 s⁻¹ (100 incréments, logarithmique, 5 s par incrément, 3 s de temps d'intégration, 25 °C) et la viscosité finale est reçue sous forme de moyenne sur la totalité des 100 valeurs.

3. Composition photodurcissable selon la revendication 1 ou 2, dans laquelle R² et R³ représentent H.

4. Composition photodurcissable selon l'une quelconque des revendications 1 à 3, dans laquelle R¹ représente un groupement méthyle.

5. Composition photodurcissable selon l'une quelconque des revendications 1 à 4, dans laquelle la viscosité du (méth)acrylate à base de limonène (A) obtenu par réaction des composés (I) et (II) est dans la plage allant de 1 à 500 Pa·s à 25 °C, dans laquelle la viscosité est mesurée sur un dispositif *MARS* de *Thermo Scientific* en utilisant une configuration plaque-plaque avec un diamètre de plaque de 35 mm et un interstice de 0,6 mm à diverses vitesses de cisaillement entre 0,1 et 100 s⁻¹ (100 incréments, logarithmique, 5 s par incrément, 3 s de temps d'intégration, 25 °C) et la viscosité finale est reçue sous forme de moyenne sur la totalité des 100 valeurs.

6. Composition photodurcissable selon l'une quelconque des revendications 1 à 5, dans laquelle le photoinitiateur (D) est un composé de formule XII 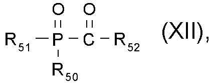 dans laquelle
R₅₀ représente un groupement non substitué cyclohexyle, cyclopentyle, phényle, naphtyle ou biphénylyle ; ou représente un groupement cyclohexyle, cyclopentyle, phényle, naphtyle ou biphénylyle substitué par un ou plusieurs groupements halogène, alkyle en C₁-C₁₂, alkoxy en C₁-C₁₂, (alkyle en C₁-C₁₂)-thio ou par NR₅₃R₅₄ ;
ou R₅₀ représente un groupement non substitué alkyle en C₁-C₂₀ ou représente un groupement alkyle en C₁-C₂₀ qui est substitué par un ou plusieurs groupements halogène, alkoxy en C₁-C₁₂, (alkyle en C₁-C₁₂) -thio, NR₅₃R₅₄ ou par - (CO)-O-(alkyle en C₁-C₂₄) ;
R₅₁ représente un groupement non substitué cyclohexyle, cyclopentyle, phényle, naphtyle ou biphénylyle ; ou représente un groupement cyclohexyle, cyclopentyle, phényle, naphtyle ou biphénylyle substitué par un ou plusieurs groupements halogène, alkyle en C₁-C₁₂, alkoxy en C₁-C₁₂, (alkyle en C₁-C₁₂)-thio ou par NR₅₃R₅₄ ; ou R₅₁ représente - (CO)R'₅₂ ; ou R₅₁ représente un groupement alkyle en C₁-C₁₂ qui est non substitué ou substitué par un ou plusieurs groupements halogène, alkoxy en C₁-C₁₂, (alkyle en C₁-C₁₂) -thio, ou par NR₅₃R₅₄ ;
R₅₂ et R'₅₂ représentent indépendamment l'un de l'autre un groupement non substitué cyclohexyle, cyclopentyle, phényle, naphtyle ou biphénylyle, ou représentent un groupement cyclohexyle, cyclopentyle, phényle, naphtyle ou biphénylyle substitué par un ou plusieurs groupements halogène, alkyle en C₁-C₄ ou alkoxy en C₁-C₄ ; ou R₅₂ représente un cycle hétérocyclique à 5 ou 6 chaînons comprenant un atome S ou un atome N ;
R₅₃ et R₅₄ représentent indépendamment l'un de l'autre un hydrogène, un groupement non substitué alkyle en C₁-C₁₂ ou un groupement alkyle en C₁-C₁₂ substitué par un ou plusieurs OH ou SH dans laquelle la chaîne alkyle est éventuellement interrompue par un à quatre atomes d'oxygène ; ou R₅₃ et R₅₄ représentent indépendamment l'un de l'autre un groupement alcényle en C₂-C₁₂, cyclopentyle, cyclohexyle, benzyle ou phényle, ou le photoinitiateur (D) est un mélange d'un composé de formule (XII) et d'un composé de formule 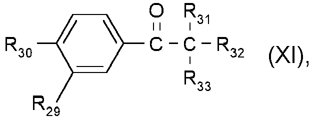 dans laquelle
R₂₉ représente un atome d'hydrogène ou un groupement alkoxy en C₁-C₁₈ ;
R₃₀ représente un hydrogène ou un groupement alkyle en C₁-C₁₈, hydroxyalkyle en C₁-C₁₂, alkoxy en C₁-C₁₈, OCH₂CH₂-OR₃₄, morpholino, S-alkyle en C₁-C₁₈, un groupement - HC=CH₂, -C(CH₃)=CH₂, 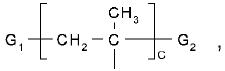 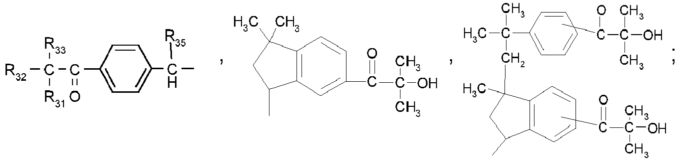 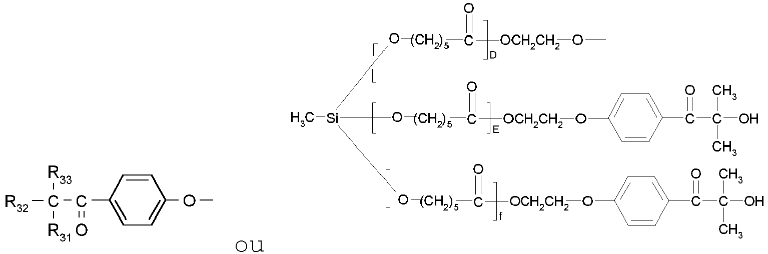
d, e et f représentent 1 à 3 ;
c représente 2 à 10 ;
G₁ et G₂ représentent indépendamment l'un de l'autre des groupements terminaux de la structure polymère, préférentiellement un atome d'hydrogène ou un groupement méthyle ;
R₃₄ représente un hydrogène, 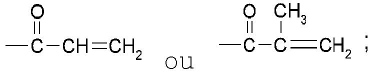
R₃₁ représente un groupement hydroxy, alkoxy en C₁-C₁₆, morpholino, diméthylamino ou -O(CH₂CH₂O)_{g}-alkyle en C₁-C16 ;
g représente 1 à 20 ;
R₃₂ et R₃₃ représentent indépendamment l'un de l'autre un hydrogène ou un groupement alkyle en C₁-C₆, alkoxy en C₁-C₁₆ ou -O(CH₂CH₂O)_{g}-alkyle en C₁-C₁₆ ; ou représentent un groupement non substitué phényle ou benzyle ; ou un groupement phényle ou benzyle substitué par un groupement alkyle en C₁-C₁₂ ; ou R₃₂ et R₃₃ forment conjointement avec l'atome de carbone auquel ils sont liés un cycle cyclohexyle ;
R₃₅ représente un hydrogène, OR₃₆ ou NR₃₇R₃₈ ;
R₃₆ représente un hydrogène, un groupement alkyle en C₁-C₁₂ qui est éventuellement interrompu par un ou plusieurs atomes O non consécutifs et lequel groupement non interrompu ou interrompu alkyle en C₁-C₁₂ est éventuellement substitué par un ou plusieurs OH,
ou R₃₆ représente 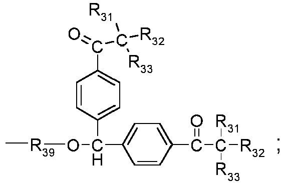 R₃₇ et R₃₈ représentent indépendamment l'un de l'autre un hydrogène ou un groupement alkyle en C₁-C₁₂ qui est non substitué ou substitué par un ou plusieurs OH ;
R₃₉ représente un groupement alkylène en C₁-C₁₂ qui est éventuellement interrompu par un ou plusieurs groupements non consécutifs O, -(CO)-NH-(alkylène en C₁-C₁₂)-NH- (CO) - ou 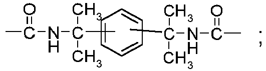
à la condition que R₃₁, R₃₂ et R₃₃ ne représentent pas tous ensemble un groupement alkoxy en C₁-C₁₆ ou - O(CH₂CH₂O)_{g}-alkyle en C₁-C₁₆, ou le photoinitiateur représente un mélange des différents composés de formule (XII), ou le photoinitiateur est un composé de trialkylbenzoyl ou de dialkyldibenzoyl germanium, ou le photoinitiateur est la camphorquinone en association avec une amine tertiaire.

7. Composition photodurcissable selon l'une quelconque des revendications 1 à 6, dans laquelle l'oligomère (B) est choisi parmi les polyester-acrylates, les polyéther-acrylates, les époxy-acrylates et les uréthane-acrylates.

8. Composition photodurcissable selon l'une quelconque des revendications 1 à 7, dans laquelle l'oligomère (B) est obtenu par réaction d'un polyalkylène glycol avec de la caprolactone, de l'isophorone diisocyanate et d'un hydroxyalkylacrylate, ou
l'oligomère (B) est obtenu par réaction de caprolactone, d'isophorone diisocyanate et d'un hydroxyalkylacrylate ; ou
l'oligomère (B) est obtenu par réaction de caprolactone, d'isophorone diisocyanate et d'hydroxyéthylacrylate ; ou
l'oligomère (B) est obtenu par réaction de triméthylhexaméthylène diisocyanate avec de l'hydroxyéthylméthacrylate, ou
l'oligomère (B) est obtenu par réaction
(B1) d'hydroxyalkylacrylate, ou d'hydroxyalkylméthacrylate,
(B2) d'un diisocyanate aliphatique, d'un diisocyanate cycloaliphatique ou d'un diisocyanate aromatique,
(B3) d'un polyester polyol, qui est dérivée d'acides dicarboxyliques aliphatiques et de diols aliphatiques, et
(B4) éventuellement d'un second polyol, spécialement le glycérol.

9. Composition photodurcissable selon l'une quelconque des revendications 1 à 8, dans laquelle le diluant (C) est choisi parmi le glycérol diméthacrylate, la N-vinyl-pyrrolidone, le vinyl-imidazole, le N-vinylcaprolactame, le N-(hydroxyméthyl)vinylamide, le N-hydroxyéthylvinylamide, le N-isopropylvinylamide, le N-isopropylméthvinylamide, le N-tert-butylvinylamide, le N,N'-méthylènebisvinylamide, le N-(isobutoxyméthyl)vinylamide, le N-(butoxyméthyl)vinylamide, le N-[3-(diméthylamino)propyl]méthvinylamide, le N,N-diméthylvinylamide, le N,N-diéthylvinylamide, le N-méthyl-N-vinylacétamide, l'acryloylmorpholine, la méthacryloylmorpholine, le N-(hydroxyméthyl)acrylamide, le N-hydroxyéthylacrylamide, le N-isopropylacrylamide, le N-isopropylméthacrylamide, le N-tert-butylacrylamide, le N,N'-méthylènebisacrylamide, le N-(isobutoxyméthyl)acrylamide, le N-(butoxyméthyl)acrylamide, le N-[3-(diméthylamino)propyl]méthacrylamide, le N,N-diméthylacrylamide, le N,N-diéthylacrylamide, le N-(hydroxyméthyl)méthacrylamide, le N-hydroxyéthylméthacrylamide, le N-isopropylméthacrylamide, le N-isopropylméthméthacrylamide, le N-tert-butylméthacrylamide, le N,N'-méthylènebisméthacrylamide, le N-(isobutoxyméthyl)méthacrylamide, le N-(butoxyméthyl)méthacrylamide, le N-[3-(diméthylamino)propyl]méthméthacrylamide, le N,N-diméthylméthacrylamide et le N,N-diéthylméthacrylamide, l'isobornylméthacrylate, le tétrahydrofurfurylméthacrylate, le phénylméthacrylate éthoxylé, le cyclohexylméthacrylate, le laurylméthacrylate, le stéarylméthacrylate, l'octylméthacrylate, l'isodécylméthacrylate, le tridécylméthacrylate, le caprolactone méthacrylate, le nonylphénolméthacrylate, le (triméthylolpropane cyclique)formalméthacrylate, les méthoxypolyéthylèneglycol méthacrylates, les méthoxypolypropylèneglycol méthacrylates, l'hydroxyéthylméthacrylate, l'hydroxypropylméthacrylate, le glycidylméthacrylate, le 1,3-butylène glycol di(méth)acrylate, le 1,4-butanediol di(méth)acrylate, l'éthylene glycol di(méth)acrylate, le diéthylene glycol di(méth)acrylate, le triéthylene glycol di(méth)acrylate, le 1,6-hexanediol di(méth)acrylate, le polyéthylène glycol (200) di(méth)acrylate, le tétraéthylene glycol di(méth)acrylate, le triéthylène glycol di(méth)acrylate, le tripropylène glycol di(méth)acrylate, le polyéthylène glycol (400) di(méth)acrylate, le (3) bisphénol A di(méth)acrylate éthoxylé, le dipropylène glycol di(méth)acrylate, l'hexanediol di(méth)acrylate alkoxylé, le (4) bisphénol A di(méth)acrylate éthoxylé, le (10) bisphénol A di(méth)acrylate éthoxylé, le polyéthylène glycol (600) di(méth)acrylate, le tricyclodécane diméthanol di(méth)acrylate, le 1,9-nonanediol di(méth)acrylate, le 1,10-décanediol di(méth)acrylate, le néopentylglycol di(méth)acrylate, le polytétraméthylène glycol di(méth)acrylate, le 3-méthyl-1,5-pentanediol di(méth)acrylate, le diméthyloltricyclodécane di(méth)acrylate, le bistriméthylolpropane tétracrylate, le pentaérythritol tétracrylate, le tétraméthylolméthane tétraméthacrylate, le pentaérythritol tétraméthacrylate, le bistriméthylolpropane tétraméthacrylate, le pentaérythritol tétracrylate éthoxylé, le pentaérythritol tétracrylate propoxylé, le dipentaérythritol tétracrylate, le dipentaérythritol tétracrylate éthoxylé et le dipentaérythritol tétracrylate propoxylé et les mélanges de ceux-ci.

10. Procédé de production d'un article tridimensionnel, comprenant
a) le fait de se munir de la composition photodurcissable selon l'une quelconque des revendications 1 à 9,
b) l'exposition de la composition photodurcissable à un rayonnement actinique pour former une section transversale durcie,
c) la répétition des étapes (a) et (b) pour construire un article tridimensionnel.

11. Procédé selon la revendication 10, comprenant une photopolymérisation en cuve, dans lequel la composition photodurcissable selon les revendications 1 à 9 dans l'étape b) est directement durcie sur un substrat translaté ou tourné, et le rayonnement est formé en un motif par stéréolithographie, holographie ou projection lumineuse numérique (DLP).

12. Procédé selon la revendication 10, comprenant
a) l'application d'une couche de la composition photodurcissable selon l'une quelconque des revendications 1 à 9 sur une surface ;
b) l'exposition de la couche sous forme d'image à un rayonnement actinique pour former une section transversale durcie formée en image ;
c) l'application d'une seconde couche de la composition photodurcissable sur la section transversale formée en image précédemment exposée ;
d) l'exposition de la couche de l'étape (c) sous forme d'images à un rayonnement actinique pour former une section transversale formée en image supplémentaire, dans laquelle le rayonnement provoque le durcissement de la seconde couche dans les zones exposées et l'adhérence à la section transversale précédemment exposée ; et
e) la répétition des étapes (c) et (d) pour construire un article tridimensionnel.

13. Article tridimensionnel, qui est un produit durci de la composition photodurcissable selon l'une quelconque des revendications 1 à 9 et/ou qui est produit par le procédé selon l'une quelconque des revendications 10 à 12.

14. Utilisation de la composition photodurcissable selon l'une quelconque des revendications 1 à 9, ou un (méth)acrylate à base de limonène, pouvant être obtenu par
a) réaction de a) 1 équivalent d'un composé de formule 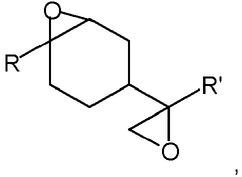 spécialement 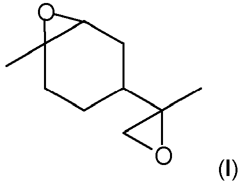 avec 1 à 3 équivalents d'un composé de formule 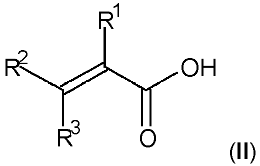 en présence d'un catalyseur et d'un inhibiteur à une température de 80 à 120 °C, dans laquelle R et R' représentent indépendamment l'un de l'autre H ou un groupement méthyle, R¹, R² et R³ représentent indépendamment l'un de l'autre H ou un groupement alkyle en C₁-C₄, spécialement un groupement méthyle, dans un procédé d'impression 3D par photopolymérisation.
